(19) Europäisches Patentamt / European Patent Office / Office européen des brevets

(11) **EP 3 571 930 B1**

(12) **FASCICULE DE BREVET EUROPEEN**

(45) Date de publication et mention de la délivrance du brevet:
**09.10.2024 Bulletin 2024/41**

(21) Numéro de dépôt: **19186686.2**

(22) Date de dépôt: **27.01.2017**

(51) Classification Internationale des Brevets (IPC):
**A23J 3/34** *(2006.01)*    **A23J 3/14** *(2006.01)*
**A23L 2/66** *(2006.01)*    **A23L 33/185** *(2016.01)*
**A23L 33/00** *(2016.01)*

(52) Classification Coopérative des Brevets (CPC):
**A23L 33/185; A23J 3/14; A23J 3/346; A23L 2/66; A23L 11/60; A23L 33/40; A61P 3/02**

(54) **ISOLAT DE PROTEINES DE POIS**

ISOLAT VON ERBSENPROTEINEN

PEA PROTEIN ISOLATE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **29.01.2016 FR 1650710**
**07.03.2016 FR 1651865**
**29.04.2016 FR 1653861**
**08.07.2016 FR 1656605**

(43) Date de publication de la demande:
**27.11.2019 Bulletin 2019/48**

(62) Numéro(s) de document de la (des) demande(s) initiale(s) en application de l'article 76 CBE:
**17706582.8 / 3 407 730**

(73) Titulaire: **Roquette Frères**
**62136 Lestrem (FR)**

(72) Inventeurs:
• **BARATA, Manuel**
**62920 MAISNIL LES RUITZ (FR)**
• **GUILLEMANT, Marilyne**
**62120 AIRE SUR LA LYS (FR)**
• **MORETTI, Emmanuelle**
**59800 LILLE (FR)**
• **MULLER, Elsa**
**62400 BETHUNE (FR)**

• **DELEBARRE, Marie**
**62136 LA COUTURE (FR)**

(74) Mandataire: **Plasseraud IP**
**104 Rue de Richelieu**
**CS92104**
**75080 Paris Cedex 02 (FR)**

(56) Documents cités:
**WO-A1-02/071863      WO-A1-2007/017572**
**WO-A1-2012/027285    WO-A1-92/15697**

• **KHETAN SHEVKANI ET AL: "Structural and functional characterization of kidney bean and field pea protein isolates: A comparative study", FOOD HYDROCOLLOIDS, vol. 43, 1 January 2015 (2015-01-01), NL, pages 679 - 689, XP055625000, ISSN: 0268-005X, DOI: 10.1016/j.foodhyd.2014.07.024**
• **STONE ANDREA K ET AL: "Functional attributes of pea protein isolates prepared using different extraction methods and cultivars", FOOD RESEARCH INTERNATIONAL, ELSEVIER, AMSTERDAM, NL, vol. 76, 18 November 2014 (2014-11-18), pages 31 - 38, XP029251370, ISSN: 0963-9969, DOI: 10.1016/J.FOODRES.2014.11.017**

**Description**

**DOMAINE TECHNIQUE DE L'INVENTION**

La présente invention concerne un isolat de protéines de pois tel que défini par les revendications 1-4.

L'application de formulations nutritionnelles comprenant ledit isolat de pois :

- en boissons, par le biais de mélanges de poudres à reconstituer, notamment pour la nutrition diététique (sport, minceur),
- en boissons prêtes-à-boire pour la nutrition diététique ou clinique,
- en liquides (boissons ou poches entérales) pour la nutrition clinique,
- en laits fermentés de type yaourts (brassés, à la grecque, à boire...)
- en boissons laitières/végétales,
- en crèmes laitières/végétales (telle que la crème pour café ou « coffee whitener »), crèmes desserts, desserts glacés ou sorbets.
- en biscuits, muffins, pancakes, barres nutritionnelles (destinés à la nutrition spécialisée / minceur ou sportif),
- en pains ou pains sans gluten enrichis en protéines,
- en céréales hyperprotéinées, obtenues par cuisson extrusion (« crisps » pour inclusion / céréales petit déjeuner / « snacks »),
- en fromages

est décrite.

L'invention est définie par les revendications annexées. If

**CONTEXTE DE L'INVENTION**

*Les poudres et liquides nutritionnels*

Les poudres et liquides nutritionnels fabriqués en pédiatrie, pour les enfants ou les adultes comprennent une sélection d'ingrédients nutritionnels bien définis (glucides, protéines, matières grasses, fibres, vitamines et/ou oligoéléments...).

Certains sont utilisés comme une source unique d'alimentation, tandis que d'autres le sont comme compléments alimentaires.

Ces produits nutritionnels comprennent des poudres qui peuvent être reconstituées, avec de l'eau ou un autre liquide aqueux, en liquides nutritionnels de type poche entérale ou boissons prêt-à-boire.

Ces formulations nutritionnelles en poudre et liquides pour les boissons prêt-à-boire et les poches entérales sont particulièrement populaires en nutrition et leur utilisation continue d'augmenter dans le monde entier.

Les formulations nutritionnelles en poudre sont typiquement préparées en faisant un mélange intime de différentes poudres.

Les formulations nutritionnelles prêtes-à-boire ou administrées par voie entérale sont typiquement préparées en faisant une ou deux solutions séparées qui sont mélangées ensuite ensemble, puis traitées thermiquement pour permettre une conservation d'au moins 12 mois à température ambiante.

Une première solution représente la phase aqueuse contenant des glucides, des protéines, des fibres, des minéraux, et des émulsifiants hydrosolubles, et la seconde représente la phase lipidique contenant l'huile et des émulsifiants liposolubles.

Il est bien entendu que l'ajout de cette seconde phase lipidique est fonction des formules nutritionnelles visées.

Ces formulations nutritionnelles en poudre et liquides sont notamment recherchées pour leur apport en protéines et leur apport en nutriments énergétiques.

De manière traditionnelle, on a surtout recours aux protéines de lait.

Pour des raisons de coût et de considérations environnementales, il est cependant préféré d'avoir recours à des protéines végétales alternatives aux protéines de lait pour l'enrichissement en protéines dans les boissons mix poudres et les boissons prêtes-à-boire.

Les protéines de soja (isolats, hydrolysats) sont utilisées en grande majorité, mais également des protéines de riz, de blé et de pommes de terre (notamment pour l'amélioration du goût végétal des produits finis).

Dans le cadre de la végétalisation des produits du marché et de la réduction de coût, il peut être proposé de développer de nouvelles solutions à base de protéines de pois pour permettre de trouver une alternative aux protéines de lait pour l'enrichissement en protéines, dans les produits finis telles que les boissons (mix poudre à reconstituer pour la nutrition diététique (sport/minceur) et les boissons prêtes-à-boire pour la nutrition clinique et diététique), et les poches entérales.

En ce cas, les protéines de pois doivent répondre à certaines fonctionnalités telles qu'une bonne solubilité, une faible viscosité en solution, une bonne résistance aux traitements thermiques pour les liquides traités thermiquement ainsi qu'une bonne stabilité de la viscosité au cours du temps.

Elles doivent aussi répondre aux recommandations nutritionnelles recommandées par la FAO/WHO, en termes de profil en acides aminés et en profil de digestibilité.

Or, il a été constaté que lorsque l'on choisit d'utiliser des protéines extraites du pois en mélange à sec dans des bases nutritionnelles poudres, même à des concentrations très faibles, et que lorsque l'on tente de reconstituer la formule nutritionnelle, celle-ci peut présenter une sensation sableuse indésirable en bouche, liée à la granulométrie, la solubilité et la composition desdites protéines.

On déplore également la trop grande viscosité des formulations à haute teneur en protéines contenant des protéines de pois.

Une solution alternative aux protéines de lait doit donc impérativement respecter les bonnes propriétés sensorielles et fonctionnelles auxquelles répondent naturellement les protéines de lait.

*Les laits ou desserts fermentés type yaourts brassés, à la Grecque, fermes*

Un yaourt, yogourt ou yoghourt est un lait ensemencé par des ferments lactiques afin de l'épaissir et de le conserver plus longtemps.

Pour s'appeler yaourt il doit contenir obligatoirement, et uniquement, deux ferments spécifiques, le *Lactobacillus delbrueckii subsp bulgaricus* et le *Streptococcus thermophilus*, qui lui donnent sa spécificité de goût, sa texture et apportent aussi certains bénéfices nutritionnels et de santé.

D'autres laits fermentés (à texture de yaourt) ont été créés au cours des dernières années. Ils peuvent contenir ou pas ces deux bactéries, et en plus des souches telles que *Lactobacillus acidophilus*, *Lactobacillus casei*, *Bifidobacterium bifidum*, *B. longum*, *B. infantis* et *B. breve*.

Les yaourts sont ainsi une excellente source de probiotiques, c'est à dire de microorganismes vivants qui, lorsqu'ils sont ingérés en quantité suffisante, exercent des effets positifs sur la santé, au-delà des effets nutritionnels traditionnels.

Qu'il soit ferme, brassé ou liquide, il conserve son appellation de yaourt, car c'est en effet, outre les définitions de la Réglementation, sa fabrication qui conditionne sa texture finale.

Ainsi, pour obtenir un yaourt ferme, on ensemence directement le lait dans le pot. Tandis que dans le cas du yaourt brassé (dit aussi « bulgare »), le lait est ensemencé dans une cuve, puis brassé avant d'être versé dans son pot.

Enfin, le yaourt liquide, dit aussi yaourt à boire, est brassé puis battu jusqu'à l'obtention de la texture adéquate et versé dans des bouteilles.

Mais il existe également d'autres types de yaourts nature, comme les yaourts à la grecque, de texture plus épaisse.

Le pourcentage en matières grasses peut également jouer sur la texture du yaourt, qui peut être fabriqué à base de lait entier, demi-écrémé ou écrémé (une étiquette ne comportant que le mot « yaourt » désigne obligatoirement un yaourt réalisé avec du lait demi-écrémé).

Dans tous les cas, sa Date Limite de Consommation (D.L.C) ne peut excéder 30 jours et il doit toujours être conservé au réfrigérateur entre 0° et 6°.

On distingue ainsi trois principales classes de yaourt :

∘ *Yaourt brassé*

Plus liquide, il est souvent plus acidulé que le yaourt nature. Seule sa texture diffère. On le nomme aussi yaourt bulgare - en référence aux origines supposées du yaourt et au *Lactobacillus bulgaricus,* l'un des deux ferments à l'œuvre dans la transformation du lait en yaourt. Il est fabriqué en cuve avant d'être conditionné en pots.

Il est particulièrement adapté à la réalisation de boissons, comme les lassis, les cocktails de fruits...

∘ *Yaourt à la grecque*

Particulièrement épais, c'est un yaourt nature très égoutté (technique traditionnelle) ou enrichi de crème. Gourmand, très savoureux, il est indispensable à la réalisation du tsatsiki et pour tous les plats d'Europe de l'Est, et tout simplement mélangé à des fines herbes, c'est un délicieux dip d'apéritif. A froid, il peut se substituer à la crème fraîche épaisse.

∘ *Yaourt à boire*

S'il existe nature, il est le plus souvent sucré et aromatisé, et fabriqué avec un yaourt brassé battu. Imaginé en 1974, il a permis aux adolescents de renouer avec le plaisir du lait, en dégustant le yaourt sans cuillère, à même la bouteille. Il existe depuis peu également en « yaourt à verser », en brique de 950 g, pour ceux qui veulent associer céréales et yaourts au petit déjeuner.

Peu énergétique - de 52 kcal pour un yaourt à 0% fabriqué à partir de lait écrémé ; à 88 kcal pour un yaourt au lait entier - le yaourt « nature » est naturellement peu riche en matières grasses et en glucides, mais contient des protéines en quantité intéressante. C'est également une source de micronutriments (notamment calcium et phosphore) ainsi que de vitamines B2, B5, B12 et A. Constitué à 80% d'eau, le yaourt participe activement à l'hydratation du corps.

La consommation régulière de yaourt est ainsi reconnue pour améliorer la digestion et l'absorption du lactose (avis du 19 octobre 2010 de l'EFSA). D'autres études montrent des bénéfices potentiels sur l'amélioration des diarrhées des enfants, et du système immunitaire chez certaines personnes comme les personnes âgées.

Toutefois, la consommation de lait de vache est de plus en plus critiquée, remise en question et on assiste à un nombre croissant de personnes qui décident tout simplement de l'éliminer de leur alimentation, pour des raisons par exemple d'intolérance au lactose, ou des problèmes d'allergénicité.

Des solutions de yaourts base laits végétaux ont donc été proposées, car les laits végétaux sont beaucoup plus digestes que le lait de vache, sont riches en vitamines, minéraux et en acides gras non saturés.

Dans la suite de notre exposé, par souci de simplicité, nous continuerons à employer le terme « yaourt » même si l'origine des protéines n'est pas laitière (officiellement, les « yaourts » qui sont fabriqués à partir d'ingrédients autres que du lait fermenté, des ingrédients laitiers, ou des ferments classiques de type *Lactobacillus delbrueckii subsp bulgaricus* et *Streptococcus thermophilus* n'ont pas le droit à cette appellation).

La source végétale la plus usitée est le soja. Cependant, même si le lait de soja présente la plus grande richesse en calcium et en protéines, il est également très indigeste ; c'est pourquoi il est déconseillé pour les enfants.

De plus, il n'est pas non plus conseillé d'abuser des produits à base de soja car leurs effets sur la santé peuvent être contre-productifs lorsqu'ils sont consommés en grande quantité.

Par ailleurs, il est communément admis que 70 % de la production mondiale du soja est OGM.

*Le lait et les boissons laitières ; les boissons végétales*

Le lait est un aliment qui contient une source de protéines non négligeables et de haute qualité biologique. Pendant longtemps, les protéines animales ont été plébiscitées pour leurs excellentes qualités nutritionnelles car elles contiennent tous les acides aminés essentiels en proportions adéquates.

Cependant, certaines protéines animales peuvent être allergènes, entraînant des réactions très gênantes, voire même dangereuses au quotidien.

L'allergie aux produits laitiers est une des réactions allergiques les plus répandues. Les études démontrent que 65 % des personnes qui souffrent d'allergies alimentaires sont allergiques au lait. La forme adulte de l'allergie au lait, appelée ici «allergie aux produits laitiers», est une réaction du système immunitaire qui crée des anticorps pour combattre l'aliment indésirable. Cette allergie est différente de l'allergie aux protéines du lait de vache (protéines bovines), également appelée l'APLV, qui touche les nouveau-nés et les enfants. Les manifestations cliniques de cette allergie sont principalement gastro-alimentaires (50 à 80% des cas), également cutanées (10 à 39% des cas) et respiratoires (19 % des cas).

Au vu de tous les désavantages cités ci-dessus liés à la consommation des protéines laitières, il en résulte un grand intérêt pour l'emploi de protéines de substitution, également appelées protéines alternatives, parmi lesquelles se classent les protéines végétales.

Les laits végétaux, obtenus à partir d'ingrédients végétaux peuvent être une alternative aux laits d'origine animale. Ils pallient et évitent les APLV. Ils sont exempts de caséine, de lactose, de cholestérol, sont riches en vitamines et en sels minéraux, sont également riches en acides gras essentiels mais pauvres en acides gras saturés. Certains possèdent également des taux de fibres intéressants.

Outre le fait que certains laits végétaux sont pauvres en calcium, que d'autres du fait de leur rareté botanique sont indisponibles dans le commerce, il faut également mentionner que certains laits végétaux sont également allergènes. C'est le cas par exemple des laits végétaux préparés à partir d'oléagineux, comme par exemple les laits de soja.

Au vu de tous les désavantages des protéines laitières mais également du caractère allergène dangereux conféré par certaines protéines végétales, il existe une réelle demande de la part des consommateurs, non satisfaite à ce jour, pour des laits végétaux possédant une innocuité indiscutable et reconnue et pouvant de ce fait être consommés par toute la famille. Les fabricants traditionnels commencent également à chercher de nouvelles sources de protéines pour enrichir leurs produits.

La société Demanderesse s'est également attelée à cette recherche afin de pouvoir répondre aux demandes croissantes des industriels et des consommateurs pour des composés possédant des propriétés nutritionnelles intéressantes sans pour autant présenter les inconvénients de certains composés déjà existants. Les travaux de la Demanderesse ont portés sur la formulation de nouveaux laits végétaux possédant une innocuité indiscutable et reconnue, et pouvant de ce fait être consommés par toute la famille.

*Les crèmes laitières pour crème à café, beurre, fromage, crèmes Chantilly, sauces, nappages, décoration de gâteaux*

Les crèmes laitières sont des produits à plus de 30 % de matière grasse (MG) obtenus par concentration du lait, se présentant sous la forme d'une émulsion de gouttelettes d'huile dans le lait écrémé. Elles peuvent être utilisées pour différentes applications, soit directement comme produit de consommation (utilisée par exemple comme crème à café) ou comme matière première en industrie pour la fabrication d'autres produits tels que le beurre, le fromage, les crèmes Chantilly, les sauces, les crèmes glacées, ou encore les nappages et la décoration de gâteaux.

Il existe différentes variétés de crèmes : fraîche, allégée, liquide, épaisse, pasteurisée. Les crèmes se distinguent selon leur teneur en matière grasse, leur conservation et leur texture.

La crème crue est la crème issue de la séparation du lait et de la crème, directement après l'écrémage et sans passer par l'étape pasteurisation. Elle est liquide et contient de 30 à 40% de matières grasses.

Toujours de texture liquide, la crème pasteurisée a subi le processus de pasteurisation. Elle a donc été chauffée à 72°C pendant une vingtaine de seconde afin d'éliminer les microorganismes indésirables pour l'homme. Cette crème se prête particulièrement bien au foisonnement. Elle prend ainsi une texture plus légère et volumineuse en étant battue pour y incorporer des bulles d'air. Elle est parfaite pour les chantilly par exemple.

Certaines crèmes liquides vendues en magasins sont dites de longue conservation. Elles peuvent être stockées plusieurs semaines dans un endroit frais et sec. Pour se conserver aussi longtemps, ces crèmes ont été soit stérilisées, soit chauffées selon le procédé UHT. Pour la stérilisation, il s'agit de chauffer la crème pendant 15 à 20 minutes à 115°C. Avec le procédé UHT (ou Ultra Haute Température), on chauffe la crème pendant 2 secondes à 150°C. La crème est ensuite rapidement refroidie, ce qui a pour résultat de mieux conserver ses qualités gustatives.

La crème est naturellement liquide, une fois qu'elle est séparée du lait, après écrémage. Afin qu'elle prenne une texture épaisse, elle passe par l'étape de l'ensemencement. On incorpore ainsi des ferments lactiques qui, après maturation, donneront à la crème cette texture plus épaisse et ce goût plus acide et plus riche.

À côté des technologies traditionnelles (millénaires ou centenaires) d'obtention de la crème à partir du lait, se sont développées depuis cette dernière décennie, des technologies d'assemblage ou de reconstitution de la crème à partir d'ingrédients laitiers.

Ces technologies nouvelles de reconstitution des crèmes laitières présentent des avantages évidents dans les procédés industriels, par rapport à la crème fraiche : faible coût de stockage des matières premières, plus grande flexibilité dans la formulation, indépendance vis-à-vis de la saisonnalité de la composition du lait.

Aussi, les crèmes laitières reconstituées peuvent bénéficier de l'image de naturalité généralement attribuée aux produits laitiers, puisque la réglementation exige pour leur fabrication l'utilisation exclusive d'ingrédients laitiers avec ou non adjonction d'eau potable et les mêmes caractéristiques de produit fini que la crème de lait (Codex Alimentarius, 2007).

Le développement du domaine des crèmes laitières reconstituées a ouvert de nouvelles possibilités dans la formulation des crèmes, et plus particulièrement celle de la naissance du concept des crèmes végétales.

Les crèmes végétales sont des produits similaires aux crèmes laitières dont la matière grasse laitière est remplacée par la matière grasse végétale (Codex Alimentarius, codex Stan 192, 1995).

Elles sont formulées au départ de quantités bien définies d'eau, de matières grasses végétales, de protéines laitières ou végétales, de stabilisants, d'épaississants et d'émulsifiants de faible poids moléculaire.

Les paramètres physico-chimiques, tels que la granulométrie, la rhéologie, la stabilité et l'aptitude au foisonnement sont les caractéristiques qui intéressent au premier chef les industriels et les chercheurs du domaine de la substitution des crèmes laitières par des crèmes végétales.

Par exemple, comme dans toute émulsion, la taille des gouttelettes dispersées (granulométrie) est un paramètre clé de la caractérisation des crèmes car il présente un impact non négligeable, d'une part, sur d'autres propriétés physico-chimiques telles que la rhéologie et la stabilité, et d'autre part, sur les propriétés sensorielles telles la texture et la couleur des crèmes.

L'influence du type d'émulsifiant inclut tant les émulsifiants de faible poids moléculaire tels les mono, diglycérides et phospholipides, que ceux de haut poids moléculaire comme les protéines, ainsi que des interactions protéines/émulsifiants de faible poids moléculaire.

Il est ainsi connu que la concentration de l'émulsifiant lipidique influence également la taille des gouttelettes des crèmes. Dans les systèmes stabilisés par des protéines, une concentration très élevée de l'émulsifiant lipidique peut entrainer une forte augmentation de la taille moyenne des gouttelettes, du fait d'une forte agrégation des gouttelettes suite à la désorption des protéines.

Le type de protéines utilisées dans la formulation peut également affecter la granulométrie des crèmes. En effet, dans les mêmes conditions d'émulsification, les crèmes à base de sources protéiques riches en caséines, telles la poudre de lait écrémé, présentent en général des diamètres moyens de gouttelettes plus petits que celles à base de sources protéiques riches en protéines de lactosérum, telles que la poudre de lactosérum.

Les différences de granulométrie entre les crèmes préparées à partir des deux sources protéiques (caséines ou protéines de lactosérum) sont liées aux différences de propriétés interfaciales à l'interface huile/eau, les caséines ayant une plus grande capacité d'abaissement de la tension interfaciale que les protéines de lactosérum.

Par ailleurs, la concentration en protéines dans la formulation influence la granulométrie des crèmes. En effet, il a été démontré qu'à fraction massique d'huile constante, la taille des gouttelettes diminue avec la concentration en protéines jusqu'à une certaine concentration au-delà de laquelle la taille varie très peu.

La présence simultanée des molécules amphiphiles de poids moléculaire faible (surfactif) et élevé (protéines) dans une formulation de crèmes se traduit généralement par une diminution de la taille des gouttelettes au cours de l'émulsification. Par ailleurs, l'adsorption compétitive à l'interface huile/eau entre tensioactifs et protéines conduit généralement au cours de la maturation à une désorption des protéines de la surface des gouttelettes, ce qui ce qui peut entrainer des modifications granulométriques.

Finalement, il apparait que les conditions d'émulsification, le choix des ingrédients (tant protéiques que lipidiques) utilisés dans la formulation, ainsi que la température, influencent les propriétés finales des crèmes.

Il apparait que les crèmes végétales peuvent conduire à de nouvelles propriétés technofonctionnelles. Ainsi, la résistance à la congélation pouvant conférer une grande stabilité aux crèmes glacées en est un exemple. Elles peuvent également présenter une stabilité en liaison chaude ou froide, ce qui est un avantage considérable, puisque ces crèmes peuvent être utilisées indifféremment dans la préparation de plats chauds ou froids.

Si les crèmes végétales peuvent apporter de nouvelles fonctionnalités et montrer des propriétés texturales comparables voire plus intéressantes que celles des crèmes laitières, il n'en demeure pas moins qu'elles peuvent présenter des défauts sensoriels, notamment par rapport à leur goût et leur odeur, même parfois après l'ajout d'arômes (cas des protéines de soja, ou des protéines de pois).

La société Demanderesse a donc mené des travaux sur les crèmes végétales (dont le domaine des « non dairy » coffee creamers) afin d'approfondir les connaissances sur l'influence de leurs ingrédients, comme les protéines de pois et leurs interactions entre eux (protéines-protéines, protéines-matière grasse, protéines-eau, etc.) sur les propriétés finales des crèmes.

La société Demanderesse a également développé des recettes de fromages végétaliens.

Le fromage est normalement un aliment obtenu à partir de lait coagulé ou de crème laitière, puis d'un égouttage suivi ou non de fermentation et éventuellement d'affinage.

Le fromage est ainsi fabriqué à partir de lait de vache principalement, mais aussi de brebis, de chèvre, de bufflonne ou d'autres mammifères. Le lait est acidifié, généralement à l'aide d'une culture bactérienne. Une enzyme, la présure, ou un substitut comme de l'acide acétique ou du vinaigre, est ensuite adjointe afin de provoquer la coagulation et former le lait caillé et le petit-lait.

Il est connu de réaliser des alternatives végétaliennes au fromage (notamment des fromages de type mozzarella), en substituant les caséinates de lait par des amidons natifs et modifiés, plus particulièrement des amidons stabilisés acétates.

Cependant, il est encore recherché à améliorer le caractère de « déchiquetabilité » (terme anglosaxon de « shredability »), la fonte, la stabilité au gel / dégel, la saveur (notamment aux Etats-Unis pour les préparations de pizza).

Des essais ont été menés en combinant huile, amidons modifiés et protéines de pois sans donner plein satisfaction.

La société Demanderesse a trouvé que l'utilisation des isolats de protéines de pois conforme à l'invention permettait de répondre à ce cahier des charges, notamment en termes de « shredability », fonte et saveur.

*Les crèmes glacées*

Les crèmes glacées contiennent classiquement des graisses animales ou végétales, des protéines (protéines de lait, protéines d'oeuf) et/ou du lactose.

Les protéines jouent alors le rôle de texturant en plus d'apporter du goût à la crème glacée.

Elles sont essentiellement produites par la pesée des ingrédients, leur pré-mélange, leur homogénéisation, pasteurisation, réfrigération à 4°C (permettant la maturation), puis la congélation avant emballage et stockage.

De nombreuses personnes souffrent cependant d'une intolérance aux produits laitiers ou d'autres ingrédients d'origine animale qui les empêchent de consommer du lait ou de la crème glacée traditionnelle.

Pour ce groupe de consommateurs, il n'y a jusqu'à présent pas d'alternative à la crème glacée contenant du lait ayant une valeur sensorielle comparable.

Dans les préparations de crème glacée jusqu'ici connues présentant des ingrédients végétaux, principalement à base de soja, des tentatives ont été faites pour remplacer les émulsifiants animaux par des protéines végétales.

Des protéines végétales séchées, obtenues dans les procédés classiques d'extraction aqueuse ou hydro-alcoolique et après le séchage sous forme de poudre ont été souvent utilisées.

Ces protéines se révèlent être des mélanges hétérogènes de polypeptides, dont certaines fractions possèdent à des degrés variables des propriétés particulièrement bonnes comme émulsifiants ou agents de formation de gel, comme agents de liaison de l'eau, de la mousse ou des agents améliorant la texture.

Jusqu'à présent, les produits de protéines végétales ont été obtenus presque exclusivement à partir de fèves de soja, sans fractionnement en fonction de leurs propriétés fonctionnelles spécifiques.

Par ailleurs, le goût des crèmes glacées préparées à partir desdites protéines de soja est rédhibitoire.

La société Demanderesse a donc mené des travaux sur les crèmes végétales et a trouvé que les isolats de protéines de pois selon l'invention permettaient de répondre au cahier des charges recherché.

*Les produits de biscuiterie, produits de pâtisserie, produits de panification et produits céréaliers hyperprotéinés*

Pour obtenir une allégation « riche en protéines » il faut, selon la réglementation en vigueur, que l'apport calorique lié aux protéines soit égal ou supérieur à 20 % de l'apport énergétique total du produit fini.

Cela signifie que, dans les produits contenant une teneur en matière grasse conséquente comme les biscuits ou les cakes (entre 10 % pour les plus maigres à 25 % pour les plus riches avec une moyenne à 18 % de matière grasse), le taux d'incorporation des protéines pour atteindre l'allégation est important et est supérieur à 20 %.

Cependant, le remplacement d'au moins 1/5$^{ème}$ de la formulation par une protéine, quelle qu'elle soit et quelle que soit la matrice (biscuits / cake), constitue un vrai défi technologique, car ces reformulations ne sont pas sans conséquence sur :

- les propriétés structurales et/ou texturales des préparations hyperprotéinées ainsi produites (liées notamment à leur niveau d'hydratation) et également des produits finis,
- le procédé de fabrication des préparations hyperprotéinées (aptitude à être façonnées par moulage, « machinabilité »),
- la qualité sensorielle des préparations hyperprotéinées et des produits finis.

La société Demanderesse a déjà proposé une protéine de pois, le NUTRALYS® BF, pour augmenter la teneur en protéines de biscuits, tout en limitant les impacts négatifs sur la préparation et le produit fini.

La solution venait d'une protéine de pois présentant peu ou pas de propriétés fonctionnelles (pouvoir émulsifiant/pouvoir gélifiant) et peu d'interaction avec l'eau, cette protéine étant peu soluble.

Cependant cette protéine ne permet pas de répondre complètement aux problématiques techniques évoquées ci-dessus.

Ainsi il est possible d'obtenir de bons résultats sur des biscuits « source de protéines », c'est-à-dire où 12 % des apports caloriques totaux sont apportés par les protéines.

Mais sur des allégations « riche en protéine » cette protéine NUTRALYS® BF présente des limites et les produits ne sont pas optimisés en termes de texture, celle-ci restant pâteuse.

La société Demanderesse a donc continué à travailler pour optimiser les qualités de protéines végétales, notamment issues du pois, en proposant de nouveaux isolats de protéines de pois conformes à l'invention, qui permettent de mieux répondre aux défis technologiques tel l'enrichissement en protéines des produits du « baking ».

En effet l'isolat de protéine de pois obtenu selon l'invention permet de combiner les bénéfices du NUTRALYS® BF, à savoir peu de fonctionnalité (pouvoir émulsifiant / pouvoir gélifiant) mais avec une forte solubilité.

La société Demanderesse a ainsi trouvé que ces deux propriétés, à sa connaissance jamais associées à ce jour, pouvaient être combinées pour offrir une source protéique permettant un fort enrichissement en protéines sans impact négatif sur le procédé de préparation ou la texture des préparations ou produits finis.

**RESUME DE L'INVENTION**

La présente invention propose un isolat de protéines de pois tel que défini à la revendication 1, susceptible de se substituer en tout ou partie à la protéine de lait ou de soja, neutre en goût. Des formulations nutritionnelles contenant l'isolat de protéines de pois sont décrites qui présentent des propriétés adaptées :

○ au mélange de poudres,
○ aux boissons stérilisées UHT prêtes-à-boire protéinées (voire riches en protéines ou dites hyperprotéinées) et
○ aux liquides nutritionnels administrées par voie entérale,

où une faible viscosité de la boisson et une amélioration de la solubilité de la protéine de pois sont recherchées et également en :

- laits fermentés de type yaourts (brassés, à la grecque, à boire...)
- boissons laitières/végétales,
- crèmes laitières/végétales (tel en « coffee whitener »), desserts glacés ou sorbets,

où la capacité émulsifiante dudit isolat de protéines de pois est d'intérêt pour son utilisation dans les matrices de ces produits laitiers en substitution partielle ou totale des protéines laitières,

- fromages végétaliens,

où l'ajout dudit isolat de protéines de pois permet d'améliorer la shredability, la fonte et la saveur des formages végétaliens de type Mozzarella.

Sont décrites également de nouvelles formulations nutritionnelles contenant un isolat de protéines de pois présentant des propriétés adaptées :

- aux biscuits, muffins, pancakes, barres nutritionnelles (destinés à la nutrition spécialisée / minceur ou sportif),
- aux pains ou pains sans gluten enrichis en protéines,
- en céréales hyperprotéinées, obtenues par cuisson extrusion (« crisps » pour inclusion / céréales petit déjeuner / « snacks »)

L'invention conduit également à améliorer le goût de la protéine de pois (diminution des notes pois, notes vertes) pour être plus neutre dans les applications/produits finis (à forte teneur en protéine et standards) utilisant l'isolat de protéines de pois en substitution partielle ou totale de protéines de lait, propriété importante pour tous les types de produits laitiers, boissons laitières ou végétales, laits fermentés de type yaourts, crèmes laitières ou végétales...

L'invention a précisément pour objet un isolat de protéines de pois qui :

- présente entre 0,5 et 2 % d'acides aminés libres,
- présente une viscosité à 20 °C dans l'eau à 15% de matière sèche:

    - de 11 à $18.10^{-3}$ Pa.s. à un taux de cisaillement de 10 $s^{-1}$,
    - de 9 à $16.10^{-3}$ Pa.s. à un taux de cisaillement à 40 $s^{-1}$, et
    - de 8 à $16.10^{-3}$ Pa.s. à un taux de cisaillement de 600 $s^{-1}$,

- présente une solubilité à 20°C dans l'eau:

    - de 30 à 40 % dans des zones de pH de 4 à 5
    - de 40 à 70 % dans des zones de pH de 6 à 8

présente un degré d'hydrolyse (DH) compris entre 5 et 10 %,
présente une teneur en protéines totales exprimée en N.6,25 de plus de 70% en poids de produit sec.

De préférence, l'isolat de protéines de pois présente une digestibilité exprimée selon le Coefficient d'Utilisation Digestive (CUD) d'une valeur comprise entre 93,5 et 95 %.

En particulier, l'isolat de protéines de pois est présenté, selon le test SYMPHID, comme une protéine de « viscosité rapide », traduisant une assimilation duodénale rapide des acides aminés constitutifs dudit isolat.

De préférence, l'isolat de protéines de pois a été pasteurisé à haute température et de courte durée avant d'être séché par atomisation.

Dans un mode de réalisation décrit, la formulation nutritionnelle comprend au moins un isolat de protéine de pois et au moins une protéine de lait. La protéine de lait représente de préférence au moins 10, 15, 20, 25, 30, 40, 45 ou 50% en poids par rapport au poids total de protéines, lorsque la formulation nutritionnelle est en poudre.

Dans un autre mode de réalisation décrit, la formulation nutritionnelle comprend au moins un isolat de protéine de pois, une protéine végétale autre, telle qu'une protéine de soja, riz et/ou blé, et au moins une protéine de lait.

L'isolat de protéines de pois représente :

- entre 40 et 100 %, de préférence entre 50 et 100 %, 60-100%, 70-100%, 80-100% ou 50-90% de la protéine totale dans la formulation nutritionnelle en poudre.

◦ entre 0,1% et 100% de la protéine totale pour les boissons prêtes-à-boire pour la nutrition clinique et minceur, de préférence entre 20-100%, 30-100%, 40-100%, 50 et 100 %, 60-100%, 70-100%, 80-100% ou 50-90% de la protéine totale dans la formulation nutritionnelle et

◦ entre 52% et 100% de la protéine totale pour les boissons prêtes-à-boire pour la nutrition du sportif, 60-100%, 70-100%, 80-100% ou 50-90% de la protéine totale dans la formulation nutritionnelle,

◦ entre 0,1 % et 100 % de la protéine totale pour les laits fermentés de type yaourts, de préférence entre 20-100%, 30-100%, 40-100%, 50 et 100 %, 60-100%, 70-100%, 80-100%, 20-60%, 30-50% ou 50-90% de la protéine totale dans la formulation nutritionnelle,

◦ entre 0,1 % et 100 % de la protéine totale pour les boissons laitières, de préférence entre 20-100%, 30-100%, 40-100%, 50 et 100 %, 60-100%, 70-100%, 80-100% ou 50-90% de la protéine totale dans la formulation nutritionnelle

◦ entre 0,1 % et 100 % de la protéine totale pour les crèmes laitières, desserts glacés ou sorbets, plus particulièrement entre 50 -100 %, 60-100%, 70-100%, 80-100% ou 50-90% de la protéine totale pour les coffee whitener et entre 20-100%, 30-100%, 40-100%, 50-100%, ou 40-90% de la protéine totale pour les crèmes laitières, desserts glacés ou sorbets,

◦ entre 5 % et 100 % de la protéine totale pour les biscuits, muffins, pancakes, barres nutritionnelles (destinés à la nutrition spécialisée / minceur ou sportif), de préférence entre 20-100%, 30-100%, 40-100%, 50 et 100 %, 60-100%, 70-100%, 80-100% ou 50-90% de la protéine totale dans la formulation nutritionnelle

◦ entre 5 % et 100 % de la protéine totale pour les pains ou pains sans gluten enrichis en protéines, de préférence entre 10-100%, 20-100%, 30-100%, 40-100%, 50 et 100 %, 60-100%, 70-100%, 80-100% ou 50-90% de la protéine totale dans la formulation nutritionnelle

◦ entre 5 % et 100 % de la protéine totale pour les céréales hyperprotéinées, obtenues par cuisson extrusion (« crisps » pour inclusion / céréales petit déjeuner / « snacks »), de préférence entre 20-100%, 30-100%, 40-100%, 50 et 100 %, 60-100%, 70-100%, 80-100% ou 50-90% de la protéine totale dans la formulation nutritionnelle.

Pour les fromages végétaliens, environ 5 % en poids d'isolats de protéines de pois dans la recette suffisent à améliorer leurs caractéristiques techniques et organoleptiques.

Par exemple, l'isolat de protéines de pois selon la présente invention peut représenter 0,1-10%, 10-20%, 20-30%, 40-50%, 50-60%, 60-70%, 70-80%, 80-90% ou 90-100%, en particulier en poids, de la protéine totale dans la formulation nutritionnelle, ou une quelconque combinaison de ces gammes de pourcentages.

## DESCRIPTION DETAILLEE DE L'INVENTION

La présente divulgation concerne des formulations nutritionnelles comprenant un isolat de protéines de pois selon la présente invention. L'invention concerne l'isolat selon les revendications 1-4. Son utilisation pour la préparation des formulations nutritionnelles est également décrite.

Plus particulièrement, la présente divulgation concerne l'application de ces formulations nutritionnelles en boissons, par le biais de mélanges de poudres à reconstituer, pour la nutrition diététique (sport, minceur), et en boissons prêtes-à-boire pour la nutrition clinique (voie orale ou poche entérale) et diététique, où l'on recherche une faible viscosité de la boisson et une amélioration de la solubilité de la protéine de pois.

Egalement, la présente divulgation concerne l'application de ces formulations nutritionnelles en boissons laitières ou végétales, en laits fermentés de type yaourts (brassés, à la grecque, à boire) et en crèmes laitières ou végétales, desserts glacés ou sorbets.

Enfin, la présente divulgation concerne l'application de ces formulations nutritionnelles en biscuits, muffins, pancakes, barres nutritionnelles (destinés à la nutrition spécialisée / minceur ou sportif), en pains ou pains sans gluten enrichis en protéines, en petites céréales obtenues par cuisson extrusion (« crisps ») hyperprotéinés, où l'on recherche plus particulièrement des solutions hyperprotéinées sans impact négatif sur le procédé de préparation ou la texture des préparations ou produits finis.

Quant au goût, il a été trouvé par la société Demanderesse que la sensation sableuse indésirable en bouche de la poudre reconstituée résultant du mélange à sec d'une protéine de pois dans une formulation nutritionnelle en poudre enrichie en protéines peut être réduite ou éliminée par la mise en oeuvre d'un isolat de protéines de pois particulier.

Il a également été trouvé que l'incorporation dans ladite formule nutritionnelle de l'isolat de protéines de pois de l'invention permet d'améliorer le goût de la protéine de pois en diminuant la note pois et la note végétale.

Au sens de de la présente divulgation, on entend par « formulations nutritionnelles en poudre », des formulations en poudre comprenant :

◦ au moins une protéine végétale, et en particulier de pois,
◦ de manière facultative, au moins une protéine d'origine laitière, et

○ de manière facultative, au moins un ingrédient de type matières grasses et glucides,

qui sont reconstituables avec un liquide aqueux, et qui conviennent pour l'administration orale à un être humain.

L'expression « mélange à sec » tel qu'utilisé ici, sauf indication contraire, se réfère au mélange des composants ou des ingrédients pour former une poudre nutritionnelle de base ou, à l'addition d'un composant sec, en poudre ou granulé ou d'un ingrédient à base poudre pour former une formulation nutritionnelle en poudre.

Tous les pourcentages, parties et rapports, tel qu'utilisé ici, se rapportent au poids de la formulation totale, sauf indication contraire.

Les formulations alimentaires en poudre et les procédés de fabrication décrits peuvent comprendre, consister ou consister essentiellement en les éléments essentiels de l'invention telle que décrite ici, ainsi que tout élément supplémentaire ou facultatif décrit ici ou autrement utiles dans les applications de la formulation nutritionnelle.

Les formulations nutritionnelles en poudre décrites comprennent un isolat de protéines de pois.

Les formulations nutritionnelles en poudre décrites sont généralement sous la forme de compositions particulaires aptes à l'écoulement ou sensiblement fluides, ou au moins des compositions particulaires qui peuvent être facilement moulées et mesurées à l'aide d'une cuillère ou d'un autre dispositif similaire, dans lequel les compositions peuvent facilement être reconstitués par l'utilisateur prévu avec une solution aqueuse, typiquement de l'eau, pour former une formulation nutritionnelle liquide pour utilisation orale ou entérale immédiate.

Dans ce contexte, l'utilisation « immédiatement » signifie généralement dans environ 48 heures, plus typiquement pendant environ 24 heures, de préférence juste après la reconstitution.

Les formulations nutritionnelles en poudre comprennent des isolats de protéines de pois qui, dans certains modes de réalisation, peuvent représenter jusqu'à 100% de la protéine apportée.

Les formulations alimentaires en poudre peuvent être formulées avec tous types et quantités de nutriments suffisantes de manière à former un complément alimentaire, ou une formulation nutritionnelle spécialisée destinée à être utilisée chez les personnes suivant un régime alimentaire particulier destiné à la diététique du sport et de la minceur.

Dans un exemple de réalisation, la formulation nutritionnelle en poudre peut être formulée pour une utilisation :

○ pour réparer les muscles après un effort intense, par exemple chez le sportif, ou
○ pour assurer chez le sportif le maintien ou la construction de la masse musculaire, ou
○ comme substitut de repas par des personnes désirant perdre du poids via un effet satiétogène.

Les formulations alimentaires en poudre peuvent avoir une densité calorique adaptée aux besoins nutritionnels de l'utilisateur final, bien que dans la plupart des cas, les poudres reconstituées comprennent d'environ 350 à environ 400 kcal / 100 ml.

Les formulations alimentaires en poudre peuvent présenter un taux protéique adapté aux besoins nutritionnels de l'utilisateur final, bien que dans la plupart des cas, les poudres reconstituées comprennent d'environ 20 à environ 91 g de protéines / 100 g, y compris d'environ 40 à environ 65 g de protéines / 100g.

Ainsi, la formulation peut comprendre entre 20 et 95 % de protéines par rapport au poids totale de la formulation, par exemple entre 20-90%, 30-80%, ou 40-60%.

Par exemple, l'isolat de protéines de pois selon la présente invention peut représenter 40-50%, 50-60%, 60-70%, 70-80%, 80-90% ou 90-100% de la protéine totale de la formulation, ou une quelconque combinaison de ces gammes de pourcentages.

Par ailleurs, les formulations alimentaires en poudre peuvent présenter un taux de matière grasse adapté aux besoins nutritionnels de l'utilisateur final, bien que dans la plupart des cas, les poudres reconstituées comprennent d'environ 0,5 à environ 13 g /100 g, y compris d'environ 3 à environ 7 g /100 g.

Ainsi, la formulation peut comprendre entre 0 et 20 % de lipides par rapport au poids totale de la formulation, par exemple entre 0,5-15%, 1-10%, ou 3-7% (en particulier % en poids).

Les formulations nutritionnelles en poudre de la présente invention peuvent être emballées et scellées dans des conteneurs simples ou multi-usage, puis stockées dans des conditions ambiantes jusqu'à environ 36 mois ou plus, plus typiquement d'environ 12 à environ 24 mois.

Pour multi-utilisation des conteneurs, ils peuvent être ouverts et couverts pour une utilisation répétée par l'utilisateur final, à condition que le paquet recouvert soit ensuite stocké dans des conditions ambiantes (par exemple, éviter les températures extrêmes) et les contenus utilisés dans environ un mois ou deux.

Les domaines d'applications des formulations nutritionnelles selon l'invention sont notamment :

○ la nutrition diététique (sport, minceur),
○ la nutrition clinique (sous forme de boisson, de crème dessert ou de poche entérale),
○ les produits laitiers (sous forme de yaourts, boissons laitières, crèmes laitières, desserts glacés ou sorbets).
○ les produits de biscuiterie, produits de pâtisserie, produits de panification et produits céréaliers hyperprotéinés.

Dans le domaine du sport, il est connu que les protéines participent à l'entretien et à la croissance du muscle. L'apport en protéines est également important pour les athlètes qui pratiquent la musculation ou le renforcement musculaire.

Ces protéines doivent être équilibrées en termes de profil en acide aminés et doivent respecter les recommandations de la FAO/WHO. Leur digestibilité est un facteur important allant d'une rapide digestibilité à une plus lente digestibilité en fonction du moment de l'apport en protéines.

Les boissons protéinées ou hyperprotéinées prêtes à boire permettent alors de faire bénéficier l'organisme d'un apport protéique de choix, les calories en moins.

Ces boissons hyperprotéinées doivent :

- être riches en protéines, pauvres en glucides et en graisses ;
- avoir bon goût ;
- être conçues pour aider à perdre du poids, en stimulant la perte de graisse et en aidant à la récupération musculaire ;
- être satiétogènes ;
- permettre de faire face aux fringales, sans sucres ni graisses ajoutées ;
- présenter un contenu équilibré en acides aminés essentiels, en fibres, en vitamines et minéraux ;
- être hypocaloriques.

Ces boissons prêtes-à-boire peuvent être avantageusement préparées avec les isolats de protéines de pois conformes à l'invention. Elles peuvent être d'ailleurs utilisées comme seule source de protéines.

Par exemple, les boissons végétales alternatives au lait de vache, contiennent en moyenne de 4,5 à 11 g de protéines pour 100 ml de boisson, de préférence de l'ordre de 7 g de protéines pour 100 ml, et sont très pauvres en fibres (de l'ordre de 0,5 à 1 g pour 100 ml).

Ainsi, la boisson peut comprendre entre 1 et 20 % de protéines par rapport au poids totale de la boisson, par exemple entre 3-15%, ou 6-8%.

Par exemple, l'isolat de protéines de pois selon la présente invention peut représenter 50-60%, 60-70%, 70-80%, 80-90% ou 90-100% de la protéine totale, ou une quelconque combinaison de ces gammes de pourcentages. De préférence, il représente au moins 52 %. Notamment, l'apport en protéines de pois est compris entre 52 et 100 % de l'apport en protéines totales.

Pour les boissons prêtes à boire, l'apport en protéines de pois peut aller de 0 à 100%, de préférence de 0,01 ou 0,1 à 100 %. Par exemple, l'isolat de protéines de pois selon la présente invention peut représenter 0,1-10%, 10-20%, 20-30%, 40-50%, 50-60%, 60-70%, 70-80%, 80-90% ou 90-100% de la protéine totale, ou une quelconque combinaison de ces gammes de pourcentages.

Grâce à un goût dépourvu de note végétale marquante, cette source protéique est bien adaptée à tout type de boissons et grâce à sa viscosité modérée, peut être incorporée jusqu'à 100 % sans altérer le goût final (même si, pour les très hautes teneurs, il peut être avantageux d'ajouter des arômes).

Dans le domaine des boissons « minceur », i.e. destinées à être utilisées dans des régimes hypocaloriques ou destinées à la perte de poids, comme mentionné précédemment, ces boissons protéinées ou enrichies en protéines ne sont pas uniquement efficaces pour une prise rapide de muscles. Ce type de boisson est également très avantageux dans le cadre d'un régime minceur basé sur la consommation de protéines.

Il est connu que les boissons minceurs sont idéales pour aider à la perte de poids. Elles permettent plus particulièrement de :

- procurer un effet de satiété
- protéger les muscles et de tonifier le corps, évitant la reprise de poids.

Comme pour les boissons « sport », ces boissons minceurs présentent :

  ◦ un contenu équilibré en acides aminés essentiels, en fibres, en vitamines et minéraux
  ◦ un contenu réduit en sucres, en matières grasses et en calories.

C'est ainsi que les boissons protéinées sont en effet d'une grande efficacité pour perdre rapidement quelques kilos. Ces préparations riches en protéines réduisent ou stoppent simplement la sensation de faim chez la personne qui en consomme. En prenant par exemple une telle boisson, l'utilisateur peut réduire de manière considérable la quantité de nourriture à consommer, et permettre une perte de pois rapide (dans le cadre d'un processus de substitut de repas pour contrôle du poids, ou substitut de la ration journalière totale pour contrôle du poids).

En nutrition clinique, il est connu que la nutrition entérale est une solution thérapeutique de nutrition par sonde qui est utilisée lorsque le tube digestif est fonctionnel et accessible mais lorsque le patient ne peut s'alimenter normalement ou encore dans les cas de dénutrition sévère.

Cette technique permet d'apporter directement les nutriments dans le tube digestif. Elle remplace, de manière totale ou partielle, l'alimentation orale traditionnelle par des formules nutritives «complètes» apportant l'ensemble des nutriments nécessaires à l'organisme.

Ces formules sont généralement conditionnées dans des poches souples (en PVC) et administrées au moyen de sondes naso-gastriques ou, gastrostomies, naso-jéjunale, nasoduodénale, jéjunostomie.

Ces mélanges nutritionnels sont composés de protéines, lipides, glucides, vitamines et minéraux avec ou sans fibres.

On distingue plusieurs catégories : les mélanges polymériques (standards) et les mélanges semi-élémentaires («prédigérés»), ces derniers étant indiqués dans des cas bien spécifiques (syndrome du grêle court, insuffisance pancréatique exocrine, etc.) :

Mélanges polymériques

 ◦ hypocaloriques (0,5 - 0,75 kcal /ml), normo ou hyperprotéinés, avec ou sans fibres
 ◦ isocaloriques (1 kcal / ml), normo ou hyperprotéinés, avec ou sans fibres
 ◦ hypercaloriques (1,25 -1,5 kcal / ml) normo ou hyperprotéinés, avec ou sans fibres
 ◦ formules spécifiques (troubles du métabolisme glycémique, insuffisance respiratoire).

Les semi-élémentaires sont des mélanges iso ou hypercaloriques normo ou hyperprotéinés, à base de peptides et de triglycérides à chaînes moyennes.

Les isolats de protéines de pois, comme source de protéines, par leurs propriétés fonctionnelles sont particulièrement bien adaptés à cet usage.

Par ailleurs, ils permettent de préserver les mêmes propriétés que les protéines de lait, et ce pour un coût moindre.

Dans le domaine de la substitution (totale ou partielle) des protéines laitières dans les yaourts, boissons laitières, crèmes laitières, crèmes glacées ou sorbets, il est recherché des protéines végétales dont les propriétés fonctionnelles sont équivalentes, voire améliorées par rapport aux protéines laitières.

Le terme « propriétés fonctionnelles » signifie dans la présente demande toute propriété non nutritionnelle qui influence l'utilité d'un ingrédient dans un produit laitier.

Ces diverses propriétés contribuent à l'obtention des caractéristiques finales désirées du produit laitier. Quelques-unes de ces propriétés fonctionnelles sont la solubilité, la viscosité, les propriétés moussantes, les capacités émulsifiantes.

Les protéines jouent également un rôle important dans les propriétés sensorielles des matrices alimentaires dans lesquelles elles sont utilisées, et il existe une réelle synergie entre les propriétés fonctionnelles et les propriétés sensorielles.

Les propriétés fonctionnelles des protéines ou fonctionnalités sont donc les propriétés physiques ou physico-chimiques qui ont une incidence sur les qualités sensorielles des systèmes alimentaires générées au cours des transformations technologiques, de la conservation ou des préparations culinaires domestiques.

On constate quel que soit l'origine de la protéine qu'elle intervient sur la couleur, la flaveur et/ou la texture d'un produit. Ces caractéristiques organoleptiques interviennent de façon déterminante dans le choix du consommateur et elles sont dans ce cas largement prises en compte par les industriels.

La fonctionnalité des protéines est le résultat d'interactions moléculaires de ces dernières avec leur environnement (autres molécules, pH, température...).

Ici, il s'agit des propriétés de surface qui regroupent les propriétés d'interaction des protéines avec d'autres structures polaires ou apolaires en phase liquide ou gazeuse : cela recouvre les propriétés émulsifiantes, moussantes...

La société Demanderesse a constaté qu'il existait un réel besoin, non satisfait, de disposer d'une formulation nutritionnelle possédant des propriétés fonctionnelles intéressantes, pouvant être employée dans la confection de produits laitiers comme un substitut au moins partiel des protéines laitières.

Les isolats de protéines de pois, comme source de protéines, notamment pour leurs propriétés d'amélioration du goût, sont particulièrement bien adaptés à cet usage.

Plus particulièrement, dans ces domaines d'application particuliers, i.e. :

 ◦ laits fermentés de type yaourts (brassés, à la grecque, à boire...)
 ◦ boissons laitières/végétales,
 ◦ crèmes laitières/végétales (tel en « coffee whitener »), desserts glacés ou sorbets, la société Demanderesse a trouvé que :

- En matière de « non dairy coffee whiteners » ou encore appelés « non dairy coffee creamers », comme il sera démontré ci-après :

  ◦ La viscosité des émulsions après pasteurisation avant séchage est plus proches du témoin lait que les protéines de pois de type NUTRALYS®, ce qui permet de sécher une émulsion de faible viscosité à haute matière sèche ;

  ◦ La floculation dans le café semble être moins importante avec les isolats de protéines de pois conformes à l'invention, qu'avec les protéines de pois de type NUTRALYS® mais ceci peut être corrélé avec l'amélioration de leur solubilité au pH acide du café, ou par une meilleure stabilité aux ions divalents contenus dans l'eau de reconstitution du café.

  Par ailleurs, de manière facultative, pour améliorer les pouvoirs floculants, il peut être choisi d'ajouter des agents tamponnant tels que des citrates de sodium, des sels type NaCl (sel) qui favorise la solubilité des protéines, ou des agents complexant des ions divalents plus performants que les sels de phosphates.

  ◦ Quant au pouvoir émulsifiant desdits isolats pour cette application particulière, il peut être avantageusement choisi d'utiliser des émulsifiants complémentaires, comme par exemple le E472 (Esters monoacétyltartriques et diacétyltartriques des mono- et diglycérides d'acides gras), ou de faire varier les concentrations en E471, ou en ajustant la concentration en protéines ou ajustant le procédés d'homogénéisation.

- En matière de « yaourts brassés », dans les recettes de fabrication telles que celles qui seront exemplifiées, ci-après,

  ◦ La température avant homogénéisation peut varier entre 65 à 80°C,
  ◦ La pression d'homogénéisation peut varier entre 150 à 250 bars,
  ◦ La température de pasteurisation peut varier entre 80-85°C pendant 30min à 90-95°C de 5 à 10min,
  ◦ La température de fermentation peut varier entre 30 à 45°C, préférentiellement de 38 à 42°C
  ◦ Il est possible d'élargir le type de ferments à tous ceux utilisés dans le domaine des yaourts comme spécifié dans la réglementation « yaourt. »

Quant à la formulation desdits yaourts :

◦ En plus de l'amidon modifié et de la pectine, comme stabilisants, il peut être choisi de la graine de caroube, ou de la gomme guar en différentes proportions,
◦ L'amidon retenu est un amidon modifié, préférentiellement un amidon qui déploie peu de viscosité, voire complètement solubilisé. Sa proportion peut varier entre 2,5 et 5 % en poids de la composition totale, de préférence entre 2,8 et 3,5 %.
◦ Il peut être avantageusement choisi d'ajouter des arômes, permettant d'apporter une note « plus laitière », lactiques, ou des préparations de fruits, même si la note végétale est bien plus atténuée avec les isolats de protéines de pois selon l'invention en regard des protéines de pois.

- En matière de « yaourts à boire », les recettes décrites sont similaires à celles mises en oeuvre pour les « yaourts brassés », la quantité de protéines est cependant plus faible que celle des yaourts brassés, la quantité d'amidon est choisie préférentiellement entre 1,5% et 2,5% en poids de la composition totale, et l'amidon peut être dans un état solubilisé ou non pour cette matrice.

Dans le domaine de l'enrichissement en protéines, l'apport en calories des protéines peut s'avérer compliqué dans les produits de cuisson (terme anglosaxon de « baking ») :

- dans les produits secs contenant de la matière grasse comme les biscuits, cela oblige à mettre une concentration importante de protéines dans la formulation. Ce qui a un impact certain :

  ◦ sur la texture du produit fini (augmentation de la dureté / perte de croustillance, texture pâteuse)
  ◦ sur le goût du produit fini (amertume, goût de haricot...) et
  ◦ sur le procédé de fabrication (problématique de façonnage/ rhéologie de la pâte / compétition pour l'eau avec les autres ingrédients...)

- dans les produits humides comme le pain, l'incorporation de protéines à un impact sur la rhéologie de la pâte. Les protéines ajoutées entrant en compétition avec le réseau de gluten, cela a pour conséquence une diminution du volume du pain et une texture plus compacte et pâteuse.

Une application plus critique encore est la réalisation de crisps hyper protéinés, c'est-à-dire de petites céréales obtenues par extrusion et destinées à être utilisées en inclusion dans des barres de céréales ou autres agglomérations de céréales comme les « clusters » ou « müesli ».

Une teneur en protéines supérieure à 70% est recherchée dans ces crisps hyperprotéinés, ce qui a pour conséquence de réduire considérablement la part d'amidon dans la recette, lequel est responsable de l'expansion et donc de la croustillance. Sans ces amidons, les crisps hyperprotéinés sont denses et très durs.

Des travaux sont réalisés depuis plusieurs années sur les fonctionnalités des protéines pour sélectionner la protéine ayant le moins d'impact sur la texture des produits de cuisson enrichis en protéines.

Pour la société Demanderesse, c'est dans ce contexte que la protéine de pois NUTRALYS® BF a été développée car elle présente une faible solubilité et peu d'interaction avec l'eau.

Cependant, cette protéine de pois ne permet toutefois pas à répondre complètement aux problématiques techniques évoquées ci-dessus.

Ainsi un biscuit « riche en protéines » avec du NUTRALYS® BF n'est pas optimisé en terme de texture, celle-ci restant pâteuse.

Pour les crisps hyper protéinés, le NUTRALYS® BF ne permet pas non plus d'atteindre la texture croustillante recherchée.

En pain, malgré une augmentation du volume du pain après cuisson, le volume reste malgré tout inférieur à celui du pain témoin.

Pour solutionner ces difficultés, la société Demanderesse a ainsi trouvé que les isolats de protéines de pois conformes à l'invention permettaient :

- d'améliorer la solubilité par rapport aux protéines de pois,
- de diminuer la viscosité dans de l'eau par rapport aux protéines de pois.

Les isolats de protéines de pois développés présentent à la fois une forte solubilité et une faible viscosité, ce qui constitue une combinaison nouvelle de propriétés.

Ce faisant, la société Demanderesse a vaincu un préjugé technique qui veut que pour répondre aux problématiques des produits de cuisson, il fallait plutôt choisir une protéine de pois ayant peu d'interaction avec l'eau, alors qu'il s'avère qu'une protéine soluble mais peu visqueuse performe mieux.

*Nature des isolats de protéines de pois*

Les isolats de protéines de pois selon l'invention sont tout d'abord caractérisés par leur teneur en acides aminés libres (déterminée selon la norme NF EN ISO13903:2005).

Cette valeur est comprise entre 0,5 et 2 %. Par exemple, cette valeur peut être comprise entre 0,5-1%, 1-1,5% ou 1,5-2 %, ou une quelconque combinaison de ces gammes de pourcentages.

A titre comparatif, les protéines de pois (tel le NUTRALYS® S85F) présentent une teneur en acides aminés libres de l'ordre de 0,18 %.

Les isolats de protéines de pois présentent une teneur en protéines totales exprimée en N.6,25 de plus de 70% en poids de produit sec, de préférence au moins 80% en poids, par exemple entre 80 et 99%, 80 et 95%, 80 et 90% ou 80 et 85%.

Les isolats de protéines de pois selon l'invention sont également caractérisés par

○ leur profil de viscosité dans l'eau à 15 % de matière sèche et à 20°C, déterminé en fonction du taux de cisaillement ;
○ leur profil de solubilité dans l'eau, en fonction du pH, de préférence à 20°C.

Pour la détermination du profil de viscosité dans l'eau, les mesures sont effectuées

○ sur une solution aqueuse d'isolats de protéines de pois à 15 % de matière sèche,
○ en rhéomètre AR2000 de la société TA Instruments,
○ présentant une géométrie à cylindres concentriques,
○ avec un taux de cisaillement de $0,6 \cdot 10^{-3}$ à 600 s$^{-1}$ en 3 minutes (log) et
○ à la température de 20°C (3 min d'équilibre température avant test).

Les taux de cisaillement produits dans le rhéomètre permettent de mimer les conditions de traitement que peuvent subir les solutions d'isolat de protéines de pois selon l'invention :

- un taux de cisaillement de 1 à 10 s$^{-1}$ est ainsi caractéristique d'une boisson au repos (texture à la cuillère pour des produits plus visqueux),
- un taux de cisaillement de 40 à 50 s$^{-1}$ est la texture en bouche,
- un taux de cisaillement de 300-1000 s$^{-1}$ est équivalent au cisaillement dans des pompes d'acheminement du produit.

C'est ainsi que les isolats de protéines de pois conformes à l'invention présentent une viscosité :

- de 11 à 18.10$^{-3}$ Pa.s. à un taux de cisaillement de 10 s$^{-1}$, de préférence 12 à 17.10$^{-3}$ Pa.s., de manière encore plus préférée de 13 à 1610$^{-3}$ Pa.s.,
- de 9 à 16.10$^{-3}$ Pa.s. à un taux de cisaillement à 40 s$^{-1}$, de préférence de 10 à 15.10$^{-3}$ Pa.s., de manière encore plus préférée de 10 à 14.10$^{-3}$ Pa.s., et
- de 8 à 16.10$^{-3}$ Pa.s. à un taux de cisaillement de 600 s$^{-1}$, de préférence de 9 à 15.10$^{-3}$ Pa.s., de manière encore plus préférée de 9,8 à 14.10$^{-3}$ Pa.s.

Ce qui traduit une remarquable stabilité desdits isolats, quelle que soit la force de cisaillement qu'ils subissent.

Les isolats de protéines de pois sont ensuite caractérisés par leur profil de solubilité dans l'eau, en fonction du pH.

Le principe de la méthode utilisée est le suivant, comme il sera développé dans la partie exemple :

- suspendre l'isolat de protéines de pois à 2,5 % en poids dans de l'eau distillée,
- ajuster au pH souhaité : ici à 3, 4, 5, 6,7 ou 8 avec NaOH 0,1 N ou HCl 0,1 N,
- mélanger pendant 30 minutes à 1100 rpm,
- centrifuger 15 minutes à 3000 g,
- mesurer la matière sèche d'une portion du surnageant.

La solubilité des isolats de protéines de pois est ainsi :

- de 30 à 40 % dans des zones de pH de 4 à 5,
- de 40 à 70 % dans des zones de pH de 6 à 8,

ce qui traduit leur remarquable solubilité dans ces zones de pH.

A titre comparatif, les protéines de pois (telle NUTRALYS® S85F) présentent :

- de 10 à 15 % de solubilité dans des zones de pH de 4 à 5,
- de 20 à 50 % de solubilité dans des zones de pH de 6 à 8.

Les isolats de protéines de pois sont caractérisés également par leur profil de digestibilité totale, en regard d'une protéine de pois intacte, et par leur cinétique de digestion.

Comme il sera exemplifié ci-après, la digestibilité mesurée *in vivo* permet d'attribuer aux isolats de protéines de pois selon l'invention un Coefficient d'Utilisation Digestive (CUD) d'une valeur comprise entre 93,5 et 95 %.

Pour mesurer la cinétique de digestion des isolats de protéines de pois, il est utilisé un modèle *in vitro* de digestion dynamique dans des conditions physiologiques équivalentes à l'estomac puis l'intestin grêle (voir exemple 1 section 4).

Comme il sera exemplifié ci-après, le comportement des isolats selon l'invention dans un tel modèle montre leur positionnement original entre des protéines intactes de pois (digestion de type « intermédiaire rapide ») et de lactosérum (digestion de type « rapide »).

Les isolats de protéines de pois sont enfin caractérisés dans un modèle de digestibilité *in vitro* comme des « protéines à digestibilité rapide ».

Pour obtenir ce résultat, le comportement gastrique de 5 protéines (protéines de pois, protéines de lactosérum et caséinates de sodium, et deux lots d'isolats de protéines de pois selon l'invention) est évalué dans un modèle de digestion *in vitro* (voir exemple page 32, exemple 1 section 5).

Les cinétiques de digestion des protéines dépendent dans une large mesure du temps de séjour dans l'estomac et du temps de vidange gastrique.

La viscosité est une caractéristique importante déterminant le taux de vidange gastrique. Ainsi, les mesures de viscosité *in vitro* dans les conditions gastriques sont sélectionnées comme paramètres pertinents pour caractériser les protéines.

Les préparations protéiques sont introduites dans un système *in vitro* de simulation la digestion gastrointestinale,

**EP 3 571 930 B1**

en l'occurrence ici le système développé par la société NIZO (système SIMPHYD pour SIMulation of PHYsiological Digestion) tel que présenté sur le site www.nizo.com dans leur brochure intitulée Bioavailabilty of your ingrédients qui fait référence à l'article publié dans Appl Environ Microbiol. 2007, Jan;73(2) : 508-15.

Ce dispositif présente un système de mesures rhéologiques en ligne qui permet de comparer le comportement des protéines testées.

Les profils de viscosité au cours du temps sont mesurés en conditions gastriques de pH et de libération d'enzymes.

Comme il est illustré ci-après, comparées aux protéines de lactosérum (classées dans la catégorie « basse viscosité ») et aux caséinates de sodium (classés dans les protéines de « haute viscosité prolongée ») :

- les protéines de pois présentent une augmentation rapide de viscosité durant l'acidification, qui revient à la ligne de base à pH 2 (protéines dites de « viscosité intermédiaire rapide »), tandis que
- les isolats de protéines de pois selon l'invention présentent une augmentation très légère de viscosité après l'acidification, diminuant ensuite pour atteindre des valeurs similaires aux protéines de petit lait, durant 30 minutes (protéines dites de « viscosité rapide »).

Basés sur leur comportement gastrique *in vitro,* les isolats de protéines de pois selon l'invention sont donc transportés rapidement dans le duodénum, ce qui résultera en une assimilation rapide de leurs acides aminés.

L'évaluation des propriétés émulsifiantes des isolats de protéines de pois est réalisée en comparaison avec les protéines de pois et les protéines de lait.

Elle a été réalisée à l'aide du granulomètre Malvern Mastersizer 2000E en voie liquide.

Le principe de la mesure est basé sur la diffraction de la lumière.

Les poudres sont mises en solution à 1 % en poids dans de l'eau azidurée sous agitation pendant 6 h à 750 rpm.

4 ml d'huile de consommation combinant 4 huiles végétales (tournesol, colza, oléisol, pépins de raisins) (par exemple huile Lesieur Isio 4) sont ajoutés à 20 ml de protéines (ou isolat de protéines) à 1 %.

L'ensemble est passé à un homogénéiseur (Ultraturax) pendant 3 minutes à 13500 rpm, puis les émulsions ainsi formées sont analysées au granulomètre afin d'en déterminer la taille des globules gras.

Comme il sera exemplifié ci-après, les isolats de protéines de pois selon l'invention présentent de meilleures propriétés émulsifiantes que les protéines laitières.

Par ailleurs, leur propriété émulsifiante équivalente aux caséinates les rend tout particulièrement intéressant pour la réalisation d'émulsions séchées type « coffee whitener ».

La présente invention est relative à l'isolat de protéines de pois tel que décrit ci-dessus et à son utilisation pour la préparation d'une formulation nutritionnelle.

*Préparation des isolats de protéines de pois selon l'invention*

La préparation des isolats de protéines de pois selon l'invention comporte une hydrolyse des protéines de pois par voie enzymatique ou non enzymatique, de manière à ce que ledit isolat de protéines de pois présente un degré d'hydrolyse (DH) compris entre 5 % et 10 %, de préférence entre 6 % et 8 %, de manière encore plus spécifique de 6,5 % à 7 %.

Dans un premier mode de réalisation, l'hydrolyse est réalisée par une endopeptidase.

Il est choisi une endopeptidase non spécifique, dérivée d'une souche *d'Aspergillus* en particulier une souche de *Aspergillus spp* ou *Aspergillus Oryzae*.

Il est choisi plus particulièrement une endopeptidase EC 3-4-11.

La quantité exacte d'enzyme ajoutée à la suspension pour obtenir les caractéristiques souhaitées des isolats de protéines de pois variera en fonction de caractéristiques spécifiques telles que:

(1) l'enzyme ou le système enzymatique utilisé;
(2) le degré final désiré d'hydrolyse; et/ou
(3) la distribution de poids moléculaire / final souhaité.

Étant donné que ces paramètres sont connus, l'homme de l'art peut facilement déterminer les conditions appropriées pour obtenir les caractéristiques désirées de l'isolat de protéines de pois.

Dans un mode de réalisation particulier, la protéine de pois initiale utilisée pour préparer l'isolat de protéines de pois selon l'invention est une composition de protéine de pois telle que décrites dans la demande WO 2007/17572 ou préparée par un procédé tel que décrit dans la demande WO 2007/17572. Dans un mode de réalisation particulier, la composition de protéines de pois initiale est la composition commercialisée par ROQUETTE FRERES sous le nom de marque NUTRALYS® S85F.

Dans un mode de réalisation préféré de l'invention, la suspension de protéines de pois est amenée à une valeur de 5 à 20 % en poids de matière sèche, en particulier de 15 à 20 %.

La température de réaction est ajustée à une valeur comprise entre 50 et 60°C, de préférence de l'ordre de 55°C.

En règle générale, le système d'enzyme ou une enzyme est ajouté à la suspension en des quantités dans la gamme d'environ 0,3 à 1 % poids/volume.

La réaction d'hydrolyse est typiquement effectuée dans une durée souhaitée afin d'obtenir le degré d'hydrolyse et/ou le profil désiré de poids moléculaire désiré, en l'occurrence ici pendant une période d'environ 45 minutes à environ 2 h 30 minutes, de préférence d'environ 1 heure.

Une fois encore, le temps nécessaire à la réaction d'hydrolyse dépend des caractéristiques comme indiqué ci-dessus, mais peut être facilement déterminée par l'homme du métier.

Dans d'autres réalisations, la suspension contenant des protéines de pois peut être hydrolysée en utilisant des moyens non enzymatiques, par exemple par hydrolyse mécanique (physique) et/ou chimique. Cette technique est également bien connue dans l'état de l'art.

Une fois que les protéines de pois ont été hydrolysées au degré souhaité, la réaction d'hydrolyse est arrêtée, par exemple, par inactivation de l'enzyme, ou par d'autres moyens classiques.

Dans un mode de réalisation, l'inactivation de l'enzyme est effectuée par traitement thermique.

Conformément à la pratique établie, la préparation d'enzyme peut convenablement être inactivée en augmentant la température de la suspension d'incubation à une température à laquelle les enzymes deviennent inactivées, par exemple à environ 70°C pendant environ 10 minutes.

Les isolats de protéines de pois ainsi obtenus sont ensuite traités à haute température courte durée (HTST), puis pasteurisés, éventuellement concentrés à une matière sèche de 10 à 30 %, avant d'être séchés par atomisation. Par exemple, l'isolat peut être pasteurisé à une température comprise entre 130°C et 150°C pendant une période d'environ 1 seconde à environ 30 secondes.

La présente divulgation est également relative à une formulation nutritionnelle comprenant un isolat de protéines de pois selon l'invention ainsi qu'à l'utilisation de cet isolat pour préparer une formulation nutritionnelle.

Les isolats de protéines de pois selon l'invention sont présents dans la formulation nutritionnelle décrite dans une quantité allant jusqu'à 100 % en poids, notamment, dans une quantité comprise entre 52 et 60 % en poids, en particulier de la formulation nutritionnelle. Par exemple, l'isolat de protéines de pois selon la présente invention peut représenter 0,1-10%, 10-20%, 20-30%, 40-50%, 50-60%, 60-70%, 70-80%, 80-90% ou 90-100% de la protéine totale de la formulation nutritionnelle, ou une quelconque combinaison de ces gammes de pourcentages.

Par ailleurs, l'isolat de protéines de pois selon la présente invention peut représenter 0,1-10%, 10-20%, 20-30%, 40-50%, 50-60%, 60-70%, 70-80%, 80-90% ou 90-100% en poids de la formulation nutritionnelle, ou une quelconque combinaison de ces gammes de pourcentages. De préférence, il représente 0,1-60%, 1-50%, 1-20% ou 1-10% ou une quelconque combinaison de ces gammes de pourcentages.

Dans un mode de réalisation particulier, l'isolat de protéines de pois selon la présente invention peut représenter 0,1-10%, 10-20%, 20-30%, 40-50%, 50-60%, 60-70%, 70-80%, 80-90% ou 90-100% en poids de la formulation nutritionnelle, ou une quelconque combinaison de ces gammes de pourcentages et il peut représenter 0,1-10%, 10-20%, 20-30%, 40-50%, 50-60%, 60-70%, 70-80%, 80-90% ou 90-100% de la protéine totale de la formulation nutritionnelle, ou une quelconque combinaison de ces gammes de pourcentages. De préférence, il représente 0,1-60%, 1-50%, 1-20% ou 1-10% ou une quelconque combinaison de ces gammes de pourcentages.

Au moins une partie des isolats de protéines de pois présents dans les formulations alimentaires en poudre est séchée par atomisation avant d'être introduite (via mélange à sec ou autre) dans la formulation nutritionnelle en poudre.

*Nature des autres ingrédients*

Les formulations en poudre nutritionnelles peuvent comprendre au moins une matière grasse, une protéine ou un hydrate de carbone, dans lesquelles au moins une partie de la protéine est un isolat de protéines de pois.

Les formulations liquides nutritionnelles peuvent comprendre au moins une protéine, hydrate de carbone et, matière grasse dans lesquelles au moins une partie de la protéine est un isolat de protéines de pois.

En général, une source de matières grasses, de glucides et de protéines, en plus de l'isolat de protéines de pois peut être utilisée ici, à condition que ces macronutriments soient également compatibles avec les éléments essentiels des formulations nutritionnelles selon la présence divulgation.

Bien que les concentrations totales ou les quantités de matière grasse, de protéines et d'hydrates de carbone peuvent varier selon les besoins nutritionnels de l'utilisateur, ces concentrations ou quantités tombent le plus souvent dans l'une des gammes suivantes, y compris de toute autre matière grasse essentielle, protéines, hydrates de carbone et ou des ingrédients tels que décrits ici :

1) pour les mix poudres pour boissons :

◦ les concentrations en matières grasses sont d'environ 0,5 % à environ 13 %, préférentiellement d'environ 1 % à environ 9 %, plus préférentiellement encore d'environ 1 % à environ 3 % en poids de la formulation nutritionnelle en poudre ;

◦ les concentrations en protéines sont d'environ 20 % à environ 91 %, préférentiellement d'environ 40 % à environ 90 %, plus préférentiellement encore comprise entre environ 40 % et environ 65 % en poids de la formulation nutritionnelle en poudre;

◦ les concentrations en glucides sont d'environ 0,9 % à environ 70 %, soit préférentiellement entre environ 2 % à environ 7 %, soit préférentiellement encore entre environ 20 % à environ 40 %, en poids de la formulation nutritionnelle en poudre.

2) pour les liquides :

◦ les concentrations en matières grasses sont d'environ 1% à environ 10 %, préférentiellement d'environ 1,5 % à environ 7 %, plus préférentiellement encore d'environ 1.5 % à environ 5 %, en poids de la formulation nutritionnelle en liquide ;

◦ les concentrations en protéines sont d'environ 1 % à environ 15 %, préférentiellement d'environ 3 % à environ 11 %, plus préférentiellement encore d'environ 4 % à environ 7 %, en poids de la formulation nutritionnelle en liquide ;

◦ les concentrations en glucides sont d'environ 5 % à environ 45 %, préférentiellement d'environ 9 % à environ 20 %, plus préférentiellement encore d'environ 13 % à environ 17 %, en poids de la formulation nutritionnelle en liquide.

3) pour les produits laitiers (sous forme de yaourts, boissons laitières, crèmes laitières, desserts glacés ou sorbets):

◦ les concentrations en matières grasses sont d'environ 0% à environ 15 %, préférentiellement d'environ 1,5 % à environ 10 %, plus préférentiellement encore d'environ 3 % à environ 6 %, en poids de la formulation nutritionnelle en liquide ;

◦ les concentrations en protéines sont d'environ 1 % à environ 25 %, préférentiellement d'environ 2 % à environ 20 %, plus préférentiellement encore d'environ 2,5 % à environ 15 %, en poids de la formulation nutritionnelle en liquide ;

◦ les concentrations en glucides sont d'environ 5 % à environ 45 %, préférentiellement d'environ 9 % à environ 25 %, plus préférentiellement encore d'environ 13 % à environ 20 %, en poids de la formulation nutritionnelle en liquide.

4) pour les produits de biscuiterie, produits de pâtisserie, produits de panification et produits céréaliers hyperprotéinés:

◦ les concentrations en matières grasses sont d'environ 0% à environ 25 %, préférentiellement d'environ 1 % à environ 20 %, plus préférentiellement encore d'environ 10 % à environ 18 %, en poids de la formulation nutritionnelle en liquide ;

◦ les concentrations en protéines sont d'environ 1 % à environ 30 %, préférentiellement d'environ 2 % à environ 25 %, plus préférentiellement encore d'environ 2,5 % à environ 15 %, en poids de la formulation nutritionnelle en liquide ;

◦ les concentrations en glucides sont d'environ 15 % à environ 75 %, préférentiellement d'environ 20 % à environ 60 %, plus préférentiellement encore d'environ 20 % à environ 55 %, en poids de la formulation nutritionnelle en liquide.

Des exemples non limitatifs de matières grasses (en poudre ou liquide) ou des sources convenables de celles-ci pour une utilisation dans les formulations alimentaires en poudre et liquides décrits ici comprennent l'huile de noix de coco, l'huile de noix de coco fractionnée, l'huile de soja, l'huile de maïs, l'huile d'olive, l'huile de carthame, l'huile de carthame riche en acide oléique, huile de tournesol, huile de tournesol riche en acide oléique, les huiles de palme et de palmiste, l'oléine de palme, huile de canola, huiles marines, huiles de coton, matières grasses d'origine laitière, et leurs combinaisons.

Des exemples non limitatifs d'hydrates de carbone ou des sources convenables de ceux-ci pour une utilisation dans les formulations alimentaires en poudre et liquides décrits ici peuvent comprendre les maltodextrines, les dextrines, l'amidon hydrolysé ou modifié ou l'amidon de maïs, les polymères de glucose, le sirop de maïs, les hydrates de carbone dérivés du riz, le glucose, le fructose, le lactose, le sirop à haute teneur en fructose, le miel, les alcools de sucre (par exemple, le maltitol, l'érythritol, le sorbitol), et des combinaisons de ceux-ci.

Des exemples non limitatifs de protéines, en plus des isolats de protéines de pois, pour une utilisation dans les formulations alimentaires en poudre et liquides comprennent des protéines hydrolysée, partiellement hydrolysées ou les protéines ou les sources de protéines non hydrolysées, qui peuvent être dérivées de toute source connue, tel que du lait (par exemple, la caséine, le lactosérum), des animaux (par exemple, viande, poisson), des céréales (par exemple, le riz, le maïs), des oléagineux (soja, colza), des légumineuses à grains (lentilles, pois chiches, haricots) ou des combinaisons de ceux-ci.

Des exemples non limitatifs de telles protéines comprennent des isolats de protéines de lait, des concentrés protéiques de lait, tels que les concentrés de protéines de lactosérum, caséine, isolats de protéines de lactosérum, les caséinates, le lait de vache entier, lait écrémé, protéine de soja isolats de protéine partiellement ou totalement hydrolysées, protéines de soja concentrées, et ainsi de suite.

Dans un mode de réalisation particulier, la formulation nutritionnelle en poudre comprend une combinaison d'un isolat de protéines de pois et d'une protéine à base de lait.

Dans un exemple de ce mode de réalisation particulier, la protéine à base de lait est présente dans la formulation nutritionnelle en poudre en une quantité d'au moins 10, 15, 20, 25, 30, 40, 45 ou 50 % en poids par rapport au poids totale de protéine, de préférence d'environ 45 % en poids par rapport au poids totale de protéine. Par exemple, la protéine à base de lait est présente dans la formulation nutritionnelle en poudre en une quantité de 10-60 %, 20-50%, 30-40% en poids par rapport au poids totale de protéine. De préférence, le reste des protéines est apporté par l'isolat de protéines de pois selon l'invention.

Dans un autre exemple de ce mode de réalisation particulier, la protéine à base de lait est présente dans la formulation nutritionnelle en liquide pour la nutrition clinique en une quantité d'au moins 10, 15, 20, 25, 30, 40, 45 ou 50 % en poids par rapport au poids totale de protéine, de préférence d'environ 50 % en poids. Par exemple, la protéine à base de lait est présente dans la formulation nutritionnelle en liquide pour la nutrition clinique en une quantité de 10-60 %, 20-50%, 30-40% ou 45-55% en poids par rapport au poids totale de protéine. De préférence, le reste des protéines est apporté par l'isolat de protéines de pois selon l'invention.

Dans un autre exemple de ce mode de réalisation particulier, la protéine à base de lait est présente dans la formulation nutritionnelle en liquide pour le sport en une quantité d'au moins 10, 15, 20, 25, 30, 40, 50, 60 ou 75 % en poids par rapport au poids totale de protéine, de préférence d'environ de 75 % en poids. Par exemple, la protéine à base de lait est présente dans la formulation nutritionnelle en liquide pour le sport en une quantité de 10-60 %, 20-50%, 30-40% ou 45-55% en poids par rapport au poids totale de protéine. De préférence, le reste des protéines est apporté par l'isolat de protéines de pois selon l'invention.

*Nature des ingrédients facultatifs*

Les formulations nutritionnelles selon la présente divulgation peuvent comprendre en outre d'autres ingrédients qui peuvent modifier les caractéristiques chimiques, physiques, hédoniques ou de transformation des produits ou servir de composants nutritionnels pharmaceutiques ou complémentaires lorsqu'elles sont utilisées pour certaine population ciblée.

Beaucoup de ces ingrédients facultatifs sont connus ou autrement adaptés pour une utilisation dans d'autres produits alimentaires et peuvent également être utilisés dans les formulations nutritionnelles décrites, à condition que ces ingrédients facultatifs soient sûrs et efficaces pour l'administration orale et sont compatibles avec les ingrédients essentiels autres du produit sélectionné.

Des exemples non limitatifs de tels ingrédients facultatifs comprennent des conservateurs, des antioxydants, des agents émulsifiants, des agents tampons, des agents actifs pharmaceutiques, des nutriments supplémentaires, des colorants, des arômes, des agents épaississants et des stabilisants, etc.

Les formulations nutritionnelles en poudre ou liquide peuvent comprendre en outre des vitamines ou des nutriments liés, tels que la vitamine A, la vitamine E, la vitamine K, la thiamine, la riboflavine, la pyridoxine, la vitamine B12, les caroténoïdes, la niacine, l'acide folique, l'acide pantothénique, la biotine, la vitamine C, la choline, l'inositol, leurs sels et leurs dérivés, et des combinaisons de ceux-ci.

Les formulations nutritionnelles en poudre ou liquide peuvent comprendre en outre des minéraux, tels que de phosphore, le magnésium, le fer, le zinc, le manganèse, le cuivre, le sodium, le potassium, le molybdène, le chrome, le sélénium, le chlorure, et des combinaisons de ceux-ci.

Les formulations nutritionnelles en poudre ou liquide peuvent également comprendre un ou plusieurs agents masquant pour réduire par exemple les saveurs amères dans les poudres reconstituées.

Des agents de masquage appropriés comprennent des édulcorants naturels et artificiels, des sources de sodium, telles que le chlorure de sodium, et des hydrocolloïdes tels que la gomme de guar, la gomme xanthane, la carraghénane, et des combinaisons de ceux-ci.

La quantité d'agent de masquage dans la formulation nutritionnelle en poudre peut varier en fonction de l'agent de masquage particulier sélectionné, les autres ingrédients de la formulation et d'autres variables de formulation ou de produits cibles.

*Procédé de fabrication de la formulation nutritionnelle en poudre selon* la présente divulgation

La poudre de base nutritive (comprenant l'isolat de protéines de pois selon l'invention) peut être préparée par mélange à sec de tous les ingrédients eux-mêmes sous forme poudre.

En variante, la poudre de base nutritive peut être préparée en utilisant des procédés conventionnels en voie humide qui comprennent généralement l'utilisation de deux ou plusieurs suspensions qui sont finalement mélangées, traitées puis séchées.

Au moins une partie de la protéine végétale présente dans la formulation nutritionnelle en poudre mélangée à sec est un isolat de protéines de pois qui a avantageusement été séché par atomisation avant d'être mélangé à sec avec la poudre de base nutritionnelle, laquelle comporte généralement au moins des glucides, des vitamines et des minéraux.

Dans certains modes de réalisation, l'isolat de protéines de pois peut être traité à haute température courte durée (HTST) puis pasteurisé avant d'être séché par atomisation.

Plus précisément, l'isolat de protéines de pois peut être ajouté à l'eau et on le laisse s'hydrater; l'eau peut ou non être chauffée.

Cette suspension est ensuite traitée HTST avant d'être séchée par atomisation. Facultativement, l'isolat de protéines de pois peut être homogénéisé classiquement après le traitement HTST et avant le séchage par atomisation. Par exemple, l'isolat peut être pasteurisé à une température comprise entre 130°C et 150°C pendant une période d'environ 1 seconde à environ 30 secondes.

L'étape de séchage par atomisation est une étape de séchage par atomisation classique qui est effectuée à des températures et des temps bien connus, conventionnels, pour produire une protéine végétale séchée par atomisation.

*Domaine d'utilisation des formulations nutritionnelles en poudre*

Les formulations nutritionnelles en poudre mélangées à sec décrites et comprenant des isolats de protéines de pois conformes à l'invention, après reconstitution, présentent une sensation en bouche améliorée.

Un individu peut consommer de préférence au moins une portion de la formulation nutritionnelle en poudre reconstituée de façon quotidienne, et dans certains modes de réalisation, peut consommer deux, trois ou même plus de portions par jour.

Chaque portion est de préférence administrée en une dose unique, bien que la portion puisse aussi être divisée en deux ou plusieurs portions partielles, à prendre deux fois ou plus au cours de la journée.

Les formulations nutritionnelles en poudre peuvent être reconstituées pour une utilisation chez les nourrissons, les enfants et les adultes.

Par « environ » est entendu la valeur plus ou moins 10 %, de préférence plus ou moins 5%.

**DESCRIPTION DES FIGURES**

**Figure 1** : Distribution de la taille des particules de l'émulsion que forme les formules nutritionnelles de nutrition clinique décrites

**Figure 2** : Analyse sensorielle de mélanges de poudres formulés avec des isolats de protéines de pois conformes à l'invention

**Figure 3** : Analyse sensorielle de boissons « prêtes-à-boire » pour la nutrition clinique.

**Figure 4** : Suivi de la viscosité au cours de la digestion in vitro des isolats de protéines de pois selon l'invention

**Figure 5** : Profil de solubilité des isolats de protéines de pois en fonction du pH

**Figure 6** : Analyse sensorielle de boissons « prêtes-à-boire » pour sportif.

**Figure 7** : Analyse sensorielle des crèmes dessert pour nutrition clinique.

**Figure 8** : Distribution des tailles des globules gras de l'émulsion préparée avec 100 % de protéines de lait pour une préparation pour desserts glacés

**Figure 9** : Distribution des tailles des globules gras de l'émulsion préparée avec 50 % de protéines de lait et 50 % de la protéine de pois NUTRALYS® S85F pour une préparation pour desserts glacés

**Figure 10 :** Distribution des tailles des globules gras de l'émulsion préparée avec 50 % de protéines de lait et 50 % d'isolat de protéines de pois selon l'invention n°1 pour une préparation pour desserts glacés

**Figure 11** : Distribution des tailles des globules gras de l'émulsion préparée avec 50 % de protéines de lait et 50 % d'isolat de protéines de pois selon l'invention n°2 pour une préparation pour desserts glacés

**Figure 12** : profil de fonte de crèmes glacées végétaliennes préparées avec les isolats de protéines de pois selon l'invention
**Figure 13** : Analyse sensorielle des desserts glacés
**Figure 14** : solubilité des isolats de protéines de pois en fonction du pH en comparaison avec les caséinates de sodium
**Figure 15** : Analyse sensorielle des yaourts brassés - Aspects Goût
**Figure 16 :** Analyse sensorielle des yaourts brassés - Aspects Texture
**Figure 17** : Analyse sensorielle des boissons laitières aromatisées à la fraise : aspect goût
**Figure 18 :** Analyse sensorielle des boissons laitières aromatisées à la fraise : aspect texture
**Figure 19** : Analyse de la viscosité des pâtes de muffins au chocolat
**Figure 20** : Analyse de la viscosité des pâtes de pancakes
**Figure 21** : Analyse de digestibilité par le suivi des viscosités selon le dispositif SIMPHYD de NIZO

L'invention sera mieux comprise à l'aide des exemples qui suivent, lesquels se veulent illustratifs et non limitatifs.

## EXEMPLES

### Matériels et méthodes

### *Mesure du DH (Degré d'Hydrolyse)*

Cette mesure est basée sur la méthode de détermination de l'azote aminé sur des protéines et isolats de protéines selon l'invention par le kit MEGAZYME (référence K-PANOPA) et le calcul du degré d'hydrolyse.

*Principe :*

Les groupes « azote aminé » des acides aminés libres de l'échantillon réagissent avec le N-acétyl-L-cystéine et l'OPhthaldialdéhyde (OPA) pour former des dérivés d'isoindole.
La quantité de dérivé d'isoindole formée au cours de cette réaction est stoechiométrique avec la quantité d'azote aminé libre. C'est le dérivé d'isoindole qui est mesuré par l'augmentation de l'absorbance à 340 nm.

*Mode opératoire* :

Dans un bécher de 100 ml, introduire une prise d'essai P*, exactement pesée, de l'échantillon à analyser. (Cette prise d'essai sera de 0,5 à 5,0 g en fonction de la teneur en azote aminé de l'échantillon.)
Ajouter environ 50 ml d'eau distillée, homogénéiser et transvaser dans une fiole jaugée de 100 ml, ajouter 5ml de SDS à 20% et amener au volume avec de l'eau distillée ; agiter pendant 15 minutes sur l'agitateur magnétique à 1000 rpm.
Dissoudre 1 comprimé du flacon 1 du kit Megazyme dans 3 ml d'eau distillée et agiter jusqu'à dissolution complète. Prévoir un comprimé par essai.
Cette solution n°1 est à préparer extemporanément.
La réaction se fait directement dans les cuves de spectrophotomètre.

∘ Blanc :
Introduire 3,00 ml de la solution n°1 et 50 µl d'eau distillée.
∘ Standard :
Introduire 3,00 ml de la solution n°1 et 50 µl du flacon 3 du kit Megazyme.

∘ Echantillon :

Introduire 3,00 ml de la solution n°1 et 50 µl de la préparation de l'échantillon.
Mélanger les cuves et lire les mesures d'absorbance (A1) des solutions après 2 mn environ au spectrophotomètre à 340 nm (spectrophotomètre équipé de cuves de 1,0 cm de trajet optique, pouvant mesurer à une longueur d'onde de 340 nm, et vérifié selon le mode opératoire décrit dans le manuel technique du constructeur qui s'y rapporte).
Amorcer ensuite les réactions immédiatement en ajoutant 100 µl de la solution OPA flacon 2 du kit Megazyme dans les cuves de spectrophotomètre.
Mélanger les cuves et les placer environ 20 minutes dans l'obscurité.
Faire ensuite la lecture des mesures d'absorbance du blanc, du standard et des échantillons au spectrophotomètre à 340 nm.

*Mode de calcul :*

La teneur en azote aminé libre, exprimée en pourcentage en masse de produit tel quel, est donnée par la formule suivante :

$$[\text{NH}_2\ \%brut] = \frac{(\Delta A_{ech} - \Delta A_{blc}) \times 3{,}15 \times 14{,}01 \times V \times 100}{6803 \times 0{,}05 \times 1000 \times m}$$

$$= \frac{(\Delta A_{ech} - \Delta A_{blc}) \times 12{,}974 \times V}{m \times 1000}$$

Où : $\Delta A = A2 - A1$
V = Volume de la fiole
m = masse de la prise d'essai en g
6803 = coefficient d'extinction du dérivé d'isoindole à 340 nm (en $L.mol^{-1}.cm^{-1}$).
14,01 = masse molaire de l'Azote (en $g.mol^{-1}$)
3,15 = volume final dans la cuve (en ml)
0,05 = prise d'essai dans la cuve (en ml)

Le degré d'hydrolyse (DH) est donné par la formule :

$$DH = \frac{\text{Azote aminé (\%)}}{\text{Azote protéique (\%)} \times 100}$$

où l'azote protéique est déterminé selon la méthode de DUMAS selon la norme ISO 16634.

**Mesure de la solubilité dans l'eau à différents pH**

Cette mesure est basée sur la dilution de l'échantillon dans de l'eau distillée, sa centrifugation et l'analyse du surnageant.

*Mode opératoire :*

Dans un bécher de 400 ml, introduire 150 g d'eau distillée à une température de 20°C +/-2°C, mélanger avec un barreau magnétique et ajouter précisément 5 g de l'échantillon à tester.
Ajuster ou non le pH à la valeur souhaitée avec NaOH 0,1 N.
Compléter le contenu en eau à 200 g.
Mélanger pendant 30 minutes à 1000 rpm et centrifuger pendant 15 minutes à 3000 g.
Collecter 25 g du surnageant.
Introduire dans un cristallisoir préalablement séché et taré.
Placer dans une étuve à 103°C +/- 2°C pendant 1 heure.
Placer ensuite dans un dessiccateur (avec agent déshydratant) pour refroidir à température ambiante et peser.
Le contenu en matières sèches solubles, exprimé en % en poids, est donné par la formule suivante :

$$\frac{(m1 - m2) \times (200 + P) \times 100}{P1 \times P} = \%\ \text{de solubilité}$$

Où :

◦ P = poids, en g, de l'échantillon = 5 g
◦ m1 = poids, en g, du cristallisoir après séchage
◦ m2 = poids, en g, du cristallisoir vide
◦ P1 = poids, en g, de l'échantillon collecté = 25 g

### *Mesure de la digestibilité in vitro*

Le dispositif SIMPHYD de NIZO est un modèle statique de simulation des processus de digestion le long du tractus gastrointestinal.

La digestion gastrique est combinée avec une mesure de viscosité en ligne au cours du temps. Adaptée aux conditions physiologiques, l'acidification gastrique est initiée avec de l'HCl concentrée et les enzymes de digestion enzymatique (pepsine et lipase) sont ajoutées.

Tous les échantillons sont soumis au dispositif SIMPHYD à la concentration de 3 % (m/v).

Les mesures sont réalisées comme suit :

◦ On détermine une ligne de base de viscosité pendant 5 minutes, à pH naturel et à 37°C
◦ On acidifie ensuite à pH 2 avec de l'HCl et maintient à 37°C pendant 15 minutes,
◦ On ajoute pepsine et lipase à 20 minutes.

La viscosité est suivie pendant 3 heures, à l'aide d'un rhéomètre TA Instruments AR-2000 sous vitesse de cisaillement de 75 s$^{-1}$.

Les mesures sont réalisées en double. Si l'écart entre deux mesures est trop important, une troisième mesure est réalisée.

Le profil des protéines testées est comparé avec ceux établis par Hall et al (article de 2003 intitulé Casein and whey exert different effects on plasma amino acid profiles, gastrointestinal hormone sécrétion ans appetite publié dans Br. J. Nutr. 89 : 239-248) pour des protéines dites « rapides » et « lentes » (respectivement protéines de lactosérum et caséinates de sodium).

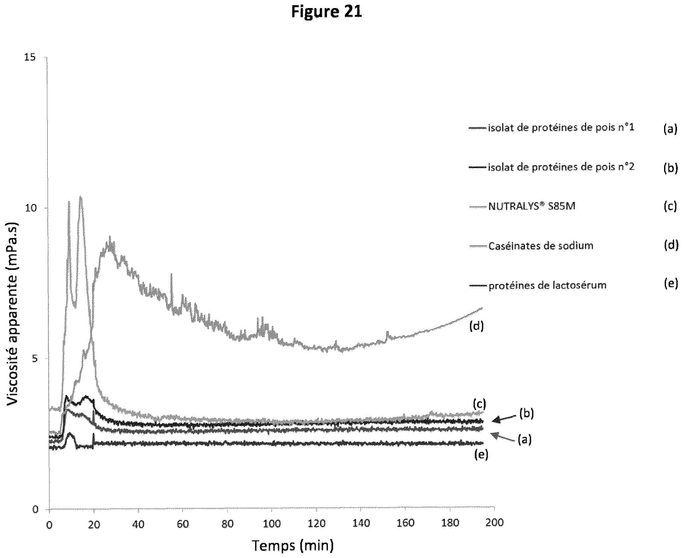

Les profils des viscosités obtenues sont présentés dans la **Figure 21.**

La viscosité apparente de l'échantillon de protéines de lactosérum témoin ne change pas durant le processus gastrique, tandis que la viscosité apparente du témoin caséinates de Na augmente après l'acidification gastrique et reste élevée après l'ajout des enzymes digestives.

Après 5 minutes d'acidification, les protéines de pois (NUTRALYS® S85M) affichent un premier pic de viscosité, suivi d'un second à 15 minutes, puis le profil de viscosité rejoint celui des protéines de lactosérum, à des valeurs légèrement supérieures.

La viscosité commence à chuter avant l'ajout des enzymes digestives.

Les isolats de protéines de pois selon l'invention montrent une très petite augmentation en viscosité apparente, qui diminue à nouveau à des valeurs légèrement au-dessus de celles des protéines de lactosérum, pendant 30 minutes.

Le comportement des isolats de protéines de pois selon l'invention traduit leur caractère « rapide » caractéristique de protéines plus satiétogènes que les protéines dites « lentes ». Ce qui induit un vidage gastrique plus rapide et une augmentation post-absorptive des aminoacides plasmiques.

### *Mesure du pouvoir émulsifiant*

Comme indiqué ci-avant, les mesures sont effectuées par diffusion de la lumière de poudre de protéines remise en solution, les émulsions obtenues étant analysées au granulomètre pour la taille des globules gras formés.

Les résultats sont exprimés par :

◦ Le Dmode, diamètre de la population principale,
◦ Le D(4,3), diamètre moyen arithmétique
◦ Les D10, D50 et D90, diamètres pour lesquels il y a 10 %, 50 % et 90 % de passants.

Le tableau ci-après regroupe la taille des globules gras des émulsions réalisés à partir :

◦ Des deux isolats de protéines de pois conformes à l'invention n°1 et n°2,
◦ De différentes protéines de lait
◦ D'un lot de caséinates de sodium.

Le ΔD correspond à la différence entre le D90et le D10 ; il traduit l'état de dispersion des émulsions.

Plus cette valeur est petite, plus les tailles des gouttelettes sont proches, et plus l'émulsion est homogène.

| | Capacité émulsifiante : taille d'émulsion (Dmode en μm) | | | | | Stabilité de l'émulsion (ΔD) |
|---|---|---|---|---|---|---|
| | Dmode | D(4,3) | D10 | D50 | D90 | |
| isolat de protéines de pois selon l'invention n°1 | 23,4 | 20,5 | 2,1 | 19,4 | 38,4 | 36,3 |
| isolat de protéines de pois selon l'invention n°2 | 23,6 | 21,4 | 3,4 | 20,1 | 39,1 | 35,7 |
| Lait écrémé | 24,9 | 20,9 | 5,9 | 20,1 | 36,2 | 30,3 |
| Protéine de lait MPC de FONTERRA | 32,8 | 28,1 | 8,7 | 27,8 | 46,3 | 37,6 |
| Protéines de lait MPC 80 Domo DMV | 25,5 | 21,8 | 6,5 | 21,1 | 37,3 | 30,8 |
| Protéine de lait MPI Prodiet 87B de INGREDIA | 25,4 | 22,6 | 6,9 | 21,4 | 39,1 | 32,2 |
| Caséinates de sodium de DMV | 31,9 | 25,3 | 6,3 | 25,3 | 43,6 | 37,3 |

Les isolats de protéines de pois selon l'invention présentent :

○ de bonnes propriétés émulsifiantes (Dmode plus faible : 23,4 et 23,6 μm, respectivement)
○ Une stabilité d'émulsion (ΔD) du même ordre voire plus faible que certaines protéines de lait concentrées ou que les caséinates de sodium et
○ une homogénéité d'émulsion équivalente aux protéines de lait.

Leurs propriétés les rendent par ailleurs tout à fait transposable aux applications où un certains pouvoir émulsifiant est recherché comme les préparations de desserts glacés, non dairy coffee whitener, pour lesquelles les caséinates sont recherchés.

**Exemple 1 : préparation des isolats de protéines de pois selon l'invention et caractérisation des isolats de protéines de pois référencés « 1 » et « 2 » selon l'invention**

*Procédé de préparation des isolats de protéines de pois selon l'invention n° 1*

On délaye 1500 kg de protéine de pois (commercialisée par la société demanderesse sous le nom de marque NUTRALYS® S85F) dans 8500 litres d'eau préchauffée à 55°C.
On agite durant 3 h à 55°C.
On ajoute 0,5 % (poids/poids) de d'endoprotéase FLAVORPRO 750 MDP (de la société BIOCATALYST).
On agite durant 1 h à 55°C.
Le degré hydrolyse obtenu est alors de 7.
On inhibe la réaction par chauffage du milieu à 70°C et on maintient à cette température pendant 10 minutes minimum.
On applique un traitement UHT (barème 140°C - 10 s).
On sèche par atomisation à une matière sèche d'environ 93%.

*Procédé de préparation des isolats de protéines de pois selon l'invention n°2*

On délaye 1500 kg de protéine de pois (commercialisée par la société demanderesse sous le nom de marque NUTRALYS® S85F) dans 8500 litres d'eau préchauffée à 55°C.
On agite durant 3 h à 55°C.
On ajoute 0,3 % (poids/poids) d'endoprotéase ENZECO FUNGAL PROTEASE (de la société EDC).
On agite durant 1h à 55°C, le degré hydrolyse obtenu est alors de 6,5.
On inhibe la réaction enzymatique par chauffage du milieu à 70°C et maintient durant 10 minutes minimum.
On applique un traitement UHT (barème 140°C - 10 s).

On sèche par atomisation ensuite à une matière sèche d'environ 93%.

*Caractéristiques des isolats de protéines de pois ainsi préparés*

*1. Teneur en acides aminés libres*

Mesures effectuées selon la norme NF EN ISO13903:2005

|  | Acides aminés libres / somme des acides aminés (g/100 g brut en %) |
|---|---|
| NUTRALYS ® S85F | 0,18 |
| isolat de protéines de pois selon l'invention n°1 | 0,77 |
| isolat de protéines de pois selon l'invention n°2 | 1,85 |

*2. Profil de viscosité*

Pour la détermination du profil de viscosité dans l'eau, les mesures sont effectuées

○ sur une solution aqueuse d'isolats de protéines de pois à 15 % de matière sèche (eau osmosée et azidurée à 200 ppm pour prévenir tout risque bactériologique),
○ en rhéomètre AR2000 de la société TA Instruments,
○ présentant une géométrie à cylindres concentriques,
○ avec un taux de cisaillement de 0,6 $10^{-3}$ à 600 $s^{-1}$ en 3 minutes (log) et
○ à la température de 20°C (3 min d'équilibre température avant test).

Avant mesure, la solution est agitée pendant au moins 10 heures, à 750 tr/min et à 20°C.
Le pH n'est pas ajusté.
Le tableau suivant permet de comparer les profils de viscosité des isolats de protéines de pois conformes à l'invention, en regard de ceux des protéines de lait contrôle et de la protéine de pois NUTRALYS® S85F.

| Solution à 15% de MS | Viscosité en Pa.s à 20°C, pH tel quel | | | | | | |
|---|---|---|---|---|---|---|---|
| référence échantillon | 5 $s^{-1}$ | 10 $s^{-1}$ | 20 $s^{-1}$ | 40$s^{-1}$ | **100$s^{-1}$** | **200$s^{-1}$** | **600 $s^{-1}$** |
| Protéine de pois NUTRALYS® S85F | 15,820 | 9,538 | 5,700 | 3,570 | 1,900 | 1,251 | 0,680 |
| Protéine de lait MPC 4882 de FONTERRA | 0,030 | 0,032 | 0,030 | 0,029 | 0,029 | 0,028 | 0,026 |
| Protéine de lait Prodiet 27B fluide de INGREDIA | 0,022 | 0,021 | 0,019 | 0,017 | 0,015 | 0,014 | 0,013 |
| Isolat de protéines de pois selon l'invention n°1 | 0,013 | 0,013 | 0,0135 | 0,011 | 0,010 | 0,010 | 0,010 |
| Isolat de protéines de pois selon l'invention n°2 | 0,015 | 0,016 | 0,016 | 0,014 | 0,013 | 0,014 | 0,014 |

On constate que les isolats de protéines de pois conformes à l'invention présentent un comportement Newtonien comme celui des protéines de lait alors que la protéine de pois NUTRALYS® S85F a un comportement rhéofluidifiant très marqué.
De plus, les viscosités des isolats de protéines de pois n°1 et 2 sont très proches des viscosités des protéines de lait, voire mêmes plus basses.

*3. Profil de solubilité dans l'eau en fonction du pH*

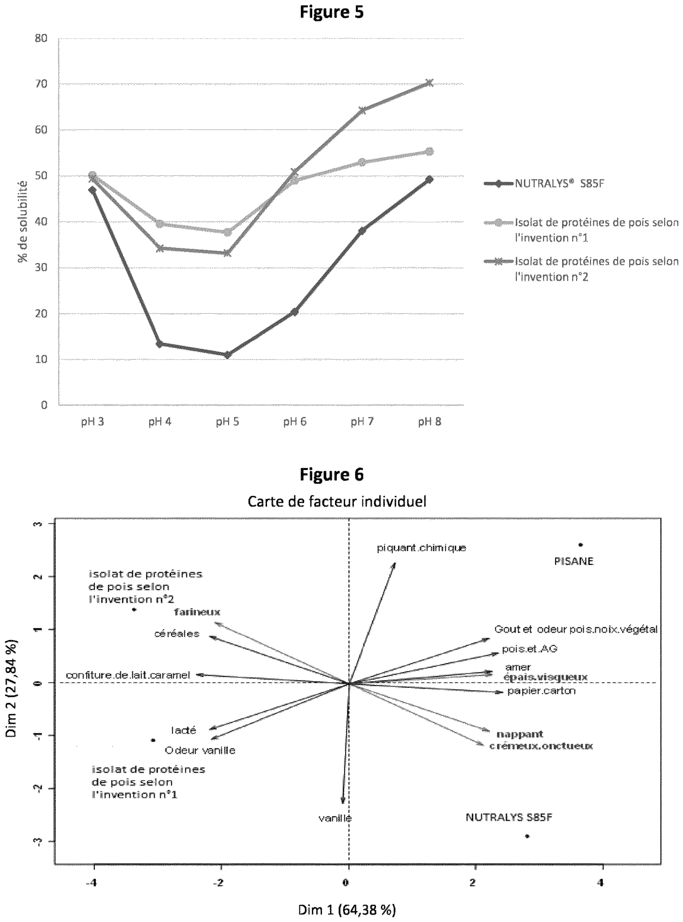

Les résultats sont présentés dans le tableau suivant et sont illustrés par la **Figure 5.**

| | Protéine de pois NUTRALYS® S85F | Isolat de protéines de pois selon l'invention n°1 | Isolat de protéines de pois selon l'invention n°2 |
|---|---|---|---|
| Solubilité en % | Moyenne sur 7 échantillons | | |
| pH 3 | 47 | 50 | 49 |
| pH 4 | 13 | 39 | 34 |
| pH 5 | 11 | 38 | 33 |
| pH 6 | 20 | 49 | 51 |
| pH 7 | 38 | 53 | 64 |
| pH 8 | 49 | 55 | 70 |

*4. Etude de stabilité*

Une étude de stabilité dans le temps des isolats de protéines de pois conformes à l'invention est menée afin de mesurer leur comportement en regard des protéines de pois intactes.

L'étude est menée après stockage 6 mois selon un barème température / humidité relative de :

∘ 40°C +/- 2°C

∘ à 75 % +/- 5 % d'humidité relative.

Les mesures sont exprimées en % de perte de solubilité (mesurée selon le mode opératoire ci-avant).

| | Isolat de protéines de pois selon l'invention n°1 | Isolat de protéines de pois selon l'invention n°2 | Protéine de pois NUTRALYS® S85F |
|---|---|---|---|
| Solubilité en % | Après 6 mois de stockage | | |
| pH 3 | -12,2 | -27,7 | -44,0 |
| pH 4 | -10,8 | -14,8 | -18,4 |
| pH 5 | -6,9 | -9,8 | - 5,2 |
| pH 6 | -8,5 | -14,7 | -19,6 |
| pH 7 | -8,8 | -19,9 | -47,4 |
| pH 8 | -5,9 | -22,0 | -57,9 |

On constate ainsi qu'à pH7, le NUTRALYS® S85F perd environ la moitié de sa solubilité alors que les isolats de protéines de pois ne perdent qu'au maximum 1/5 de leur solubilité, et conservent dans tous les cas une solubilité supérieure à celle du NUTRALYS® S85F initial.

*4-Profil de digestibilité*

Le but de cette étude est d'évaluer la digestibilité protéique totale des isolats de protéines de pois selon l'invention n°1 et 2 et de la comparer au NUTRALYS® S85F.

Pour cette étude, 48 rats Sprague Dawley (Charles River, Lyon, France) de 100 - 125 grammes au début de l'étude ont été randomisés en fonction de leur poids dans 4 groupes de 12 rats.

Cette expérience a été conduite en accord avec la législation européenne d'expérimentation animale et dans le respect du bien-être animal (projet APAFIS n° 0000501).

A leur arrivée, les rats ont subi une période de quarantaine de 7 jours pendant laquelle ils ont reçu un aliment standard pour rats en croissance.

A partir du premier jour d'étude, les rats ont reçu les régimes suivants et ce, pour 10 jours :

| | Contrôle | Protéine de pois NUTRALYS® S85F | Isolat de protéines de pois selon l'invention n°1 | Isolat de protéines de pois selon l'invention n°2 |
|---|---|---|---|---|
| | en % | en % | en % | en % |
| Produit à tester | 0 | 12,5 | 12,4 | 12,5 |
| Cellulose microcristalline de MB Biomedicals | 5 | 5 | 5 | 5 |
| Amidon de maïs | 72,7 | 60,2 | 60,2 | 60,2 |
| qsp 100 % en amidon de maïs | 12,9 | 0,39 | 0,44 | 0,4 |
| Huile de soja - Huileries de Sérignan | 7,5 | 7,5 | 7,5 | 7,5 |
| Saccharose | 10 | 10 | 10 | 10 |
| Maltodextrine GLUCIDEX® IT21 de ROQUETTE FRERES | 0 | 0 | 0 | 0 |
| Choline bitartrate | 0,25 | 0,25 | 0,25 | 0,25 |
| t-butyl-hydroquinone | 0,0018 | 0,0018 | 0,0018 | 0,0018 |
| Mélange de minéraux AIN-93G de MP Biomedicals | 3,5 | 3,5 | 3,5 | 3,5 |
| Mélange de vitamines AIN-93-VX de MP Biomedicals | 1 | 1 | 1 | 1 |

Les quantités étant indiquées en pourcentage en poids.

Les consommations d'aliment, de boisson et l'évolution pondérale sont suivis les premier et cinquième jours d'étude puis de façon quotidienne jusqu'au dixième et dernier jour d'étude.

Lors des 5 derniers jours d'étude, urines et fèces sont aussi collectés de façon quotidienne. Les taux protéiques des aliments et fèces sont déterminés par la méthode Kjeldahl (norme ISO 1871 :2009).

Les analyses de l'azote des fèces et aliments permettra de calculer le Coefficient d'Utilisation Digestive (CUD) :

$$\text{CUD (\%)} = \frac{[quantité.absorbée - quantité.excrétée.dans.les.fèces]}{quantité.absorbée}$$

Tous les rats ont eu la croissance attendue. Elle était significativement plus faible dans le groupe contrôle déficient en protéine, comme toujours dans ce schéma expérimental.

La consommation de boisson n'a pas été modifiée par les différents régimes.

Les modifications des autres paramètres urinaires et fécaux sont directement liées au régime contrôle ou expérimental.

En fonction des différents jours expérimentaux, on a pu calculer les digestibilités suivantes :

| Régime | Digestibilité (%) CUD | J6 | J7 | J8 | J9 | J10 | | Moyenne |
|---|---|---|---|---|---|---|---|---|
| | n | 12 | 12 | 12 | 12 | 12 | | 12,00 |
| Protéine de pois NUTRALYS® S85F | moyenne | **96,5** | **96,3** | **95,3** | **95,0** | **95,8** | | **95,8** |
| | écartype | 2,6 | 1,4 | 1,4 | 1,7 | 1,9 | | 0,8 |

(suite)

| Régime | Digestibilité (%) CUD | J6 | J7 | J8 | J9 | J10 | | Moyenne |
|---|---|---|---|---|---|---|---|---|
| Isolat de protéines de pois selon l'invention n°1 | n | 12 | 12 | 12 | 12 | 12 | | 12,00 |
| | moyenne | **94,6** | **94,7** | **93,8** | **92,1** | **93,8** | | **93,8** |
| | écartype | 4,6 | 2,1 | 1,8 | 3,6 | 3,5 | | 1,3 |
| Isolat de protéines de pois selon l'invention n°2 | n | 12 | 12 | 12 | 12 | 12 | | 12,00 |
| | moyenne | **95,2** | **95,7** | **95,2** | **93,2** | **94,6** | | **94,8** |
| | écartvpe | 2,9 | 1,9 | 2,1 | 2,8 | 1,8 | | 1,0 |

D'un point de vue statistique, la digestibilité protéique du NUTRALYS® S85F est significativement différente de celle de l'isolat de protéines de pois selon l'invention n°1 (p = 0.0003).

Néanmoins, d'un point de vue biologique, ces différences ne sont absolument pas significatives.

On peut donc conclure à des digestibilités similaires entre le NUTRALYS et les isolats de protéines de pois selon l'invention n°1 et n°2 avec les arrondis suivants :

| Régime | | Global (arrondi) |
|---|---|---|
| Protéine de pois NUTRALYS® S85F | moyenne | **96** |
| | écartype | 1 |
| Isolat de protéines de pois selon l'invention n°1 | moyenne | **94** |
| | écartype | 1 |
| Isolat de protéines de pois selon l'invention n°2 | moyenne | **95** |
| | écartype | 1 |

*5- Cinétique de digestion.*

Cet essai utilise une technique *in vitro* de simulation de digestion protéique selon la méthode suivante.

L'utilisation de méthodes de digestion *in vitro* permet un criblage efficace de différents produits alimentaires riches en protéines en fonction de leurs propriétés physico-chimiques et de leur comportement au cours de leur passage dans l'estomac et l'intestin grêle.

Ici, nous comparons des solutions à 3% (m/m) de protéine pour le NUTRALYS® S85F, les isolats de protéines de pois selon l'invention n°1 et n°2 et les contrôles communément utilisés dans ce genre de tests que sont la caséine et le lactosérum.

Ces 5 solutions sont donc testées dans un modèle *in vitro* de digestion dynamique dans des conditions physiologiques équivalentes à l'estomac puis l'intestin grêle.

Ce modèle de digestion est couplé à un suivi en temps réel de la viscosité grâce à un rhéomètre à contrainte imposée (AR-2000, TA Instruments, New Castle, DE, USA) équipé d'un rotor à ailette en acier inoxydable (hauteur 39 mm et diamètre 28 mm).

Les solutions protéiques ont été testées dans les mêmes conditions, à savoir un cisaillement régulier à 37°C et à la vitesse de 150 $s^{-1}$ pendant 3h.

La viscosité de base a été monitorée pendant 5 minutes avant de procéder à une acidification progressive de la solution jusqu'à un pH contenu entre 1,5 et 2.

Cette acidification prend en général 15 minutes.

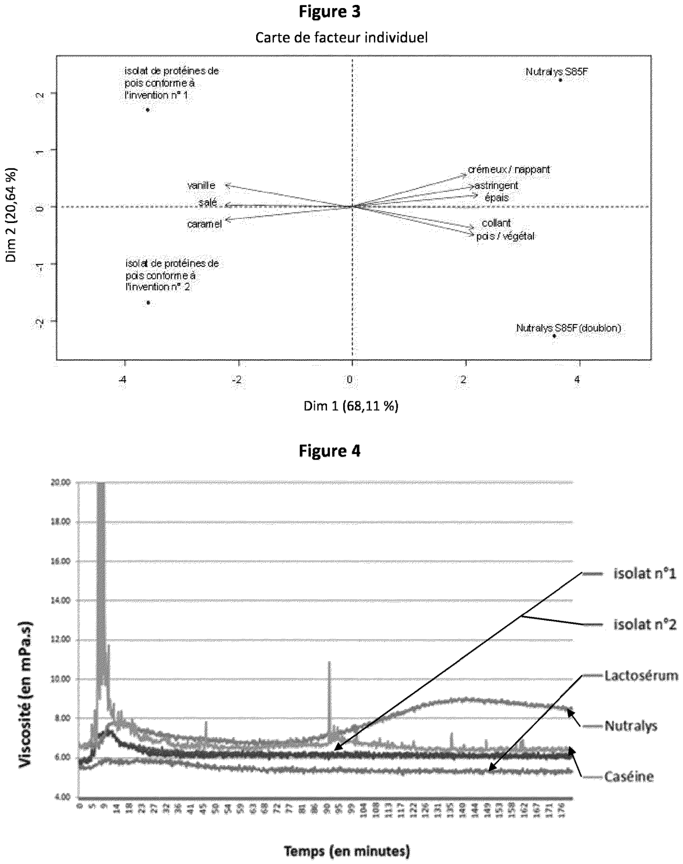

Une fois le pH de la solution stabilisé entre 1,5 et 2, on ajoute un cocktail enzymatique de pepsine stomacale (Sigma-Aldrich, St. Louis, MO, USA) et de lipase (Novozyme, Gladesaxe, Danemark). Les courbes de suivis de viscosité sont présentées dans la **Figure 4.**

Le suivi de la viscosité au cours de la digestion *in vitro* traduit bien la cinétique de digestion des protéines. Ainsi la digestion du lactosérum ne donne pas lieu à une modification de la viscosité car il s'agit d'une protéine rapidement digérée. La caséine quant à elle montre une viscosité très augmentée après l'acidification, ce qui traduit une digestion lente.

La protéine de pois de type NUTRALYS® démontre un comportement intermédiaire entre ces 2 standards, il est qualifié « d'intermédiaire rapide ».

Cependant, les isolats de protéines de pois selon l'invention n°1 et n°2 montrent un comportement encore intermédiaire entre le NUTRALYS® S85F et le lactosérum.

Il est à noter que l'association de protéines rapides avec des protéines intermédiaires peut faciliter la digestion et allonger le temps de diffusion des acides aminés dans la circulation sanguine, ce qui présente un intérêt pour la synthèse protéique au sein des muscles après un effort long.

**Exemple 2** : **Remplacement des protéines de lait par les isolats de protéines de pois dans des boissons prêtes-à-boire traitées UHT pour la nutrition clinique**

Les formulations nutritives à base de protéines de lait, de pois et d'isolats de protéines de pois sont présentées dans le tableau suivant :

| Ingrédients | Formulation contrôle | Formulation nutritionnelle selon l'invention n°1 | Formulation nutritionnelle selon l'invention n°2 | Formulation nutritionnelle avec protéines de pois |
|---|---|---|---|---|
| Maltodextrine GLUCIDEX ® IT19 (ROQUETTE FRERES) | 17,10 | 17,60 | 17,60 | 17,60 |
| Concentrât de protéines de lait (MPC 80 - FONTERRA) | 11,00 | 5,53 | 5,53 | 5,53 |

| | | | | |
|---|---|---|---|---|
| Protéines de pois NUTRALYS® S85F | | | | 5,67 |
| Isolat de protéines de pois n°1 (selon l'invention - cf. exemple 1 ci-dessus) | | 5,67 | | |
| Isolat de protéines de pois n°2 (selon l'invention - cf. exemple 1 ci-dessus) | | | 5,67 | |
| Saccharose | 3,20 | 3,4 | 3,4 | 3,4 |
| Huile de colza | 4,02 | 3,78 | 3,78 | 3,78 |
| Huile de tournesol | 2,68 | 2,52 | 2,52 | 2,52 |
| Lécithine de soja | 0,40 | 0,40 | 0,40 | 0,40 |
| Arôme vanille | 0,36 | 0,36 | 0,36 | 0,36 |
| Mélange de minéraux VITABLEND 7398 | 0,492 | 0,273 | 0,273 | 0,273 |
| Mélange de minéraux VITABLEND 7402 | | 0,469 | 0,469 | 0,469 |
| Eau déminéralisée | 60,75 | 60 | 60 | 60 |
| Total | 100 | 100 | 100 | 100 |

Les quantités étant indiquées en pourcentage en poids.

Leurs valeurs nutritionnelles pour 100 ml sont les suivantes.

| | Formulation contrôle | Formulation nutritionnelle selon l'invention n°1 | Formulation nutritionnelle selon l'invention n°2 | Formulation nutritionnelle avec protéines de pois |
|---|---|---|---|---|
| Energie calorique (kCal) | 204 | 203 | 203 | 198 |
| Teneur en protéines (g) *Dont protéines de lait* | 10,0 | 10,0 | 10,0 | 9,75 |

(suite)

| | Formulation contrôle | Formulation nutritionnelle selon l'invention n°1 | Formulation nutritionnelle selon l'invention n°2 | Formulation nutritionnelle avec protéines de pois |
|---|---|---|---|---|
| *Isolats protéines de pois* | *10,0* | *5,0* | *5,0* | *4,9* |
| | *0* | *5,0* | *5,0* | *0* |
| *protéines de pois* | *0* | *0* | *0* | *4,9* |
| Matières grasses (g) | 8,0 | 8,0 | 8,0 | 7,85 |
| Hydrates de carbone (g) *Dont saccharose* | 5,1 | 5,1 | 5,1 | 5,0 |
| Fibres (g) *Dont solubles* | 0,0 | 0,0 | 0,0 | 0,0 |
| *Dont insolubles* | 0,0 | 0,1 | 0,1 | 0,1 |

Composition en minéraux (pour 100 ml) :

| (mg) | Formulation contrôle | | Formulation nutritionnelle selon l'invention n°2 | |
|---|---|---|---|---|
| Na | 67,8 | | 107,7 | |
| Ca | 242,0 | | 241,4 | |
| K | 163,9 | | 160,4 | |
| Cl | 65,0 | | 64,1 | |
| P | 154,0 | | 135,3 | |
| Mg | 25,4 | | 24,9 | |

Le procédé de fabrication des boissons est le suivant :

◦ Mélanger à sec toutes les poudres (protéines de lait, protéines de pois, isolats de protéines de pois, maltodextrines et le saccharose),
◦ Peser 90 % de l'eau à 55°C,
◦ Ajouter le mélange de poudre dans l'eau à 55°C, disperser avec un fouet pendant 1 min puis mélanger avec un SILVERSON pendant 30 minutes à 55°C,
◦ Dans un récipient de mélange à part, dissoudre les minéraux avec l'eau restante à 50°C et ajouter immédiatement à la solution,
◦ Ajouter l'arôme vanille à ladite solution,
◦ Placer la lécithine et l'huile dans un récipient de mélange à part, agiter et chauffer à environ 55°C,
◦ Après 35 min d'hydratation, ajouter la lécithine et le mélange d'huile au lot principal en utilisant un cisaillement de 10 000 trs/min pendant 5 minutes,
◦ Préchauffer le produit à 75°C par batch de 3 l en bécher au bain marie (montée en température de l'ordre de 10 min) et homogénéiser à 200 bar (1 étage) sur un homogénéiseur de 10 l/h (environ 20 min par batch),
◦ Refroidir le produit à 30°C pendant 5 minutes à 45 minutes selon les batch,
◦ Ajuster à pH 6,8 - 7, à 30°C, avec de la soude à 30 %,
◦ Chauffer de nouveau le produit au bain-Marie à 75°C (montée en température pendant 10 minutes),
◦ Stériliser le produit à 142°C pendant 5 secondes dans un échangeur tubulaire puis refroidir dans l'échangeur à 25°C
◦ Conditionner le produit en bouteilles
◦ Stocker à + 4°C.

Les analyses réalisées sur la formulation sont les suivantes :

1. Analyse des tailles de particules de l'émulsion que forment les formulations nutritionnelles selon l'invention

L'objectif ici est d'analyser l'aspect des formulations nutritionnelles base isolats de protéines de pois en regard du contrôle base protéines de lait.

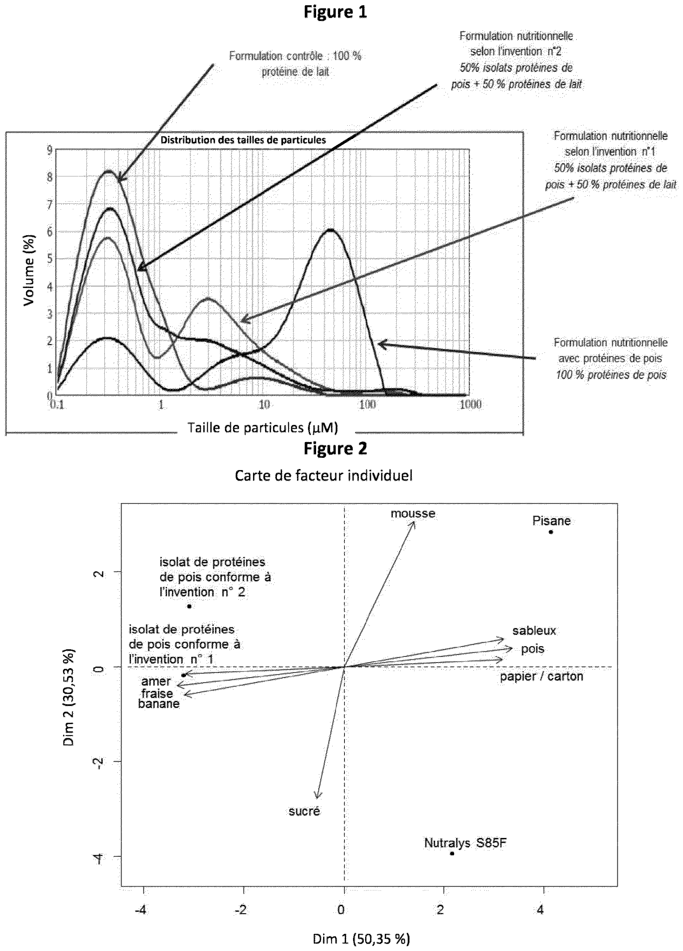

Les analyses ont été effectuées avec un analyseur de taille de particule réf. 2000 de MALVERN. Les résultats obtenus sont présentés dans la **Figure 1** et dans le tableau suivant (les valeurs du tableau sont la moyenne de 3 mesures).

Le Dmode est le diamètre principal des particules. Le d10, d50 et d90 sont les valeurs de diamètre des particules représentant respectivement 10 %, 50 % et 90 % des particules totales.

| | Taille des particules ($\mu$m) | | | |
| --- | --- | --- | --- | --- |
| | Après homogénéisation et avant l'étape de traitement thermique | | | |
| | Dmode | d10 | d50 | d90 |
| Formulation contrôle : 100 % protéine de lait | 0,3 | 0,2 | 0,4 | 1,4 |
| Formulation nutritionnelle selon l'invention n°1 *50% isolats protéines de pois n° 1 + 50 % protéines de lait* | 0,3 | 0,2 | 0,9 | 10,3 |
| Formulation nutritionnelle selon l'invention n°2 *50% isolats protéines de pois n° 2+ 50 % protéines de lait* | 0,3 | 0,2 | 0,5 | 6,7 |
| Formulation nutritionnelle avec protéines de pois *100 % protéines de pois* | 46,1 | 0,3 | 25,4 | 78,4 |

Les quatre échantillons présentent une répartition de particules bimodale. Le premier pic (première famille de particules), centré sur 0,3 $\mu$m, est majoritaire dans les trois premières formulations. Pour la formulation nutritionnelle avec protéines de pois, cette population est minoritaire.

Le second pic de la répartition bimodale (deuxième famille de particules) est fonction de l'échantillon :

◦ pour les formulations 1 et 2 selon l'invention, il y a une population centrée sur 3 $\mu$m, mais le volume de ladite population est plus forte pour la formulation n°1 ;

◦ pour la formule contrôle : le deuxième pic est centré sur 10 $\mu$m ;

◦ la formulation nutritionnelle avec protéines de pois marque sa différence en présentant un second pic centré sur 46 $\mu$m.

Il en est déduit que les isolats de protéines de pois n° 1 et n° 2 permettent d'obtenir des tailles d'émulsion de la boisson proche de celle obtenue avec de la protéine de lait.

Par ailleurs, l'isolat de protéines de pois n°2 donne même une meilleure répartition de la taille d'émulsion (répartition moins bimodale et meilleure stabilité d'émulsion représentée par la différence entre le D90 et D10) que l'isolat de protéines de pois n°1.

2. Analyse des viscosités

L'objectif ici est de démontrer la stabilité des formulations nutritionnelles base isolats de protéines de pois en regard du contrôle base protéines de lait, et de démontrer également l'avantage technologique à choisir ces isolats en regard des protéines de pois.

Les paramètres de mesure sont les suivants :

◦ Rhéomètre: Physica MCR301
◦ Outils : Tools: concentric cylinder CC27
◦ Température: 20°C (5 minutes pour être à l'équilibre)
◦ Cisaillement : 0,05 à 1000 s$^{-1}$ en 6 minutes

Le résultat des mesures de viscosités est présenté dans le tableau suivant (l'isolat de protéines de pois selon l'invention n°1 est analysé ici) :

| | Viscosité en Pa.s à 20°C | | | | | | | | |
|---|---|---|---|---|---|---|---|---|---|
| | Avant traitement thermique | | | Après traitement thermique | | | Après 1 mois de stockage à + 4°C | | |
| | | 10s⁻¹ 100s⁻¹ | 1000s⁻¹ | | 10s⁻¹ 100s⁻¹ | 1000s⁻¹ | 10s⁻¹ | 100s⁻¹ | 1000s⁻¹ |
| Formulation contrôle : 100 % protéine de lait | 0,125 | 0,107 | 0,088 | 0,048 | 0,045 | 0,044 | 0,055 | 0,051 | 0,047 |
| Formulation nutritionnelle selon l'invention n°1 *50% isolats protéines de pois n°1 + 50 % protéines de lait* | 0,063 | 0,053 | 0,048 | 0,079 | 0,053 | 0,040 | 0,327 | 0,151 | 0,099 |
| Formulation nutritionnelle avec protéines de pois *100 % protéines de pois* | 0,105 | 0,097 | 0,087 | 1,160 | 0,421 | 0,183 | 1,110 | 0,392 | 0,169 |

Le traitement thermique n'affecte pas les 3 formulations nutritionnelles de la même manière :

◦ Pour la formulation contrôle, le traitement thermique conduit à une diminution de la viscosité ;

◦ Pour la formulation n°1, il y a augmentation de la viscosité et augmentation du comportement rhéofluidifiant, mais cette augmentation de viscosité reste faible et se rapproche de celle de la formulation contrôle.

On constate en effet qu'avant le traitement thermique, la formulation contrôle présente la viscosité la plus importante, puis la formulation base protéines de pois.

Après le traitement thermique, au contraire, la formulation contrôle présente la viscosité la plus basse, puis la formulation conforme à l'invention n°1.

En conclusion, la formule nutritionnelle n°1 selon l'invention et la formule contrôle base protéines de lait ont un comportement rhéologique qui est semblable en termes de viscosité et de résistance thermique.

D'autres mesures de viscosités ont été faites sur les mêmes recettes de boisson, cette fois en prenant également une formule nutritionnelle préparée avec l'isolat de protéines de pois n°2 (en changeant notamment le mode de cuisson, ici stérilisation UHT (142°C - 5 s) à 20 l/h en ligne avec une homogénéisation en phase descendante à 75°C à 200 bars après le traitement thermique) et après un mois de stockage des boissons à + 4°C.

| | Viscosité en Pa.s à 20°C | | | | | |
|---|---|---|---|---|---|---|
| | Après traitement thermique | | | Après 1 mois stockés à 4°C | | |
| | 10s⁻¹ | 100s⁻¹ | 1000s⁻¹ | 10s⁻¹ | 100s⁻¹ | 1000s⁻¹ |
| Formulation contrôle : 100 % protéine de lait | 0,049 | 0,047 | 0,045 | 0,085 | 0,076 | 0,068 |
| Formulation nutritionnelle selon l'invention n°1 *50% isolats protéines de pois n°1 + 50 % protéines de lait* | 0,101 | 0,073 | 0,057 | 0,157 | 0,097 | 0,065 |
| Formulation nutritionnelle selon l'invention n°2 *50% isolats protéines de pois n° 2 + 50 % protéines de lait* | 0,107 | 0,075 | 0,057 | 0,155 | 0,095 | 0,064 |
| Formulation nutritionnelle avec protéines de pois *100 % protéines de pois* | 0,963 | 0,343 | 0,159 | 1,47 | 0,459 | 0,202 |

Ces résultats montrent que toutes les formulations ont une viscosité qui augmente après un mois à 4°C. Les formulations n°1 et 2 ont une viscosité qui augmente très légèrement, du même ordre de grandeur que celle de la formule contrôle, après un mois, en comparaison avec la formulation avec la protéine de pois.

Les boissons contenant les isolats de protéine de pois n°1 et 2 sont beaucoup plus stables que la boisson contenant la protéine de pois, et se rapprochent de la stabilité de la boisson contenant la protéine de lait.

**Exemple 3** : **Remplacement des protéines de lait par les isolats de protéines de pois dans des boissons prêtes-à-boire traitées UHT pour la nutrition du sportif**

Les formules nutritionnelles présentent les compositions suivantes :

| N° de la recette | 1 | 2 | 3 | 4 | 5 |
|---|---|---|---|---|---|
| | % en poids | % en poids | % en poids | % en poids | % en poids |
| Concentrat de protéine de lait (MPC 80 DMV) | 8,05 | 4,85 | 4,85 | 4,85 | 4,85 |
| Isolat de protéines de pois n°1 (selon l'invention - cf. exemple 1 ci-dessus) | | 3,30 | | | |
| Isolat de protéines de pois n°1 (selon l'invention - cf. exemple 2 ci-dessus) | | | 3,30 | | |
| Protéine de pois NUTRALYS ® S85F (ROQUETTE FRERES) | | | | 3,30 | |
| Protéine de pois PISANE® de la société COSUCRA | | | | | 3,30 |
| Maltodextrine GLUCIDEX® IT19 (ROQUETTE FRERES) | 1,70 | 1,60 | 1,60 | 1,60 | 1,60 |
| Saccharose | 3,80 | 4 | 4 | 4 | 4 |
| Arôme vanille | 0,36 | 0,36 | 0,36 | 0,36 | 0,36 |
| MATVIS C3000 (AGI) | 0,04 | 0,04 | 0,04 | 0,04 | 0,04 |
| Lait écrémé liquide | 86,05 | 85,85 | 85,85 | 85,85 | 85,85 |
| *TOTAL* | *100* | *100* | *100* | *100* | *100* |

Les quantités étant indiquées en pourcentage en poids.

Leurs valeurs nutritionnelles pour 100 ml sont les suivantes

| N° de la recette | 1 | 2 | 3 | 4 | 5 |
|---|---|---|---|---|---|
| Energie calorique (kCal) | 85 | 86 | 86 | 86 | 86 |
| Teneur en protéines (g) | 10 | 10 | 10 | 10 | 10 |
| *Dont protéines de lait* | 10 | 7,2 | 7,2 | 7,2 | 7,2 |
| *isolat protéines de pois* | 0 | 2,8 | 2,8 | 0 | 0 |
| *protéines de pois* | 0 | 0 | 0 | 2,8 | 2,8 |
| Matières grasses (g) | 0,2 | 0,5 | 0,5 | 0,5 | 0,5 |
| Hydrates de carbone (g) | 10,5 | 10,5 | 10,5 | 10,5 | 10,5 |
| *Dont sucres* | 8,5 | 8,5 | 8,5 | 8,5 | 8,5 |
| Fibres (g) | 0 | 0,1 | 0,1 | 0,1 | 0,1 |
| *Dont solubles* | 0 | 0 | 0 | 0 | 0 |
| *Dont insolubles* | 0 | 0,1 | 0,1 | 0,1 | 0,1 |

Les conditions de préparation desdites boissons sont les mêmes que celles de l'exemple 2.

On réalise des mesures de viscosité desdites boissons prêtes-à-boire pour sportifs.

L'objectif ici est également de démontrer la stabilité des formulations nutritionnelles base isolats de protéines de pois en regard du contrôle base protéines de lait, et de démontrer également l'avantage technologique à choisir ces isolats en regard des protéines de pois.

Les paramètres de mesure sont les suivants :

◦ Rhéomètre: Physica MCR301

◦ Outils : Tools: concentric cylinder CC27

◦ Température: 20°C (5 minutes pour être à l'équilibre)

◦ Cisaillement : 0,05 à 1000 s$^{-1}$ en 6 minutes

Le résultat des mesures de viscosités est présenté dans le tableau suivant :

|  |  | Viscosité (Pa,s) à | | |
|---|---|---|---|---|
| Référence de la recette | traitement thermique | 10s$^{-1}$ | 100 s$^{-1}$ | 100 s$^{-1}$ |
| 1 | Avant UHT | 0,74 | 0,27 | 0,09 |
| 1 | Apres UHT | 0,27 | 0,09 | 0,043 |
| 2 | Avant UHT | 0,067 | 0,043 | 0,028 |
| 2 | Apres UHT | 0,024 | 0,02 | 0,018 |
| 3 | Avant UHT | 0,106 | 0,056 | 0,032 |
| 3 | Apres UHT | 0,026 | 0,021 | 0,019 |
| 4 | Avant UHT | 0,04 | 0,027 | 0,02 |
| 4 | Apres UHT | 0,15 | 0,075 | 0,042 |
| 5 | Avant UHT | 0,042 | 0,029 | 0,022 |
| 5 | Apres UHT | 0,091 | 0,055 | 0,035 |

On en déduit que :

- avant UHT, les formulations contenant des isolats de protéines de pois ont une viscosité intermédiaire entre les protéines de lait et les protéines de pois,
- après UHT, les formulations contenant des isolats de protéines de pois perdent de la viscosité, alors que les protéines de pois en gagnent ; les protéines de lait restant les plus visqueuses.

Les isolats de protéines de pois sont donc plus stables au traitement thermique que les protéines de pois et sont plus adaptés aux boissons UHT prêtes-à-boire sport de par leur faible viscosité, propriétés requises par les boissons UHT prêtes-à-boire sport.

**Exemple 4 : Remplacement des protéines de lait par les isolats de protéines de pois dans une formule liquide nutritionnelle traitée UHT pour la nutrition clinique entérale**

Les formules nutritionnelles ont alors les compositions suivantes :

|  | 1 | 2 |
|---|---|---|
|  | Témoin 100% Caséinate de calcium | 50 % Substitution des protéines de lait |
| Caséinate de calcium (DMV) | 4,96 | 2,48 |

(suite)

|  | **1** | **2** |
|---|---|---|
|  | Témoin 100% Caséinate de calcium | 50 % Substitution des protéines de lait |
| Isolat de protéines de pois N°1 ou N°2 | 0,00 | 2,82 |
| Maltodextrine GLUCIDEX® IT 19 | 14,60 | 14,60 |
| Huile colza | 4,40 | 4,40 |
| Eau | 75,04 | 74,70 |
| Mix minéraux Vitablend | 1,00 | 1,00 |
| *TOTAL* | *100,00* | *100,00* |

Les quantités étant indiquées en pourcentage en poids.

Les valeurs nutritionnelles pour 100 ml sont les suivantes.

| N° de la recette | 1 | 2 |
|---|---|---|
| Matière sèche (%) | 24,2 | 24,5 |
| Energie calorique (kCal) pour 100 g | 116 | 117 |
| Matières grasses (g) | 4,5 | 4,7 |
| Teneur en protéines (g) | 4,52 | 4,52 |
| *Dont protéines de lait* | 4,52 | 2,26 |
| *isolat protéines de pois* | 0 | 2,26 |
| Hydrates de carbone (g) | 14 | 14 |
| *Dont sucres* | 0,9 | 0,9 |
| Fibres (g) | 0 | 0 |
| *Dont solubles* | 0 | 0 |
| *Dont insolubles* | 0 | 0 |

Le procédé de fabrication des boissons est le suivant :

∘ Mélanger les caséinates dans l'eau à 50°C, ajouter les protéines, et mélanger sur plaque magnétique pendant 10 minutes,

∘ Ajouter les hydrates de carbone et les minéraux sous mélangeur SILVERSON,

∘ Ajouter l'huile sous SILVERSON pendant 5 min (10 000 rpm),

∘ Homogénéiser à 60°C à 250 bars dans un homogénéisateur haute pression NIRO SOAVI 2K (2 étages), o Ajuster le pH à 6,9 avec une solution d'acide citrique à 50 %,

o Stériliser à l'autoclave dans des bocaux en verre à 120°C pendant 15 minutes,

o Refroidir à température ambiante.

Dans ces conditions opératoires, les isolats de protéines de pois peuvent être avantageusement mis en oeuvre pour substituer les protéines de lait.

**Exemple 5 : Comparaison des propriétés sensorielles de 4 mélanges de poudre formulés avec des isolats de protéines de pois conformes à l'invention pour sportifs**

Le panel est constitué de 13 personnes.

Le panel est qualifié à la dégustation de produits formulés avec la protéine de pois. Il a reçu une formation et un entraînement afin de vérifier ses performances en termes de :

- Capacité à discriminer les produits

- Consensus, utilisation correcte des descripteurs

- Répétabilité, capacité à repérer un doublon

En effet, il a reçu une formation pour la bonne utilisation des descripteurs sensoriels de goût et de texture, tels que, par exemple :

| Descripteur | Définition | Mode opératoire | Référence |
|---|---|---|---|
| POIS | Goût typique de pois | Goûtez le produit | solution à 3% en poids de petits pois dans de l'eau (homogénéisation au blender) |
| FRAISE | Goût typique de fraise | Goûtez le produit | solution d'arôme fraise |
| BANANE | Goût typique de banane | Goûtez le produit | solution d'arôme banane |
| CARTON | Goût typique de carton | Goûtez le produit | Solution d'acide tannique à 0.5g/L dans de l'eau minérale |
| SUCRE | Goût sucré | Goûtez le produit | Solution de saccharose à 6g/L dans de l'eau minérale |
| AMER | Goût amer | Goûtez le produit | Solution de caféine à 0.5g/L dans de l'eau minérale |
| SABLEUX | Evaluation de la granulosité et du nombre de particules d'un produit (pas sableux / très sableux) | Mâchez une unité de produit jusqu'à ce qu'il soit prêt à avaler et évaluez la perception de petits grains dans la bouche | Certaines poires |

La méthode leur permet également de s'exprimer sur d'autres descripteurs qui n'auraient pas été anticipés dans cette liste.

**Produits**

Les formules nutritionnelles sont des mélanges de poudre destinés au sportif de composition suivante :

| Rapport Protéines de pois ou isolats de protéines de pois selon l'invention / Protéines de lait | 55/45 | 55/45 | 55/45 | 55/45 |
|---|---|---|---|---|
| Formules nutritionnelles à base de : | Protéine de pois NUTRALYS® S85F | Isolat de protéines de pois selon l'invention n°1 | Isolat de protéines de pois selon l'invention n°2 | Protéine de pois PISANE® de la société COSUCRA |
| | % en poids | % en poids | % en poids | % en poids |
| Concentrat de protéine de Lactosérum (WPC 80 Fonterra) | 43,2 | 43,2 | 43,2 | 43,2 |

(suite)

| Rapport Protéines de pois ou isolats de protéines de pois selon l'invention / Protéines de lait | 55/45 | 55/45 | 55/45 | 55/45 |
|---|---|---|---|---|
| Formules nutritionnelles à base de : | **Protéine de pois NUTRALYS® S85F** | **Isolat de protéines de pois selon l'invention n°1** | **Isolat de protéines de pois selon l'invention n°2** | **Protéine de pois PISANE® de la société COSUCRA** |
| | % en poids | % en poids | % en poids | % en poids |
| Isolat de protéines de pois selon l'invention n°1 | 0 | 52,8 | | |
| Isolat de protéines de pois selon l'invention n°2 | 0 | 0 | 52,8 | 0 |
| Protéine de pois PISANE® de la société COSUCRA | 0 | 0 | 0 | 52,8 |
| NUTRALYS® S85F | 52,8 | 0 | 0 | 0 |
| Arôme banane (N-Capture MSD M_ 0058032) | 0,18 | 0,18 | 0,18 | 0,18 |
| Arôme fraise (N-Capture MSD M_0055259) | 2,7 | 2,7 | 2,7 | 2,7 |
| Sel | 0,5 | 0,5 | 0,5 | 0,5 |
| Gomme xanthane F80 (AGI) | 0,35 | 0,35 | 0,35 | 0,35 |
| Acesulfame K | 0,1 | 0,1 | 0,1 | 0,1 |
| Sucralose | 0,1 | 0,1 | 0,1 | 0,1 |
| *TOTAL* | *100* | *100* | *100* | *100* |

Les quantités étant indiquées en pourcentage en poids.

Ils sont reconstitués juste avant la dégustation dans de l'eau à température ambiante.

Conditions de dégustation

- Au laboratoire d'analyse sensorielle : box individuels de dégustation, murs blancs, ambiance calme (pour faciliter la concentration)

- Lumière blanche (pour avoir exactement la même vision du produit)

- En fin de matinée ou d'après-midi (pour être au maximum des capacités sensorielles)

- Produits anonymés par un code à 3 chiffres (pour éviter que le code n'influence l'appréciation des produits)

- Produits présentés dans un ordre aléatoire (pour éviter les effets d'ordre et de rémanence)

**Test**

La méthode employée pour comparer les produits a été le Profil Flash (JM Sieffermann, 2000 - Le profil Flash: Un outil rapide et innovant d'évaluation sensorielle descriptive. In: L'innovation: de l'idée au succès - Douzièmes rencontres AGORAL. Pp. 335-340, 22 & 23 mars 2000. Paris, France: Lavoisier, Tec & Doc.).

Les produits sont tous présentés simultanément. Il s'agit de comparer les produits entre eux en réalisant une succession de classements : les panélistes choisissent les descripteurs qui leur semblent les plus pertinents pour discriminer les produits entre eux et classent les produits selon ces descripteurs, il est possible que plusieurs produits soient regroupés sur un même rang.

Exemple :

Descripteur sensoriel : *Croustillant*

F         A       (E D B)    C        +

────────────────────────────►

**Traitement des données**

La méthode de traitement statistique adaptée à ce type de données est l'Analyse Factorielle Multiple (J. Pagès, 1994 - Multiple factor analysis (AFMULT package). In: Computational Statistics & Data Analysis, Volume 18, Issue 1, August 1994, Pages 121-140) sur les données-rangs des produits.

Pour que les résultats soient plus clairs, l'AFM a été réalisée plusieurs fois; en global, et par critère (aspect, odeur, goût, texture). Les graphiques présentés synthétisent l'ensemble des résultats fournis par cette méthode.

Les analyses ont été réalisées à l'aide du logiciel R (en vente libre) :

R version 2.14.1 (2011-12-22)
Copyright (C) 2011 The R Foundation for Statistical Computing
ISBN 3-900051-07-0
20 Platform: i386-pc-mingw32/i386 (32-bit)

Le logiciel est un environnement de travail qui nécessite le chargement de modules contenant les fonctions de calcul comme le package FactoMineR version 1.19

**Résultats**

La représentation graphique des résultats est présentée sur la **Figure 2.**

3 groupes se distinguent : la protéine de pois PISANE® commercialisée par la société COSUCRA, la protéine de pois NUTRALYS® S85F, et les deux isolats de protéines de pois conformes à l'invention de l'exemple 1.

Les panélistes ont établi peu de différence entre deux isolats de protéines de pois conformes à l'invention de l'exemple 1.

Ceux-ci sont moins sableux, pois et papier / carton que les produits témoins et sont plus amers et plus fraise / banane (arômes utilisés dans cette formulation).

Le mix avec PISANE® s'est démarqué en termes de texture car sa mise en application a généré la formation de mousse.

Le mix avec NUTRALYS® S85F s'est quant à lui démarqué pour son goût sucré.

**Exemple 6 : Comparaison des propriétés sensorielles de boissons « prêtes-à-boire » pour la nutrition clinique**

Le panel est constitué de 14 personnes.

Le panel est, comme dans l'exemple 3, qualifié à la dégustation de produits formulés avec la protéine de pois. Il a reçu une formation et un entraînement afin de vérifier ses performances en termes de :

• Capacité à discriminer les produits

• Consensus, utilisation correcte des descripteurs

• Répétabilité, capacité à repérer un doublon

En effet, il a reçu une formation pour la bonne utilisation des descripteurs sensoriels de goût et de texture, tels que, par exemple :

| Descripteur | Définition | Mode opératoire | Référence |
|---|---|---|---|
| POIS | Goût typique de pois | Goûtez le produit | Solution à 3% en poids de petits pois dans de l'eau (homogénéisation au mélangeur) |
| VANILLE | Goût typique de vanille | Goûtez le produit | Solution d'arôme vanille |
| CARAMEL | Goût typique de caramel | Goûtez le produit | Caramel |
| SALE | Goût salé | Goûtez le produit | Solution de chlorure de sodium à 0.01 g/L dans de l'eau minérale |
| ASTRINGENT | Sensation d'assèchement | Goûtez le produit | Solution d'acide tannique à 0.5g/L dans de l'eau minérale |
| CREMEUX/ NAPPANT | Evaluation de la texture douce et fondante du produit. (pas onctueux / très onctueux) | Mâchez une unité de produit et vérifiez s'il provoque un contact mou et s'il tapisse la bouche. | Crème fraîche, crème épaisse |
| COLLANT | Evaluation de la force nécessaire pour décoller des produits qui adhèrent à l'intérieur de la cavité buccale. (pas collant / très collant) | Au bout de quelques mastications consécutives, pressez le produit entre les dents et mesurez la force nécessaire pour qu'il se décolle des dents. | Caramel mou |
| EPAIS | Evaluation de la facilité du produit à s'écouler en bouche. (pas épais / très épais) | Frottez la langue contre le palais et vérifiez si le produit s'écoule facilement. | Lait concentré sucré, miel |

La méthode leur permet également de s'exprimer sur d'autres descripteurs qui n'auraient pas été anticipés dans cette liste.

**Produits**

Les produits sont des boissons « prêtes-à-boire » dont les recettes sont celles de l'exemple 2.

Ils sont présentés aux panélistes à température ambiante.

Conditions de dégustation

- Au laboratoire d'analyse sensorielle : box individuels de dégustation, murs blancs, ambiance calme (pour faciliter la concentration)
- Lumière blanche (pour avoir exactement la même vision du produit)
- En fin de matinée ou d'après-midi (pour être au maximum des capacités sensorielles)
- Produits anonymés par un code à 3 chiffres (pour éviter que le code n'influence l'appréciation des produits)
- Produits présentés dans un ordre aléatoire (pour éviter les effets d'ordre et de rémanence)

**Exercice**

La méthode employée pour comparer les produits a été le Profil Flash (JM Sieffermann, 2000).

Les produits sont tous présentés simultanément. Il s'agit de comparer les produits entre eux en réalisant une succession de classements : les panélistes choisissent les descripteurs qui leur semblent les plus pertinents pour discriminer les produits entre eux et classent les produits selon ces descripteurs, il est possible que plusieurs produits soient regroupés sur un même rang.

Exemple :

Descripteur sensoriel : *Croustillant*

F          A          E D B          C          +

**Traitement des données**

La méthode de traitement statistique adaptée à ce type de données est l'Analyse Factorielle Multiple (J. Pagès, 1994) sur les données-rangs des produits. Pour que les résultats soient plus clairs, l'AFM a été réalisée plusieurs fois; en global, et par critère (aspect, odeur, goût, texture). Les graphiques présentés synthétisent l'ensemble des résultats fournis par cette méthode.

Les analyses ont été réalisées à l'aide du logiciel R (en vente libre) :

R version 2.14.1 (2011-12-22)
Copyright (C) 2011 The R Foundation for Statistical Computing
ISBN 3-900051-07-0
20 Platform: i386-pc-mingw32/i386 (32-bit)

Le logiciel est un environnement de travail qui nécessite le chargement de modules contenant les fonctions de calcul comme le package FactoMineR version 1.19

**Résultats**

La représentation graphique des résultats est présentée sur la **Figure 3.**
Le NUTRALYS® S85F a été présenté en double pour tester la répétabilité du panel : on peut voir sur le graphique que les deux points sont proches sur la première dimension (la plus importante) mais pas sur la deuxième, on considère donc que cette deuxième dimension est constituée de bruit de mesure. Ainsi il n'y a pas de différence significative entre les deux isolats de protéines de pois conformes à l'invention, puisqu'ils sont proches sur la première dimension.
Il apparait que les deux isolats de protéines de pois conformes à l'invention sont plus vanille / caramel et salés que le NUTRALYS® S85F qui se trouve être plus pois / végétal et astringent en goût ; plus crémeux / nappant, épais et collant en texture.

**Exemple 7 : Comparaison des propriétés sensorielles de boissons « prêtes-à-boire » pour sportifs**

Le panel est constitué de 12 personnes.
Le panel est, comme dans l'exemple 3, qualifié à la dégustation de produits formulés avec la protéine de pois. Il a reçu une formation et un entraînement afin de vérifier ses performances en termes de :

• Capacité à discriminer les produits
• Consensus, utilisation correcte des descripteurs
• Répétabilité, capacité à repérer un doublon

**Produits**

Les produits sont des boissons « prêtes-à-boire » dont les recettes sont celles de l'exemple 3. Ils sont présentés aux panélistes à température ambiante.

**Exercice**

La méthode employée pour comparer les produits a été le Profil Flash (JM Sieffermann, 2000).

Les produits sont tous présentés simultanément. Il s'agit de comparer les produits entre eux en réalisant une succession de classements : les panélistes choisissent les descripteurs qui leur semblent les plus pertinents pour discriminer les produits entre eux et classent les produits selon ces descripteurs, il est possible que plusieurs produits soient regroupés sur un même rang.

Exemple :

Descripteur sensoriel : noix fraîche

F       A       E  D  B       C                              +

Voici la liste de descripteurs présentée aux panélistes à titre indicatif :

| arôme / odeur | | | | saveur / sensation bouche | texture |
|---|---|---|---|---|---|
| **lacté** | **végétal** | **arôme** | **off flaveur** | | |
| lait | AG pois / végétal | vanille | papier / carton | acide | collant |
| lactosérum | céréales | caramel | lessive | amer | décante |
| lait fermenté | végétal | | chimique | salé | crémeux |
| lait reconstitué bébé | noix fraiche | | colle | astringent / asséchant | consistance crème dessert |
| yaourt | pomme de terre | | métallique | sucré | épais |
| beurre | | | | piquant | flottard aqueux |
| | | | | | mousseux aéré |
| | | | | | nappant |
| | | | | | râpeux poudreux sableux |
| | | | | | lisse |

**Traitement des données**

La méthode de traitement statistique adaptée à ce type de données est l'Analyse Factorielle Multiple (J. Pagès, 1994) sur les notes des produits. L'ensemble des descripteurs généré par un juge est un groupe de variable. Les graphiques présentés synthétisent l'ensemble des résultats fournis par cette méthode.
Les traitements statistiques ont été effectués avec le logiciel R version 2.14.1 (2011-12-22).

**Résultats**

La représentation graphique des résultats est présentée sur la **Figure 6.**
Sur la dimension 1, on distingue deux familles : les deux isolats de protéines de pois selon l'invention / les deux protéines de pois et avec la dimension 2 on peut caractériser les 4 échantillons selon leur texture, odeur et goût.
En texture, les boissons prêtes à boire avec la PISANE ® et le NUTRALYS® S85F sont plus épaisses que celles avec les isolats de protéines de pois selon l'invention.
En odeur, la boisson avec l'isolat de protéines de pois selon l'invention n°1 est plus vanille que l'isolat de protéines de pois selon l'invention n°2, tandis qu'avec la PISANE®, l'odeur est plus pois.

En goût, la PISANE® apparaît piquant et chimique et comme pour l'odeur, plus pois, noix et végétale. Les boissons prêtes à boire avec la PISANE et le NUTRALYS® S85F ont en commun le caractère amer et papier-carton.

Concernant les boissons avec les isolats de protéines de pois conformes à l'invention, le la boisson n°1 (avec isolat n°1) est plus lactée et vanille et la boisson n°2 est plus céréales et confiture de lait / caramel.

**Exemple 8** : **Remplacement des protéines de laits par des isolats de protéines de pois dans des crèmes desserts traitées UHT pour la nutrition clinique.**

Les formulations nutritives à base de protéines de lait, de pois, de pois concurrent et d'isolats de protéines de pois selon l'invention sont présentées dans le tableau suivant (substitution de l'ordre de 23 %) :

| Ingrédients | Formulation contrôle | Formulation nutritionnelle selon l'invention n°2 | Formulation nutritionnelle avec protéines de pois | Formulation nutritionnelle avec protéines de pois concurrente |
|---|---|---|---|---|
| Eau déminéralisée | 64,30 | 64,30 | 64,30 | 64,30 |
| Saccharose | 12,40 | 12,40 | 12,40 | 12,40 |
| Isolat de protéines de lait (MPI - Ingredia) | 12,00 | 9,60 | 9,60 | 9,60 |
| Huile de colza | 3,94 | 3,94 | 3,94 | 3,94 |
| Amidon modifié de maïs CLEARAM® CR3020 (ROQUETTE FRERES) | 3,50 | 3,50 | 3,50 | 3,50 |
| Maltodextrine GLUCIDEX® IT19 (ROQUETTE FRERES) | 1,70 | 1,70 | 1,70 | 1,70 |
| DEXTRINE de maïs NUTRIOSE® FM06 | 1,50 | 1,50 | 1,50 | 1,50 |
| Arôme lait | 0,36 | 0,36 | 0,36 | 0,36 |
| Lécithine de soja | 0,30 | 0,30 | 0,30 | 0,30 |
| Isolat de protéines de pois n°2 (selon l'invention - cf. exemple 1 ci-dessus) | | 2,40 | | |
| Protéines de pois NUTRALYS® S85F (ROQUETTE FRERES) | | | 2,40 | |
| Protéines de pois PISANE® (COSUCRA) | | | | 2,40 |
| Total | 100 | 100 | 100 | 100 |

Les quantités étant indiquées en pourcentage en poids.

Les valeurs nutritionnelles pour 100 g sont les suivantes :

| | Formulation contrôle | Formulation nutritionnelle selon l'invention n°2 | Formulation nutritionnelle avec protéines de pois | Formulation nutritionnelle avec protéines de pois concurrente |
|---|---|---|---|---|
| Energie calorique (kCal) | 151 | 153 | 153 | 153 |

(suite)

| | Formulation contrôle | Formulation nutritionnelle selon l'invention n°2 | Formulation nutritionnelle avec protéines de pois | Formulation nutritionnelle avec protéines de pois concurrente |
|---|---|---|---|---|
| Teneur en protéines (g) | 10,3 | 10,1 | 10,1 | 10,1 |
| Dont protéines de lait | 10,3 | 8,1 | 8,1 | 8,1 |
| Dont isolats protéines de pois | 0 | 0 | 0 | 0 |
| Dont protéines de pois | 0 | 2 | 2 | 2 |
| Matières grasses (g) | 4,7 | 4,9 | 4,9 | 4,9 |
| Hydrates de carbone (g) | 17,8 | 17,7 | 17,7 | 17,7 |
| Dont saccharose | 12,9 | 12,8 | 12,8 | 12,8 |
| Fibres (g) | 1,2 | 1,3 | 1,3 | 1,3 |
| Dont solubles | 1,2 | 1,3 | 1,3 | 1,3 |
| Dont insolubles | 0 | 0 | 0 | 0 |

Le procédé de fabrication des boissons est le suivant :

o Préchauffer l'eau à 50°C,

o Mélanger à sec toutes les poudres (protéines de lait, protéines de pois, isolats de protéines de pois, maltodextrines, dextrines, le saccharose et l'amidon),

o Ajouter le mélange de poudre dans l'eau à 50°C, disperser avec un fouet pendant 1 min puis mélanger avec un mélangeur SILVERSON à 3000 trs/min pendant 30 minutes à 50°C,

o Ajouter l'arôme à ladite solution,

o Placer la lécithine et l'huile dans un récipient de mélange à part, agiter et chauffer à 50°C,

o Après 30 min d'hydratation, ajouter la lécithine et le mélange d'huile au lot principal en utilisant un cisaillement de 10 000 trs/min pendant 5 minutes,

o Stériliser le produit à 133°C pendant 55 sec dans un échangeur tubulaire puis conditionner à 70°C,

o Stocker à 4°C.

*Comparaison des propriétés sensorielles de crèmes desserts pour la nutrition clinique.*

Le panel est qualifié à la dégustation de produits formulés. Il a reçu une formation et un entrainement afin de vérifier ses performances en termes de :

- Capacité à discriminer les produits
- Consensus, utilisation correcte des descripteurs
- Répétabilité, capacité à repérer un doublon

Le panel est constitué de 26 personnes, parmi le personnel Roquette, et le jour de la dégustation, 11 personnes étaient présentes, parmi lesquelles 6, ont spécifiquement été entraînées sur la thématique des crèmes desserts.

Les produits ont été préparés puis conservés au réfrigérateur.

Ils sont servis aux panélistes à température ambiante.

## Conditions de dégustation

Au laboratoire d'analyse sensorielle : box individuels de dégustation, murs blancs, ambiance calme (pour faciliter la concentration)

- Lumière blanche (pour avoir exactement la même vision du produit)
- En fin de matinée ou d'après-midi (pour être au maximum des capacités sensorielles)
- Produits anonymés par un code à 3 chiffres (pour éviter que le code n'influence l'appréciation des produits)
- Produits présentés dans un ordre aléatoire (pour éviter les effets d'ordre et de rémanence)

## Exercice

La méthode employée pour comparer les produits a été le Profil Flash (JM Sieffermann, 2000).

Les produits sont tous présentés simultanément. Il s'agit de comparer les produits entre eux en réalisant une succession de classements : les panélistes choisissent les descripteurs qui leur semblent les plus pertinents pour discriminer les produits entre eux et classent les produits selon ces descripteurs, il est possible que plusieurs produits soient regroupés sur un même rang.

Exemple :

Descripteur sensoriel : *Fondant*

Voici la liste de descripteurs présentée aux panélistes à titre indicatif :

| CREMES DESSERTS | | | |
|---|---|---|---|
| **Descripteur** | **Définition** | **Mode opératoire** | **Référence** |
| **ASPECT** | | | |
| **Brillant** | qui renvoie la lumière. (pas brillant / très brillant) | explorez visuellement le produit | Jaune d'oeuf cru |
| **Granuleux (aspect)** | qui présente des particules (nombreuses et/ou de taille importante) (pas granuleux / très granuleux) | explorez visuellement le produit | Sucre roux |
| **GOUT** | | | |
| **Sucré** | Saveur élémentaire provoquée par une solution de saccharose | Goûtez le produit | Sucre en poudre |
| **Amer** | Saveur élémentaire qui provoque une sensation âpre, désagréable, par une solution de caféine | Goûtez le produit | Caféine / café |
| **Pois** | Qui le goût de pois | Goûtez le produit | Pois |

| TEXTURE | | | |
|---|---|---|---|
| **à la cuillère** | | | |
| **Epais (cuillère)** | qui résiste à l'écoulement (pas épais / très épais) | évaluez la résistance du produit à la contrainte en tournant la cuillère dans le pot | Miel |
| **Court** | qui forme un mince filet d'écoulement / tombe par paquet (texture longue / courte) | prenez une unité de produit avec une cuillère. Levez et tournez la cuillère. Vérifiez la longueur du filet d'écoulement | SojaSun |
| **Texture en bouche** | | | |
| **Phase initiale (perçue dès l'introduction dans la bouche)** | | | |
| **Epais (en bouche)** | qui s'écoule difficilement en bouche (pas épais / très épais) | prenez une unité de produit en bouche et faites le circuler dans la cavité buccale | Mascarpone |
| **Phase masticatoire (perçue pendant la mastication)** | | | |
| **Fondant** | Evaluation de la capacité de dissolution du produit en bouche sous l'action de la salive. (pas fondant / très fondant) | gardez le produit en mouvement dans la bouche et évaluez le temps mis pour le dissoudre. | Glace |
| **Crémeux** | qui présente un contact doux et fond en bouche (pas crémeux / très crémeux) | prenez une unité de produit en bouche et vérifiez s'il provoque un contact mou et s'il tapisse la cavité buccale | Crème fraîche épaisse |
| **Granuleux (en bouche)** | qui présente des particules (lisse / très granuleux) | glissez la langue contre le palais et évaluer la perception de petits grains en bouche | Sucre roux |
| **Phase résiduelle (modifications opérées pendant la mastication)** | | | |
| **Collant** | qui adhère aux parois de la cavité buccale (pas collant / très collant) | placez un échantillon sur la langue, le comprimer contre le palais et évaluez la force nécessaire pour l'enlever avec la langue | Caramel mou |

**Traitement des données**

La méthode de traitement statistique adaptée à ce type de données est l'Analyse Factorielle Multiple (J. Pagès, 1994) sur les données-rangs des produits. Pour que les résultats soient plus clairs, l'AFM a été réalisée plusieurs fois; en global, et par critère (aspect, odeur, goût, texture). Les graphiques présentés synthétisent l'ensemble des résultats fournis par cette méthode.
Les traitements statistiques ont été effectués avec le logiciel R version 2.14.1 (2011-12-22)

**Résultats**

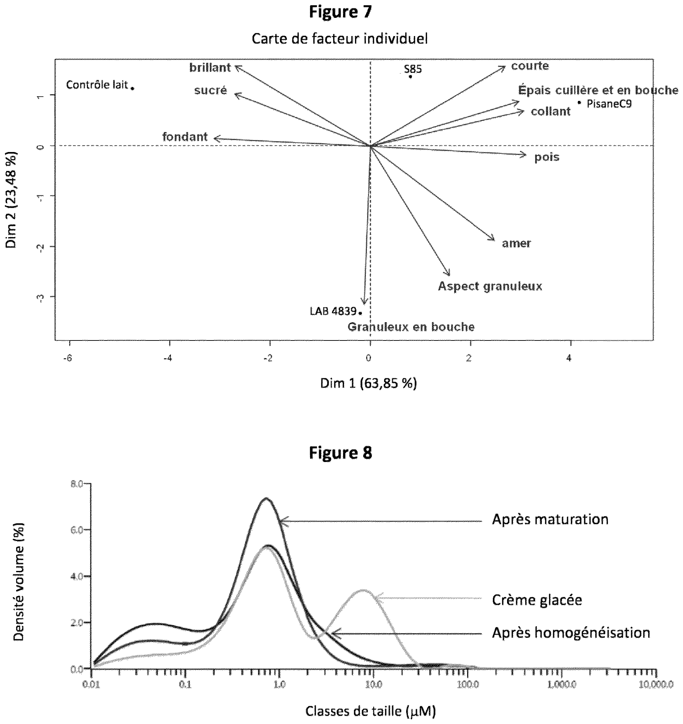

Comme il apparaît sur la **Figure 7,** les crèmes desserts sont discriminés de façon consensuelle par l'ensemble des panélistes, avec une dimension 1 très importante à presque 64% qui nous décrit les produits extrêmes de la façon suivante.
Le témoin lait est le plus brillant en aspect, fondant en bouche mais le moins épais, et en goût le plus sucré.
En texture, les crèmes desserts avec la PISANE® C9 et le NUTRALYS® S85F sont plus épaisses que celles avec l'isolat de protéine de pois selon l'invention.
En goût, la crème dessert avec l'isolat de protéine de pois est moins pois que l'essai avec la PISANE® C9 et le NUTRALYS® S85F.

**Exemple 9. Remplacement des protéines de lait par les isolats de protéines de pois dans les crèmes glacées/desserts glacés**

4 recettes sont élaborées :

o Témoin : avec 100 % de protéines de lait

o Recette n°1 : 50 % de protéine de lait remplacés par de la protéine de pois NUTRALYS® S85F.

o Recette n°2 : 50 % de protéine de lait remplacés par de l'isolat de protéines de pois selon l'invention n°1 ;

o Recette n°3 : 50 % de protéine de lait remplacés par de l'isolat de protéines de pois selon l'invention n°2 ;

|  | Témoin | Recette n°1 | Recette n°2 | Recette n°3 |
|---|---|---|---|---|
| Eau | 48,70 | 48,77 | 48,79 | 48,79 |
| Saccharose | 13,00 | 13,00 | 13,00 | 13,00 |
| Crème (35 % de matière grasse) | 27,40 | 27,40 | 27,40 | 27,40 |
| Lactose | 0,00 | 2,10 | 2,10 | 2,10 |
| Poudre de lait écrémée | 5,80 | 2,05 | 2,05 | 2,05 |
| Sirop de glucose 70/81 | 4,00 | 4,00 | 4,00 | 4,00 |
| Protéine de pois NUTRALYS ® S85F | 0,00 | 1,58 | 0,00 | 0,00 |
| Isolat de protéines de pois selon l'invention n°1 | 0,00 | 0,00 | 1,56 | 0,00 |
| Isolat de protéines de pois selon l'invention n°2 | 0,00 | 0,00 | 0,00 | 1,56 |
| Stabilisants (CREMODAN SE30) | 0,60 | 0,60 | 0,60 | 0,60 |
| Arôme vanille IFF 10836706 | 0,50 | 0,50 | 0,50 | 0,50 |
| **TOTAL** | **100,00** | **100,00** | **100,00** | **100,00** |

Les quantités étant indiquées en pourcentage en poids.

| Valeurs nutritionnelles pour 100 g | | | | |
|---|---|---|---|---|
| Energie (kcal) | 181,75 | 174,63 | 174,47 | 168,31 |
| Matière grasse (g) | 10,03 | 10,15 | 10,15 | 10,15 |
| Glucides (g) | 20,33 | 20,30 | 20,33 | 20,30 |
| Dont sucres (g) | 19,59 | 17,54 | 17,55 | 17,54 |
| Protéines (g) | 2,53 | 2,50 | 2,50 | 2,50 |
| Dont protéines de lait (q) | 2,53 | 1,25 | 1,25 | 1,25 |

| Dont protéines végétales (g) | 0,00 | 1,25 | 1,25 | 1,25 |
|---|---|---|---|---|
| Fibres (g) | 0,14 | 0,14 | 0,14 | 0,14 |
| Matière sèche (%) | 33,98 | 33,85 | 33,86 | 33,87 |
| Lactose (%) | 4,09 | 4,06 | 4,06 | 4,06 |
| ESDL (%) | 3,84 | 3,95 | 3,95 | 3,95 |
| Taux de substitution de la protéine de lait (%) | 0 | 50 | 50 | 50 |

Le procédé de fabrication est le suivant :

∘ Ajouter le lait écrémé dans le récipient (40/45°C),
∘ Ajouter les ingrédients en poudre dans le récipient et mélanger pendant 15 minutes à 80 Hz dans le batch cooker CHOCOTEC,
∘ Mélanger les stabilisants et le sucre ensemble, puis incorporer le mélange dans le récipient,
∘ Mélanger pendant 20 minutes à 80 Hz,
∘ Incorporer la crème et le sirop de glucose
∘ Mélanger pendant 15 minutes à 80 Hz
∘ Pasteuriser à 80°C pendant 3 minutes,
∘ Refroidir à 70°C - La moitié du mélange obtenu est homogénéisé directement ; l'autre moitié est refroidie à 50°C. Quand le premier lot est homogénéisé, le deuxième lot est réchauffé à 70°C et ensuite homogénéisé,
∘ Homogénéiser à 200 bar,
∘ Refroidir dans le récipient de maturation à 4°C et ajouter l'arôme
∘ Laisser mâturer durant 23 h
∘ Fouetter pour obtenir un foisonnement de 95 - 100 % et congeler à -30°C pendant 1 heure
∘ Conserver la crème glacée à -20°C.

**Analyses**

*Caractérisation des mélanges durant le procédé de fabrication*

|  | Témoin | Recette n°1 | Recette n°2 | Recette n°3 |
|---|---|---|---|---|
| pH avant maturation | 6,72 (0,5°C) | 7,00 (1°C) | - | 6,90 (0,6°C) |
| pH après maturation | 6,73 (-1,5°C) | 7,07 (0,1°C) | 6,82 (-2,4°C) | 7,01 (-2°C) |
| Foisonnement du mélange (%) | 103 | 111 | 97 | 98 |
| Température de sortie (°C ) | -5,4 | -5,6 | -6 | -5,6 |

On note que l'aptitude au foisonnement des préparations réalisées avec les isolats de protéines de pois conforme à l'invention est identique à celle du témoin et n'est pas significativement différent de celle réalisée avec la protéine de pois.

*Mesures de la viscosité*

| Viscosité (Pa.s) | | | |
|---|---|---|---|
|  | Maturation | 10 s-1 | 100 s-1 |
| Témoin | Avant | 0,24 | 0,098 |
|  | Après | 0,23 | 0,095 |
| Recette n°1 | Avant | 0,36 | 0,18 |
|  | Après | 0,35 | 0,2 |
| Recette n°2 | Avant | 0,18 | 0,10 |
|  | Après | 0,18 | 0,10 |
| Recette n°3 | Avant | 0,2 | 0,11 |
|  | Après | 0,16 | 0,098 |

La recette avec la protéine de pois présente les plus fortes viscosités. Les recettes avec isolat de protéines de pois conformes à l'invention sont équivalentes à la recette témoin.

*Analyse des tailles de particules*

L'analyse de la taille des particules a été réalisée à différentes étapes de préparation de la crème glacée dans le but d'évaluer la capacité émulsifiante et la stabilité de l'émulsion :

◦ Distribution des tailles des globules gras après l'étape d'homogénéisation,
◦ Distribution des tailles de globules gras après l'étape de maturation,
◦ Distribution des tailles des globules gras de la crème glacée (équivalent à la distribution des tailles des globules gras après l'étape de fouettage).

Ces analyses ont été également faites avec addition de SDS à 0,1 % afin de déterminer si l'émulsion a été créée par agrégation / floculation ou par coalescence.

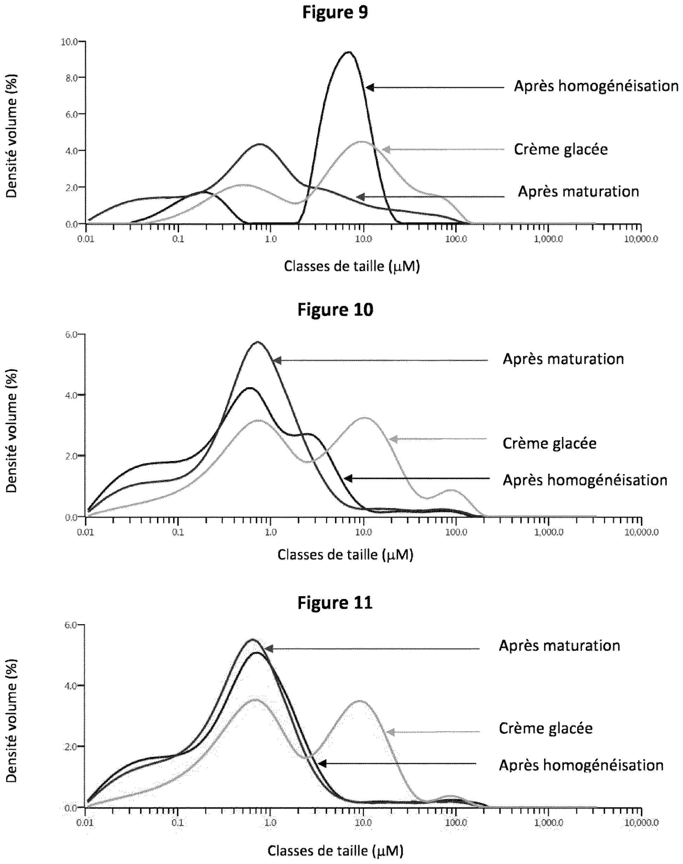

Les résultats sont présentés dans les **Figures 8 à 11.**

Pour chaque recette, la distribution des tailles de particules a tendance à diminuer ou à devenir plus monomodale après maturation.

Cette évolution est très perceptible pour la recette avec la protéine de pois NUTRALYS ® S85F. Cela montre que la protéine de pois NUTRALYS® S85F est le plus lent émulsifiant pour migrer à l'interface des globules gras.

Au contraire, la recette n°3 (avec l'isolat de protéines de pois conforme à l'invention n°2) est aussi bon émulsifiant que la recette à 100 % protéines de lait.

L'isolat de protéines de pois conforme à l'invention n°1 est moins émulsifiant que l'isolat de protéines de pois conforme à l'invention n°2 après homogénéisation mais a tendance à être aussi bon après maturation.

**Exemple 10** : **Substitution totale des protéines de lait par les isolats de protéines de pois dans les crèmes glacées/desserts glacés**

3 recettes sont élaborées pour ces crèmes glacées végétaliennes :

◦ Témoin : 100 % protéines de pois NUTRALYS® S85F,

◦ Recette 1 : 100 % d'isolats de protéines de pois selon l'invention n°1,

◦ Recette 2 : 100 % d'isolats de protéines de pois selon l'invention n°2.

|  | Témoin | Recette 1 | Recette2 |
|---|---|---|---|
| Eau | 64,25 | 64,25 | 64,25 |
| Saccharose | 12,00 | 12,00 | 12,00 |
| Huile de coco hydrogénée | 8,00 | 8,00 | 8,00 |
| Sirop de glucose Roquette 4280 | 11,50 | 11,50 | 11,50 |
| NUTRALYS® S85F | 3,50 | 0,00 | 0,00 |
| Stabilisants (CREMODAN SE30) | 0,25 | 0,25 | 0,25 |
| Arôme vanille IFF 10836706 | 0,50 | 0,50 | 0,50 |
| Isolat de protéines de pois selon l'invention n°1 | 0,00 | 3,50 | 0,00 |
| Isolat de protéines de pois selon l'invention n°2 | 0,00 | 0,00 | 3,50 |
| Total | 100 % | 100 % | 100 % |

Les quantités étant indiquées en pourcentage en poids.

Les valeurs nutritionnelles (pour 100 g sont les suivantes) :

| | Témoin | Recette 1 | Recette2 |
|---|---|---|---|
| Valeur énergétique (kcal) | 172 | 172 | 172 |
| Matières grasses totales | 8,5 | 8,5 | 8,5 |
| dont matières grasses saturées | 7,6 | 7,6 | 7,6 |
| Hydrates de carbone, sans fibres | 21,2 | 21,2 | 21,2 |
| dont les sucres | 14,6 | 14,6 | 14,6 |
| Fibres | 0,1 | 0,1 | 0,1 |
| Protéines | 2,8 | 2,8 | 2,8 |
| Sel (sodiumx2,5) | 0,11 | 0,14 | 0,14 |
| Matière sèche | 33,1 | 33,2 | 33,2 |

Le procédé de fabrication est le suivant :

○ Chauffer l'eau à 45°C,
○ Mélanger les ingrédients,
○ Mélanger les stabilisants avec le saccharose,
○ Ajouter l'eau et mélanger pendant 20 minutes,
○ Introduire la matière grasse (huile de coco fondue) et mélanger,
○ Pasteuriser à 80°C pendant 3 minutes
○ Refroidir à 70°C,
○ Homogénéiser le mélange à 200 bars (en 2 étages) - 30 % dans le 2ème étage,
○ Ajouter l'arôme,
○ Laisser mâturer sous agitation à 4°C pendant 20 minutes,
○ Fouetter entre 90 - 100 % et refroidir à -30°C pendant 1 heure,
○ Stocker à - 18°C.

## Analyses

### *Mesure du pouvoir foisonnant (desserts glacés)*

○ Pesée d'une coupelle de volume V donné, vide Masse mesurée = $m_c$ où $m_c$ est la masse de la coupelle vide
○ Pesée d'une coupelle de volume V donné, remplie à ras bord par le mélange avant foisonnement
Masse mesurée= mc + $m_{mix}$ où $m_{mix}$ est la masse de mélange correspondant au volume V
○ Pesée d'une coupelle de volume V donné, remplie à ras bord par le mélange après foisonnement (sortie de congélateur)
Masse mesurée = mc + $m_{glace}$ où $m_{glace}$ est la masse de glace (mélange foisonné en sortie du congélateur) correspondant au volume V.

La mesure de foisonnement est alors donnée par la formule :

$$Foisonnement = \frac{(m_{mix} - m_{glace})}{m_{glace}} \times 100$$

### *Caractérisation du procédé de préparation*

|  | Témoin | Recette 1 | Recette2 |
|---|---|---|---|
| pH après maturation | 7,25 | 6,84 | 6,89 |
| Densité du mélange (g/ml) | 1,05 | 1,06 | 1,07 |
| Pouvoir foisonnant (%) | 89 | 80 | 101 |
| Température à la sortie du congélateur | -5 | -4,8 | -4,8 |

*Mesure de viscosité*

- ∘ Mesures réalisées à 4°C
- ∘ Rhéomètre : Physica MCR 301 Anton Paar
- ∘ Géométrie : cylindre concentrique CC27
- ∘ Consigne : 0 à 200 s$^{-1}$ en 5 minutes

| Avant maturation | Viscosité (mPa.s) | | |
|---|---|---|---|
| Référence | 10 s$^{-1}$ | 100 s$^{-1}$ | 200 s$^{-1}$ |
| Témoin | 131 | 60 | 50 |
| Recette 1 | 23 | 51 | 40 |
| Recette 2 | 22 | 50 | 39 |

| Après maturation | Viscosité (mPa.s) | | |
|---|---|---|---|
| Référence | 10 s$^{-1}$ | 100 s$^{-1}$ | 200 s$^{-1}$ |
| Témoin | 120 | 65 | 56 |
| Recette 1 | 76 | 50 | 48 |
| Recette 2 | 74 | 50 | 44 |

On note ainsi une plus faible viscosité lorsque la recette comporte des isolats de protéines de pois selon l'invention.

*Mesure de la texture*

- ∘ Température de mesure : sortie du congélateur,
- ∘ Rhéomètre : Machine INSTRON 9506
- ∘ Géométrie : conique
- ∘ Consigne : déformation imposée jusqu'à 20 minutes,

|  | Dureté (N) | | |
|---|---|---|---|
| Durée de stockage au congélateur avant mesure | 7 jours | 14 jours | 30 jours |
| Témoin | 38,4 | 42,7 | 60,5 |
| Recette 1 | 126,2 | 128,4 | 137 |

(suite)

| | Dureté (N) | | |
|---|---|---|---|
| Durée de stockage au congélateur avant mesure | 7 jours | 14 jours | 30 jours |
| Recette 2 | 76 | 67,9 | 63 |

On constate que la dureté est globalement meilleure pour les recettes avec isolats de protéines de pois selon l'invention. Plus particulièrement, l'isolat de protéines de pois selon l'invention n°2 présente une dureté remarquablement élevé, sans doute en relation avec son pouvoir foisonnant plus élevé (101 %).

*Mesure de la taille de l'émulsion du mélange et du dessert glacé*

Protocole :

◦ Analyseur laser de taille de particules (granulomètre) MALVERN 3000 en voie liquide (le solvant est de l'eau déminéralisée)
◦ Modèle optique : 1,46+0.001i avec une vitesse d'agitation de 1900 tours/minutes.

Les mélanges avant et après maturation sont caractérisés avec et sans SDS :

◦ Sans SDS : l'échantillon est introduit directement dans le bécher du granulomètre ne contenant que de l'eau,
◦ Avec SDS : 0,1 %, soit 0,6 g de SDS est introduit directement dans le bécher du granulomètre. Après solubilisation du SDS, l'échantillon est ajouté pour analyse.

La crème glacée finale est introduite non décongelée dans le bol du granulomètre. Après fusion et dispersion de la glace, la mesure est effectuée.

La taille de l'émulsion, avant et après maturation, avec et sans SDS, est donnée dans le tableau suivant.

| Avant maturation, sans SDS | Dx (10) $\mu$m | Dx (50) $\mu$m | Dx (90) $\mu$m | Dmode $\mu$m | D (4,3) $\mu$m |
|---|---|---|---|---|---|
| Témoin | 0,073 | 0,377 | 3,380 | 0,429 | 1,410 |
| Recette 1 | 0,693 | 10,300 | 22,200 | 13,000 | 11,400 |
| Recette 2 | 0,520 | 8,400 | 18,200 | 10,000 | 9,310 |

Sans SDS, l'émulsion du mélange contenant de la protéine de pois (témoin) présente une taille de particules plus petite que les émulsions préparées à partir des isolats de protéines de pois selon l'invention.

| Avant maturation, avec SDS | Dx (10) $\mu$m | Dx (50) $\mu$m | Dx (90) $\mu$m | Dmode $\mu$m | D (4,3) $\mu$m |
|---|---|---|---|---|---|
| Témoin | 0,103 | 0,340 | 1,010 | 0,389 | 0,698 |
| Recette 1 | 0,065 | 0,605 | 5,110 | 0,621 | 1,650 |
| Recette 2 | 0,115 | 0,503 | 1,830 | 0,564 | 0,913 |

Avec SDS, les agglomérats de matières grasses sont dispersés, and le Dmode est ainsi plus proche pour les 3 essais. A noter que la formule avec l'isolat de protéines de pois selon l'invention n°1 présente un pic de granulométrie avec de plus larges particules.

| Après maturation, sans SDS | Dx (10) $\mu$m | Dx (50) $\mu$m | Dx (90) $\mu$m | Dmode $\mu$m | D (4,3) $\mu$m |
|---|---|---|---|---|---|
| Témoin | 0,085 | 0,379 | 2,690 | 0,433 | 1,210 |
| Recette 1 | 0,102 | 5,300 | 16,200 | 8,470 | 6,780 |
| Recette 2 | 0,088 | 4,460 | 15,400 | 7,820 | 6,150 |

| Après maturation, avec SDS | Dx (10) $\mu$m | Dx (50) $\mu$m | Dx (90) $\mu$m | Dmode $\mu$m | D (4,3) $\mu$m |
|---|---|---|---|---|---|
| Témoin | 0,103 | 0,329 | 0,894 | 0,381 | 0,658 |
| Recette 1 | 0,039 | 0,509 | 4,830 | 0,616 | 1,620 |
| Recette 2 | 0,098 | 0,504 | 2,070 | 0,573 | 1,110 |

On ne constate aucun changement majeur après maturation. Les formules avec les isolats de protéines de pois selon l'invention sont plus polydisperses par rapport à celle avec les protéines de pois.

La taille d'émulsion de la crème glacée, en tant que telle, est mesurée sans présence de SDS.

| Sans SDS | Dx (10) $\mu$m | Dx (50) $\mu$m | Dx (90) $\mu$m | Dmode $\mu$m | D (4,3) $\mu$m |
|---|---|---|---|---|---|
| Témoin | 0,092 | 0,333 | 3,950 | 0,352 | 2,390 |
| Recette 1 | 0,131 | 0,776 | 15,900 | 0,531 | 5,820 |
| Recette 2 | 0,178 | 0,814 | 51,400 | 0,518 | 14,500 |

La taille du pic majeur (Dmode) est similaire pour les trois crèmes glacées. Cependant, les formules avec les isolats de protéines de pois selon l'invention sont plus polydisperses, spécialement avec l'isolat n°2.

Une étude comparative a été réalisée avec des glaces commerciales, qui montrent que ces dernières ont encore plus de particules grossières que les recettes témoins, 1 et 2, en relation avec leur contenu élevée en globules de matières grasses.

*Mesure du comportement à la fonte*

Protocole :
De manière empirique, des échantillons de dessert glacés d'un volume donné sont posés sur une grille au-dessus d'un bécher. On mesure ensuite :

- ◦ Le temps au bout duquel la première goutte tombe dans le bécher,
- ◦ Le % de glace fondue au cours du temps, pendant 3 heures.

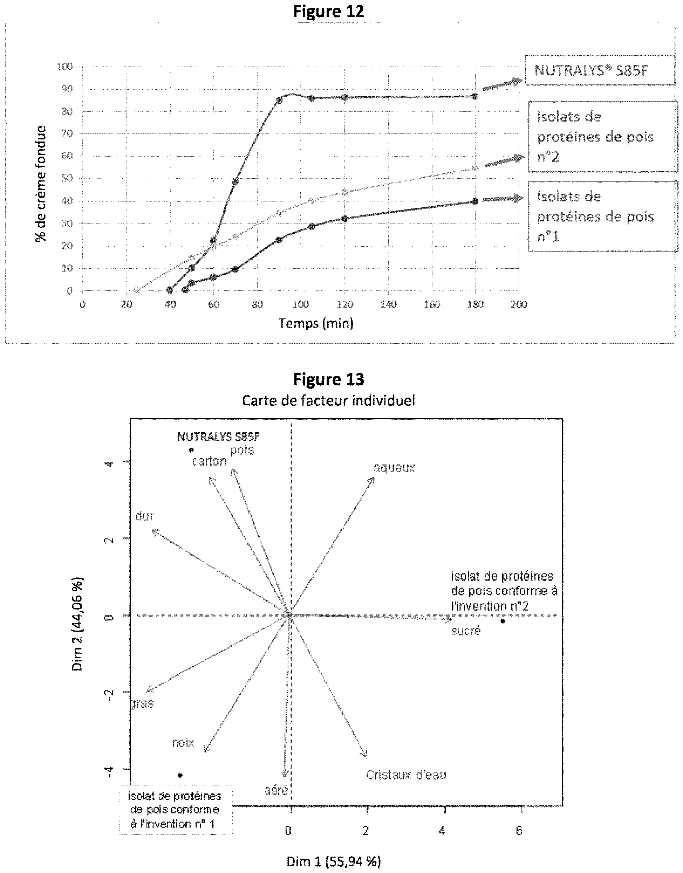

La **Figure 12** illustre bien le fait que la fonte est plus faible pour les crèmes glacées préparées à partir des isolats de protéines de pois selon l'invention.

*Analyse sensorielle*

Le panel est constitué de 15 personnes.

Le panel est, comme dans les exemples précédents, qualifié à la dégustation de produits formulés avec la protéine de pois. Il a reçu une formation et un entraînement afin de vérifier ses performances en termes de :

- • Capacité à discriminer les produits
- • Consensus, utilisation correcte des descripteurs
- • Répétabilité, capacité à repérer un doublon.

Comparés aux glaces préparées avec les protéines de pois, celles de l'invention sont moins amères, ont moins le goût de pois et sont moins colorées.

Les desserts glacés avec les isolats de protéines de pois selon l'invention n°1 présentent un peu de cristaux de glace et un goût vanille plus prononcé, sont plus sucrés, et plus gras que les autres produits.

Les desserts glacés avec les isolats de protéines de pois selon l'invention n°2 sont sucrés et gras, plus crémeux. Ils présentent un goût « thé vert » un peu plus prononcé.

*Conclusion*

Durant le procédé de fabrication des desserts glacés, les isolats de protéines de pois selon l'invention conduisent à une plus faible viscosité en comparaison avec les protéines de pois.

La texture avec l'isolat n°1 est plus dure, mais n'est pas perçue par les panélistes.

Les deux isolats conduisent surtout à abaisser la fonte des desserts glacés correspondant.

En terme de goût, la meilleure perception est pour les desserts glacés préparés avec l'isolat n°1, de saveur sucrée et d'arôme prononcé, de moindres amertume et saveur « pois ».

**Exemple 11** : **comparaison des propriétés sensorielles de crèmes glacées**

Le panel est constitué de 20 personnes.

Le panel est qualifié à la dégustation de produits formulés avec la protéine de pois. Il a reçu une formation et un entraînement afin de vérifier ses performances en termes de :

- Capacité à discriminer les produits
- Consensus, utilisation correcte des descripteurs
- Répétabilité, capacité à repérer un doublon

En effet, il a reçu une formation pour la bonne utilisation des descripteurs sensoriels de goût et de texture, tels que, par exemple :

| Descripteur | Définition | Mode opératoire | Référence |
|---|---|---|---|
| **Flaveurs** | | | |
| **Goût Pois** | Goût typique de pois. | Goûtez le produit. | Pois |
| **Sucré** | Saveur élémentaire provoquée par une solution de saccharose. | Goûtez le produit. | Sucre en poudre |
| **Texture en bouche** | | | |
| **Dur** | Evaluation de la force nécessaire pour obtenir une déformation ou une rupture du produit. (pas dur / très dur) | Comprimez une unité de produit entre les dents incisives. | Confiserie type sucre cuit |
| **Aéré** | Evaluation de la quantité de bulles d'air emprisonnées et visibles. (pas aéré / très aéré) | Explorez visuellement et tactilement en déplaçant le doigt parallèlement à l'état de surface et exercez des pressions différentes de haut en bas. | Mousse au chocolat |
| **Aqueux** | Evaluation de propriété de | Dégustez une unité de produit | Pastèque |

| | | | |
|---|---|---|---|
| | texture de la surface qualifiant la perception de la quantité d'eau libérée par un produit. (pas aqueux / très aqueux) | et évaluez la quantité d'eau perçue en bouche. | |
| **Cristaux d'eau** | Evaluation de la présence de cristaux d'eau (pas de cristaux / beaucoup de cristaux) | Frottez la langue contre le palais et vérifiez si le produit contient des cristaux | Glace recristallisée |
| **Gras** | Evaluation de la pellicule grasse après déglutition. (pas gras / très gras) | Après déglutition d'une unité de produit, évaluez la présence ou non d'une pellicule grasse au niveau du palais ou des dents en balayant leur surface avec la langue. | Huile d'olive |

La méthode leur permet également de s'exprimer sur d'autres descripteurs qui n'auraient pas été anticipés dans cette liste.

*Produits*

Les crèmes glacées sont les recettes n°1, n°2 et n°3 celles de l'exemple 9.

*Conditions de dégustation*

- Au laboratoire d'analyse sensorielle : box individuels de dégustation, murs blancs, ambiance calme (pour faciliter la concentration)
- Lumière blanche (pour avoir exactement la même vision du produit)
- En fin de matinée ou d'après-midi (pour être au maximum des capacités sensorielles)
- Produits anonymés par un code à 3 chiffres (pour éviter que le code n'influence l'appréciation des produits)
- Produits présentés dans un ordre aléatoire (pour éviter les effets d'ordre et de rémanence)

*Exercice*

La méthode employée pour comparer les produits a été le Profil Flash (JM Sieffermann, 2000).

Les produits sont tous présentés simultanément. Il s'agit de comparer les produits entre eux en réalisant une succession de classements : les panélistes choisissent les descripteurs qui leur semblent les plus pertinents pour discriminer les produits entre eux et classent les produits selon ces descripteurs, il est possible que plusieurs produits soient regroupés sur un même rang.

Exemple :

Descripteur sensoriel : *Croustillant*

*Traitement des données*

La méthode de traitement statistique adaptée à ce type de données est l'Analyse Factorielle Multiple (J. Pagès, 1994) sur les données-rangs des produits. Pour que les résultats soient plus clairs, l'AFM a été réalisée plusieurs fois; en global, et par critère (aspect, odeur, goût, texture). Les graphiques présentés synthétisent l'ensemble des résultats fournis par cette méthode.

Les analyses ont été réalisées à l'aide du logiciel R (en vente libre) :

R version 2.14.1 (2011-12-22)
Copyright (C) 2011 The R Foundation for Statistical Computing
ISBN 3-900051-07-0
20 Platform: i386-pc-mingw32/i386 (32-bit)

Le logiciel est un environnement de travail qui nécessite le chargement de modules contenant les fonctions de calcul comme le package FactoMineR version 1.19

**Résultats**

Les résultats sont présentés dans la **Figure 13.**

Les 3 échantillons sont tous évalués en texture onctueux/crémeux, froid et fondant et en goût pois, vanille et amer.

Néanmoins quelques descripteurs permettent de les différencier :

- La crème glacée au NUTRALYS® S85F apparaît plus dure avec un goût de pois et carton.

- Celle avec isolat de protéines de pois conforme à l'invention n° 1 est plus grasse et aérée avec une note noix.

- Celle avec isolat de protéines de pois conforme à l'invention n° 2 est jugée plus sucrée.

**Exemple 12** : **Utilisation des isolats de protéines de pois dans des matrices de « non dairy coffee creamers/whiteners »**

**a. Substitution de 100 % des caséinates de sodium**

L'objectif est ici de substituer 100 % des caséinates de sodium et d'obtenir un produit stable dans le café.
La mesure de la viscosité des émulsions après pasteurisation et celle de la stabilité dans le café permettent d'illustrer l'amélioration des propriétés fonctionnelles des isolats de protéines de pois par rapport au NUTRALYS® dans leurs capacités à substituer les caséinates de sodium.
Les recettes élaborées sont les suivantes :

| | Recette 1 (Recette Contrôle) | Recette 2 | Recette 3 | Recette 4 |
|---|---|---|---|---|
| | 100 % caséinate de sodium (60 % MS) | 100 % isolats de protéines de pois selon l'invention n°1 (60 % MS) | 100 % isolats de protéines de pois selon l'invention n°2 (60 % MS) | 100 % NUTRALYS® S85F (60% MS) |
| | % | % | % | % |
| Sirop de glucose 3072 (Roquette FRERES) | 45,85 | 45,85 | 45,85 | 45,85 |
| Huile de coco hydrogénée 32-34 (Dislab) | 23,36 | 23,36 | 23,36 | 23,36 |
| NUTRALYS® S85F batch WB67J | | | | 1,75 |
| Isolats de protéines de pois selon l'invention n°1 | | 1,75 | | |
| Isolats de protéines de pois selon l'invention n°2 | | | 1,75 | |
| Caséinate de sodium EM7 (DMV) | 1,75 | | | |
| $K_2HPO_4$ _E340 (Merck) | 1,46 | 1,46 | 1,46 | 1,46 |
| Dimodan HP_E471 (Danisco) | 0,58 | 0,58 | 0,58 | 0,58 |
| Eau | 27,00 | 27,00 | 27,00 | 27,00 |
| *TOTAL* | *100,00* | *100,00* | *100,00* | *100,00* |

Les quantités étant données en pourcentage en poids.
Le procédé de fabrication est le suivant :

○ Fondre la matière grasse à 80° sous agitation constante,

∘ Ajouter le Dimodan HP dans la matière grasse fondue pour solubiliser les monoglycérides,

∘ Chauffer 90 % de l'eau à 50°C et ajouter les protéines. Hydrater sous agitation constante pendant 30 minutes,

∘ Solubiliser les sels de phosphates dans l'eau résiduelle à 40°C,

∘ Après 30 minutes d'hydratation, ajouter le sirop de glucose et les sels de phosphates dans le mélange principal,

∘ Pré-émulsifier le mélange de matières grasses/Dimodan HP dans le mélange principal pendant 5 minutes à 10.000 rpm,

∘ Placer le produit à 75°C dans un homogénéisateur à haute pression Niro Panda 2K Soavi (GEA) à une pression de 160 bar au 1er étage, 30 bar dans le deuxième étage,

∘ Pasteuriser à 80°C durant quelques secondes puis placer le produit dans l'eau froide pour stopper le traitement thermique

Les analyses réalisées sur la formulation sont les suivantes :

*1. Analyse de viscosité*

Les mesures de viscosité des émulsions concentrées après l'étape de traitement thermique sont réalisées à 65°C, température habituelle d'atomisation.
Appareillage :

∘ Rhéomètre Physica MCR 301 Anton Paar
∘ Géométrie : CC27
∘ Méthode 0 à 1000 s$^{-1}$ en 660 s

Les résultats obtenus sur les différentes recettes sont les suivants :

|  | Viscosity (mPa.s) | | | | |
|---|---|---|---|---|---|
|  | 5s-1 | 10s-1 | 40s-1 | 100s-1 | 1000s-1 |
| Recette Contrôle | 89 | 69 | 47 | 44 | 42 |
| Recette 2 | 77 | 59 | 40 | 34 | 24 |
| Recette 3 | 77 | 60 | 42 | 36 | 26 |
| Recette 4 | 775 | 530 | 270 | 197 | 85 |

Les viscosités des émulsions des recettes 2 et 3 après pasteurisation sont plus proches du témoin lait que celle de la recette 4 préparée avec de la protéine de pois, ce qui permet de sécher une émulsion de faible viscosité à haute matière sèche comme ici à 60 % en poids.

*2. Solubilité en fonction du pH*

|  | NUTRALYS S85F | isolats de protéines de pois selon l'invention n°1 | isolats de protéines de pois selon l'invention n°2 | Caséinate de sodium EM7 (DMV) |
|---|---|---|---|---|
| pH 3 | 50,3 | 53,3 | 58,9 | 82,0 |
| pH 4 | 15,2 | 39,7 | 38,6 | 7,0 |

(suite)

|  | NUTRALYS S85F | isolats de protéines de pois selon l'invention n°1 | isolats de protéines de pois selon l'invention n°2 | Caséinate de sodium EM7 (DMV) |
|---|---|---|---|---|
| pH 5 | 11,3 | 37,7 | 36,8 | 9,0 |
| pH 6 | 21,2 | 50,3 | 53,9 | 94,0 |
| pH 7 | 36,8 | 54,8 | 59,9 | 94,0 |
| pH 8 | 55,1 | 57,4 | 62,4 | 94,0 |

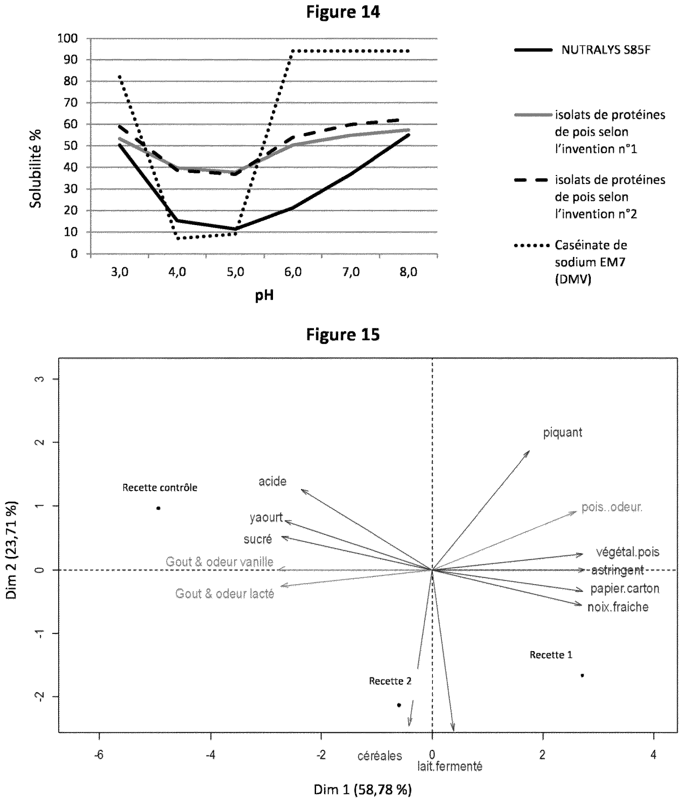

La **Figure 14** illustre l'évolution de la solubilité des isolats de protéines de pois selon l'invention par rapport au caséinate, en fonction du pH, et traduit leur excellent comportement.

*Evaluation de la stabilité dans le café*

*Reconstitution du* café :

◦ Peser 2 g de café soluble
◦ chauffer de l'eau de consommation (charge en calcium de 136mg et en magnésium de 60mg) à 80°C et ajouter 135 g de ladite eau au 2 g,
◦ ajouter 12,7 g d'émulsion concentrée dans le café.

La floculation dans le café semble être moins importante avec les recettes contenant les isolats de protéines de pois selon l'invention, par rapport à celle obtenue avec la protéine de pois. Cependant, ce peut être corrélé avec l'amélioration de la solubilité desdits isolats par rapport à la protéine de pois.

**b. Substitution de 50 % des caséinates de sodium**

L'objectif est ici de substituer 50 % des caséinates de sodium et d'obtenir un produit stable dans le café.
La mesure de la viscosité des émulsions après pasteurisation et celle de la stabilité dans le café permettent d'illustrer l'amélioration des propriétés fonctionnelles des isolats de protéines de pois par rapport au NUTRALYS® dans leurs capacités à substituer les caséinates de sodium.
Les recettes élaborées sont les suivantes :

|  | Recette 1 (Recette Contrôle) | Recette 2 | Recette 3 |
|---|---|---|---|
|  | 100 % caséinate de sodium (60 % MS) | 50 % isolats de protéines de pois selon l'invention n°2 + 50 % caséinate de sodium (60 % MS) | 50% NUTRALYS® S85F + 50 % caséinate de sodium (60% MS) |
|  | % | % | % |
| Sirop de glucose 3072 (Roquette FRERES) | 45,85 | 45,85 | 45,85 |
| Huile de coco hydrogénée 32-34 (Dislab) | 23,36 | 23,36 | 23,36 |
| NUTRALYS® S85F batch WB67J |  |  | 0,87 |

(suite)

|  | Recette 1 (Recette Contrôle) 100 % caséinate de sodium (60 % MS) | Recette 2 50 % isolats de protéines de pois selon l'invention n°2 + 50 % caséinate de sodium (60 % MS) | Recette 3 50% NUTRALYS® S85F + 50 % caséinate de sodium (60% MS) |
|---|---|---|---|
|  | % | % | % |
| Isolats de protéines de pois selon l'invention n°2 |  | 0,87 |  |
| Caséinate de sodium EM7 (DMV) | 1,75 | 0,88 | 0,88 |
| Joha@ KM2_E339_E452_E331 | 1,46 | 1,46 | 1,46 |
| Dimodan HP_E471 (Danisco) | 0,58 | 0,58 | 0,58 |
| Eau | 27,00 | 27,00 | 27,00 |
| TOTAL | 100,00 | 100,00 | 100,00 |

Les quantités étant données en pourcentage en poids.

Les valeurs nutritionnelles pour 100 g sont les suivantes.

| N° de la recette | Recette 1 | Recettes 2 et 3 |
|---|---|---|
| Humidité (%) | 40 | 40 |
| Energie calorique (kCal) pour 100 g | 369 | 369 |
| Matières grasses (g) | 24,0 | 24,0 |
| Teneur en protéines (g) | 1,6 | 1,5 |
| *Dont protéines de lait* | 1,6 | 0,8 |
| *isolat protéines de pois* | 0 | 0,7 |
| Hydrates de carbone (g) | 33,0 | 33,0 |
| *Dont sucres* | 5,3 | 5,3 |

Le procédé de fabrication est le suivant :

◦ Fondre la matière grasse à 80° sous agitation constante,
◦ Solubiliser les mono et diglycérides dans l'huile liquide,
◦ Solubiliser les protéines en poudre dans de l'eau à 50°C pendant 30 minutes,
◦ Ajouter le sirop de glucose et les sels de phosphates déjà solubilisé dans une part d'eau
◦ Pré-émulsifier les matières grasses fondues dans la solution aqueuse par agitation à 10.000 rpm
◦ Pasteuriser à 80°C durant quelques secondes
◦ Placer le produit à 75°C dans un homogénéisateur à haute pression Niro Panda 2K Soavi (GEA) à une pression de 160 bar au 1$^{er}$ étage, 30 bar dans le deuxième étage,
◦ Diluer le mélange à 50 % de Matière Sèche pour atomiser à 180°C ($T_{inlet}$) et 90°C ($T_{outlet}$) dans dispositif présentant une capacité d'évaporation de 10 à 12 l/h.

Les analyses réalisées sur la formulation sont les suivantes :
1) pH de l'émulsion

| pH | Recette 1 | Recette 2 | Recette 3 |
|---|---|---|---|
| Sur l'émulsion à 25°C | 7,69 | 9,24 | 8,72 |
| Sur le café à 75°C | 6,45 | 6,35 | 6,10 |

2) Capacité d'émulsion

La mesure de la taille des globules lipidiques (par analyseur laser de taille de particules) permet de déterminer la capacité des isolats de protéines de pois selon l'invention à former les globules lipidiques de taille la plus petite possible.

| | Dx(10) ($\mu$m) | Dx(50) ($\mu$m) | Dx(90) ($\mu$m) | D [4,3] ($\mu$m) | Mode ($\mu$m) |
|---|---|---|---|---|---|
| Recette 1 | 0,281 | 0,577 | 1,23 | 0,681 | 0,551 |
| Recette 2 | 0,289 | 0,563 | 1,10 | 0,668 | 0,559 |
| Recette 3 | 0,279 | 0,564 | 1,23 | 1,15 | 0,534 |

Ces résultats montrent bien que le mélange 50/50 présente une distribution granulométrique semblable au témoin 100 % caséinate.

3) Viscosité des émulsions à 60% de matière sèche à 65 °C (avant atomisation) Appareillage :

- ◦ Rhéomètre Physica MCR 301 Anton Paar
- ◦ Géométrie : CC27
- ◦ Méthode 0 à 1000 s$^{-1}$ en 660 s

| | Viscosité (mPa.s) | | | |
|---|---|---|---|---|
| | 5 s$^{-1}$ | 10 s$^{-1}$ | 40 s$^{-1}$ | 100 s$^{-1}$ |
| Recette 1 | 89 | 69 | 47 | 44 |
| Recette 2 | 49 | 48 | 43 | 39 |
| Recette 3 | 170 | 132 | 89 | 77 |

La plus faible viscosité du mélange 50/50 permet d'atomiser à une matière sèche supérieure à celle requise classiquement pour les caséinates.

*4) Stabilisation du « non dairy coffee creamer » en poudre dans le café*

*Reconstitution du café :*

a. Peser 2 g de café soluble

b. ajouter 8 g d'émulsion et 150 ml d'eau de consommation (charge en calcium de 136 mg et en magnésium de 60 mg) à 80°C

La stabilité de l'émulsion dans le café est déterminée par la mesure de la variation de couleur de la préparation - mesure de coloration selon les codifications L (balance des blancs), a (balance des jaunes) et b (balance des verts), la couleur blanche dans le café étant un des critères clefs recherchés par l'industriel et le consommateur.

L'écart de 2 points pour la mesure du paramètre L des cafés préparés à partir du mélange 50/50 (L= +96) en regard des cafés témoins préparés à partir de caséinates (L= +98) traduit une excellente stabilité du mélange avec les isolats de protéines de pois conformes à l'invention.

### Exemple 13. Utilisation des isolats de protéines de pois pour la préparation de yaourts brassés

L'objectif est ici de substituer 30 % des protéines de lait.

Les recettes élaborées sont les suivantes :

| En % | Recette Contrôle | Recette avec protéines de pois Recette 1 | Recette avec isolat de protéines de pois conformes à l'invention n°1 Recette 2 | Recette avec protéines de pois Recette 3 |
|---|---|---|---|---|
| Lait écrémé reconsititué | 88,80 | 74,50 | 74,50 | 74,50 |
| Crème (Les faves 35% MG) | 2,75 | 2,42 | 2,42 | 2,42 |
| Sucre | 6,00 | 6,00 | 6,00 | 6,00 |
| Amidon modifié CLEARAM® CR 4015 de Roquette Frères | 1,50 | 3,20 | 3,20 | 3,20 |
| NUTRALYS® S85F | | 1,40 | | |
| NUTRALYS® F85F | | | | 1,40 |
| Isolat protéines de pois selon l'invention n°1 | | | 1,40 | |
| PROMILK 852 A Ingredia | 0,77 | | | |
| Pectine CM 020 HERBSTREITH & FOX | 0,18 | 0,16 | 0,16 | 0,16 |
| Eau | | 12,32 | 12,32 | 12,32 |
| | | | | |
| Total | 100,00 | 100,00 | 100,00 | 100 |
| Les quantités étant données en pourcentage en poids. | | | | |
| MS (matière sèche) | 17,73 | 18,28 | 18,31 | 18,28 |
| | | | | |
| Protéines totales | 3,70 | 3,70 | 3,70 | 3,70 |
| Protéines laitières | 3,70 | 2,58 | 2,58 | 2,58 |
| Protéines végétales | 0,00 | 1,12 | 1,12 | 1,12 |
| Lipides | 1,01 | 1,01 | 1,01 | 1,01 |
| Glucides | 12,30 | 12,99 | 12,99 | 12,99 |
| dont sucres | 10,98 | 10,14 | 10,14 | 10,14 |
| Kcal/100g | 73,10 | 75,84 | 75,86 | 75,84 |
| | | | | |
| Taux de substitution | 0,00 | 30,19 | 30,19 | 30,19 |

Le procédé de fabrication est quant à lui le suivant :

◦ chauffer de l'eau à 60°C,

◦ ajouter les protéines et laisser hydrater pendant 1 heure,

◦ ajouter la crème sous POLYTRON durant 2 minutes,

∘ ajouter le mélange sucre/amidon pendant 10 à 15 minutes,

∘ homogénéiser à haute pression (2 étages : 1er étage 180 bars - 2éme étage 200 bars) à 75-80°C,

∘ pasteuriser avec un échangeur tubulaire Power Point International à 95°C, 6 minutes - 20 l/h,

∘ ajouter les ferments (YoFlex® YF-L812 - 50 U/250L),

∘ acidifier à 42°C jusqu'à pH 4,6 (durée d'acidification de 5 - 6 heures),

∘ agiter à 3600 rpm rt à 42°C,

∘ lisser à 37/38°C à 3600 rpm en Spindle 2G

∘ mettre en pot et stocker à 4°C.

*Mesure de la viscosité*

| | Température de mesure : 13°C |
|---|---|
| Rhéomètre : | Physica MCR 301 Anton Paar |
| Géométrie : | CC27 |
| méthode : | 0 à 350 s⁻¹ en 180 s et retour de 350 s⁻¹ à 0 en 180 s |

Les valeurs sont données à ± 5 %.

| | J+3 | | | | | |
|---|---|---|---|---|---|---|
| | Viscosité (Pa.s) | | | | | Surface d'hystérésis (Pa) |
| référence | $5s^{-1}$ | $10s^{-1}$ | $40s^{-1}$ | $100\ s^{-1}$ | $350\ s^{-1}$ | |
| Recette 2 | 4,3 | 2,6 | 1,03 | 0,53 | 0,2 | 4130 |
| Recette 1 | 3,9 | 2,38 | 0,98 | 0,52 | 0,21 | 3335 |
| Recette 3 | 3,76 | 2,3 | 0,97 | 0,53 | 0,22 | 2610 |
| Recette Contrôle | 3,12 | 2,1 | 0,79 | 0,4 | 0,15 | 2420 |

| | J+7 | | | | | |
|---|---|---|---|---|---|---|
| | Viscosité (Pa.s) | | | | | Surface d'hystérésis (Pa) |
| référence | $5s^{-1}$ | $10\ s^{-1}$ | $40\ s^{-1}$ | $100\ s^{-1}$ | $350\ s^{-1}$ | |
| Recette 2 | 4,12 | 2,49 | 1,02 | 0,535 | 0,212 | 3710 |
| Recette 1 | 3,26 | 2,21 | 0,84 | 0,41 | 0,16 | 2551 |
| Recette 3 | 3,71 | 2,29 | 0,96 | 0,52 | 0,21 | 2905 |
| Recette Contrôle | 4,17 | 2,51 | 1,02 | 0,53 | 0,2 | 4200 |

| | J+14 | | | | | |
|---|---|---|---|---|---|---|
| | Viscosité (Pa.s) | | | | | Surface d'hystérésis (Pa) |
| référence | 5s$^{-1}$ | 10 s$^{-1}$ | 40 s$^{-1}$ | 100 s$^{-1}$ | 350 s$^{-1}$ | |
| Recette 2 | 3,93 | 2,38 | 0,99 | 0,524 | 0,21 | 3260 |
| Recette 1 | 3,54 | 2,17 | 0,94 | 0,52 | 0,22 | 2420 |
| Recette 3 | 4,13 | 2,51 | 1,02 | 0,51 | 0,19 | 4860 |
| Recette Contrôle | 2,74 | 1,82 | 0,7 | 0,35 | 0,138 | 2200 |

La Recette 3 a le comportement le plus proche de la Recette contrôle avec cependant une inversion de la courbe de viscosité par rapport à l'évolution de la viscosité de la Recette contrôle à J+7 et J+14.

La recette 3 reprend en effet en viscosité à J+14, et est la plus résistante au cisaillement à J+14.

La recette 1 est plus visqueuse et résistante au cisaillement que la Recette 3 à J+7, mais cela s'inverse à partir de J+ 14.

La Recette 2, avec l'isolat de protéines de pois conforme à l'invention, est la plus visqueuse des 4 recettes, et est plus visqueuse que la recette Contrôle. Sa viscosité diminue au cours du temps.

Ces résultats démontrent que par son comportement, l'isolat de protéines de pois conforme à l'invention permettrait de diminuer la quantité d'amidon dans cette Recette s'il est souhaité de la rapprocher de la viscosité de la Recette Contrôle.

Même chose mais dans une moindre mesure pour les Recettes 1 et 3.

**Exemple 14** : **comparaison des propriétés sensorielles des yaourts brassés**

Pour l'évaluation du goût, le panel est constitué de 11 personnes. Pour celui de la texture, le panel est constitué de 12 personnes

Les panels sont qualifiés à la dégustation de produits formulés avec la protéine de pois. Ils ont reçu une formation et un entraînement afin de vérifier ses performances en termes de :

• Capacité à discriminer les produits
• Consensus, utilisation correcte des descripteurs
• Répétabilité, capacité à repérer un doublon

En effet, ils ont reçu une formation pour la bonne utilisation des descripteurs sensoriels de de goût et de texture, tels que, par exemple :

Descripteurs de goût :

| arôme / odeur | | | | saveur / sensation bouche |
|---|---|---|---|---|
| lacté | végétal | arôme | off flaveur | |
| lait | AG pois / végétal | vanille | papier / carton | acide |
| lactosérum | céréales | caramel | lessive | amer |
| lait fermenté | végétal | | chimique | salé |
| lait reconstitué bébé | noix fraiche | | colle | astringent / asséchant |
| yaourt | pomme de terre | | métallique | sucré |
| beurre | | | | piquant |

Descripteurs de texture

| Descripteur | Définition | Mode opératoire | Référence |
|---|---|---|---|
| **Aspect** | | | |
| **Brillant** | Aspect brillant ou lustré résultant de la tendance d'une surface à réfléchir la lumière. (pas brillant / très brillant) | Explorez visuellement le produit. | Jaune d'oeuf cru |
| **Granuleux** | Evaluation de la granulosité et du nombre et taille de particules d'un produit. (pas qranuleux / très granuleux) | Explorez visuellement le produit. | Sucre roux |
| **Texture à la cuillère** | | | |
| **Gélifié** | Evaluation de la consistance du produit. (pas gélifié / très gélifié) | Explorez visuellement le produit. | Gélatine |
| **Epais** | Evaluation de la facilité du produit à s'écouler sous une action mécanique. (pas épais / très épais) | Prenez une unité de produit avec une cuillère. Levez et tournez la cuillère. Vérifiez la capacité d'écoulement. | Miel |
| **Filant** | Evaluation de la capacité de filer sans se diviser en gouttes. (pas filant / très filant) | Appliquez la cuillère perpendiculairement à la surface, mettre en pression et la retirer verticalement doucement. | Eau |
| **Nappant** | Evaluation de la capacité de formation d'une nappe au dos de la cuillère. (pas nappant / très nappant) | Appliquez la cuillère perpendiculairement à la surface et la retirer verticalement et doucement. | Crème anglaise |
| **Texture en bouche** | | | |
| **Aqueux** | Evaluation de propriété de texture de la surface qualifiant la perception de la quantité d'eau libérée par un produit. (pas aqueux / très aqueux) | Dégustez une unité de produit et évaluez la quantité d'eau perçue en bouche. | Pastèque |
| **Asséchant** | Evaluation de la propriété de texture décrivant la perception de l'absorption d'humidité par le produit. (pas asséchant / très asséchant) | Mâchez une unité de produit et vérifiez si l'intérieur de la bouche devient sec. | Jus de canneberge |
| **Gras** | Evaluation de la pellicule grasse après déglutition. (pas gras / très gras) | Après déglutition d'une unité de produit, évaluez la présence ou non d'une pellicule grasse au niveau du palais ou des dents en balayant leur surface avec la lanque. | Huile d'olive |
| **Crémeux Onctueux** | Evaluation de la texture douce et fondante du produit. (pas onctueux / très onctueux) | Mâchez une unité de produit et vérifiez s'il provoque un contact mou et s'il tapisse la bouche. | Crème fraîche, crème épaisse |
| **Epais** | Evaluation de la facilité du produit à s'écouler en bouche. (pas épais / très épais) | Frottez la langue contre le palais et vérifiez si le produit s'écoule facilement. | Lait concentré sucré, miel |

(suite)

| Texture en bouche | | | |
|---|---|---|---|
| **Collant** | Evaluation de la force nécessaire pour décoller des produits qui adhèrent à l'intérieur de la cavité buccale. (pas collant / très collant) | Au bout de quelques mastications consécutives, pressez le produit entre les dents et mesurez la force nécessaire pour qu'il se décolle des dents. | Caramel mou |
| **Granuleux** | Evaluation de la granulosité et du nombre et taille de particules d'un produit. (pas granuleux / très granuleux) | Frottez la langue contre le palais et vérifiez si le produit contient des particules. | Sucre roux |

*Produits*

Les 3 produits de l'exemple 11 testés (recette contrôle, recette 1 et recette 2) ont été évalués 3 jours après leur production et ont été présentés à une température d'environ 10°C (produits stockés au réfrigérateur, évalués à sa sortie).

*Conditions de dégustation*

- Au laboratoire d'analyse sensorielle : box individuels de dégustation, murs blancs, ambiance calme (pour faciliter la concentration)
- Lumière blanche (pour avoir exactement la même vision du produit)
- En fin de matinée ou d'après-midi (pour être au maximum des capacités sensorielles)
- Produits anonymés par un code à 3 chiffres (pour éviter que le code n'influence l'appréciation des produits)
- Produits présentés dans un ordre aléatoire (pour éviter les effets d'ordre et de rémanence)

*Exercice*

La méthode employée pour comparer les produits a été le Profil Flash (JM Sieffermann, 2000).

Les produits sont tous présentés simultanément. Il s'agit de comparer les produits entre eux en réalisant une succession de classements : les panélistes choisissent les descripteurs qui leur semblent les plus pertinents pour discriminer les produits entre eux et classent les produits selon ces descripteurs, il est possible que plusieurs produits soient regroupés sur un même rang.

*Exemple :*

Descripteur sensoriel : *Croustillant*

Deux listes de descripteurs, relatives au goût ou à la texture, ont été proposées aux panélistes à titre indicatif, celles-ci vous sont fournies en annexe de ce rapport.

*Traitement des données*

La méthode de traitement statistique adaptée à ce type de données est l'Analyse Factorielle Multiple (J. Pagès, 1994) sur les données-rangs des produits. Pour que les résultats soient plus clairs, l'AFM a été réalisée plusieurs fois; en global, et par critère (aspect, odeur, goût, texture). Les graphiques présentés synthétisent l'ensemble des résultats fournis par cette méthode.

Les traitements statistiques ont été effectués avec le logiciel R version 2.14.1 (2011-12-22).

*Résultats* :

Les résultats sont présentés dans les **Figures 15** (goût) et **16** (texture) :

- Le yaourt brassé au NUTRALYS® S85F a une texture filante et granuleuse en bouche accompagnée d'un goût de pois, carton, noix fraiche ;
- Celui avec la protéine de lait apparaît plus gras et crémeux, épais avec un aspect granuleux, son goût est plus typique du yaourt, sucré et lacté ;
- Celui avec isolat de protéines de pois conforme à l'invention n° 1 se situe entre le contrôle et les essais avec NUTRALYS® S85F, se démarque par un goût céréale et lait fermenté ainsi qu'une texture particulièrement nappante en bouche.

## Exemple 15. Utilisation des isolats de protéines de pois pour la préparation de boissons laitières aromatisées à la fraise

L'objectif est ici de substituer 50 % des protéines de lait.

Les recettes élaborées sont les suivantes :

| | **Contrôle 100 % Protéines de lait** | **50% substitution avec l'isolat de protéines de pois selon l'invention n°1** | **50% substitution avec NUTRALYS® S85F** |
|---|---|---|---|
| Les quantités étant données en pourcentage en poids | % | % | % |
| Lait écrémé UHT | 86,7 | 56,1 | 56,1 |
| Eau | | 28,3 | 28,3 |
| Saccharose | 5,80 | 7,20 | 7,20 |
| Crème (35 % matières grasses) METRO | 4,70 | 4,20 | 4,20 |
| Concentrat de protéines de lait 4892 (Fonterra) | 1,05 | | |
| NUTRALYS® S85F batch WB67J | | | 2,40 |
| Isolat de protéines de pois selon l'invention n°1 | | 2,40 | |
| Purée de fraises (Metro) | 1,35 | 1,35 | 1,35 |
| Amidon modifié CLEARAM® CH 3020 Roquette Frères | 0,40 | 0,40 | 0,40 |
| Arôme fraise (M_0051126) | 0,005 | 0,005 | 0,005 |
| Agent colorant E124 (dilué 1/10) | 0,013 | 0,013 | 0,013 |
| *TOTAL* | *100,00* | *100,00* | *100,00* |

Le procédé de fabrication est quant à lui le suivant :

- ◦ Chauffer le lait et l'eau à 50°C
- ◦ Ajouter les protéines dans le mélange,
- ◦ Hydrater pendant 1 heure à 50°C sous agitation,
- ◦ Préchauffer la matière grasse à 50°C après 45 minutes,

∘ Ajouter le sucre et l'amidon à la préparation principale,

∘ Ajouter l'agent colorant, l'arôme et la purée de fraises à la préparation principale,

∘ Mélanger pendant 5 minutes,

∘ Pré-émulsifier la matière grasse ans la préparation principale pendant 5 minutes à 10000 rpm,

∘ Homogénéiser le produit en ligne à 65°C - 190 bars (2 étages)

∘ Stériliser le produit à 30 l/h avec un temps de séjour de 7 secondes à 138°C sur un échangeur tubulaire

∘ Refroidir à 40°C et stocker à +4°C.

**Exemple 16 : comparaison des propriétés sensorielles des boissons laitières aromatisées à la fraise**

Pour l'évaluation du goût, le panel est constitué de 12 personnes.

Les panels sont qualifiés à la dégustation de produits formulés avec la protéine de pois. Ils ont reçu une formation et un entraînement afin de vérifier ses performances en termes de :

- Capacité à discriminer les produits

- Consensus, utilisation correcte des descripteurs

- Répétabilité, capacité à repérer un doublon

En effet, ils ont reçu une formation pour la bonne utilisation des descripteurs sensoriels de de goût et de texture, tels que, par exemple :

Liste des descripteurs :

| Descripteur | Définition | Mode opératoire | Référence |
|---|---|---|---|
| **Aspect** | | | |
| **Lisse** | absence de particules hétérogènes à la surface du produit (pas lisse / très lisse) | Explorez visuellement le produit. | Matrice liquide : eau pure Matrice poudre : sucre glace |
| **Brillant** | Aspect brillant ou lustré résultant de la tendance d'une surface à réfléchir la lumière. (pas brillant / très brillant) | Explorez visuellement le produit. | Jaune d'oeuf cru |
| **Texture en bouche** | | | |
| **Epais** | Evaluation de la facilité du produit à s'écouler en bouche. (pas épais / très épais) | Frottez la langue contre le palais et vérifiez si le produit s'écoule facilement. | Lait concentré sucré, miel |
| **Aqueux** | Evaluation de propriété de texture de la surface qualifiant la perception de la quantité d'eau libérée par un produit. (pas aqueux / très aqueux) | Dégustez une unité de produit et évaluez la quantité d'eau perçue en bouche. | Pastèque |
| **Crémeux** | Evaluation de la texture douce et fondante du produit. (pas crémeux / très crémeux) | Mâchez une unité de produit et vérifiez s'il provoque un contact mou et s'il tapisse la bouche. | Crème fraîche, crème épaisse |
| **Sableux** | Evaluation de la granulosité et du nombre de particules d'un produit. (pas sableux / très sableux) | Mâchez une unité de produit jusqu'à ce qu'il soit prêt à avaler et évaluez la perception de petits grains dans la bouche. | Certaines poires |

Odeur et goût

| arôme / odeur | | | | saveur / sensation bouche |
|---|---|---|---|---|
| **lacté** | **végétal** | **arôme** | **off flaveur** | |
| lait | AG pois / végétal | vanille | papier / carton | acide |
| lactosérum | céréales | fraise | lessive | amer |
| lait fermenté | végétal | | chimique | salé |
| lait reconstitué bébé | noix fraiche | | colle | astringent / asséchant |
| yaourt | pomme de terre | | métallique | sucré |
| beurre | | | | piquant |

*Conditions de dégustation*

- Au laboratoire d'analyse sensorielle : box individuels de dégustation, murs blancs, ambiance calme (pour faciliter la concentration)
- Lumière blanche (pour avoir exactement la même vision du produit)
- En fin de matinée ou d'après-midi (pour être au maximum des capacités sensorielles)
- Produits anonymés par un code à 3 chiffres (pour éviter que le code n'influence l'appréciation des produits)
- Produits présentés dans un ordre aléatoire (pour éviter les effets d'ordre et de rémanence)

*Exercice*

La méthode employée pour comparer les produits a été le Profil Flash (JM Sieffermann, 2000).

Les produits sont tous présentés simultanément. Il s'agit de comparer les produits entre eux en réalisant une succession de classements : les panélistes choisissent les descripteurs qui leur semblent les plus pertinents pour discriminer les produits entre eux et classent les produits selon ces descripteurs, il est possible que plusieurs produits soient regroupés sur un même rang.

*Exemple :*

Descripteur sensoriel : <u>*Croustillant*</u>

Deux listes de descripteurs, relatives au goût ou à la texture, ont été proposées aux panélistes à titre indicatif, celles-ci vous sont fournies en annexe de ce rapport.

*Traitement des données*

La méthode de traitement statistique adaptée à ce type de données est l'Analyse Factorielle Multiple (J. Pagès, 1994) sur les données-rangs des produits. Pour que les résultats soient plus clairs, l'AFM a été réalisée plusieurs fois; en global, et par critère (aspect, odeur, goût, texture). Les graphiques présentés synthétisent l'ensemble des résultats fournis par cette méthode.

Les traitements statistiques ont été effectués avec le logiciel R version 2.14.1 (2011-12-22).

*Résultats :*

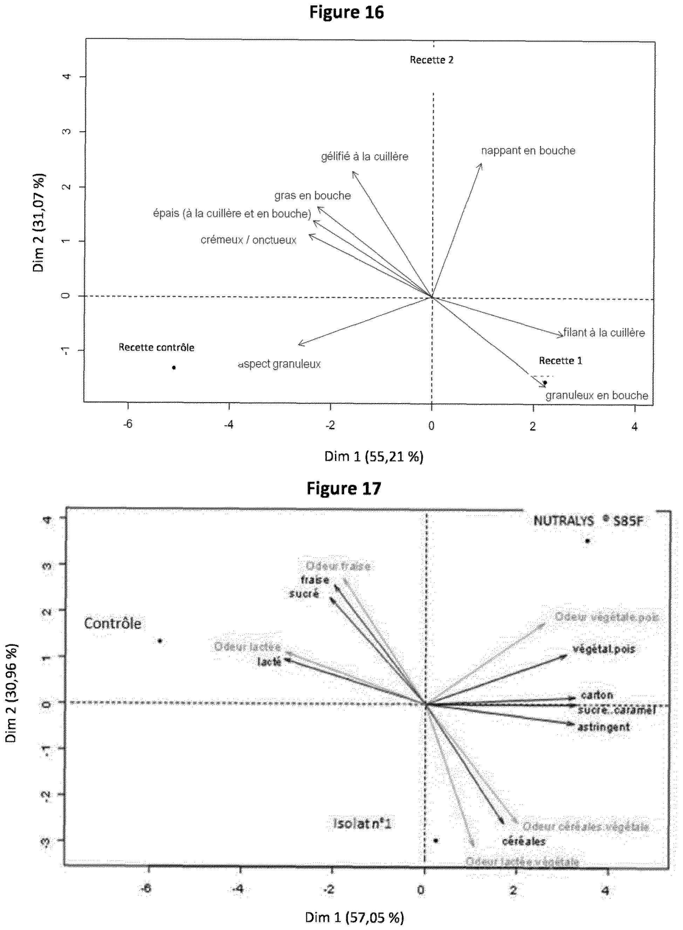

Les résultats sont présentés dans les **Figures 17** (goût) et **18** (aspect et texture en bouche).

En termes de goût, les panélistes ont bien identifié le contrôle en le qualifiant de plus sucré, lacté (odeur et gout), et fraise (odeur et gout) que les essais formulés avec la protéine de pois.

L'essai avec l'isolat de protéines de pois selon l'invention n°1 est qualifié en odeur et goût de végétal-céréales en gardant une odeur lactée, tandis que l'essai au NUTRALYS reste en odeur et goût végétal-pois.

En terme de texture, tous les produits ont été jugés aqueux. Leur caractérisation se fait essentiellement sur la dimension 1, on distingue alors deux familles :

- le témoin lait qui est jugé plus nappant en bouche suivi de l'essai avec le NUTRALYS®.
- l'isolat de protéines de pois selon l'invention n°1 est jugé plus gras.

**Exemple 17 : enrichissement en protéines de biscuits (destinés à la nutrition spécialisée / minceur ou sportif)**

Les formulations présentent la composition suivante :

|  | Témoin | NUTRALYS®S85F Roquette Frères | NUTRALYS® Pea-BF | Isolat de protéines de pois selon l'invention n°1 | Isolat de protéines de pois selon l'invention n°2 |
|---|---|---|---|---|---|
| Eau | 6 | 7,4 | 6,7 | 6,5 | 6,5 |
| Sodium bicarbonate | 0,3 | 0,3 | 0,3 | 0,3 | 0,3 |
| Ammonium bicarbonate | 0,2 | 0,2 | 0,2 | 0,2 | 0,2 |
| Saccharose | 18 | 17,7 | 17,7 | 17,9 | 17,9 |
| Sirop de glucose 4779 Roquette Frères | 2,5 | 2,5 | 2,5 | 2,5 | 2,5 |
| Matière grasse *Biscuitine 500* | 13 | 12,8 | 12,8 | 12,9 | 12,9 |
| Lécithine de soja | 0,2 | 0,2 | 0,2 | 0,2 | 0,2 |
| Farine de blé | 57,8 | 37,2 | 37,2 | 37,6 | 37,6 |
| **Protéines testées** | 0 | 19,7 | 19,7 | 19,9 | 19,9 |
| Poudre de lait écrémé | 1,5 | 1,5 | 1,5 | 1,5 | 1,5 |
| Sel | 0,2 | 0,2 | 0,2 | 0,2 | 0,2 |
| Sodium pyrophosphate | 0,2 | 0,2 | 0,2 | 0,2 | 0,2 |
| Poudre de vanille | 0,1 | 0,1 | 0,1 | 0,1 | 0,1 |
|  | **100** | **100** | **100** | **100** | **100** |

Les quantités étant indiquées en pourcentage en poids.

Les valeurs nutritionnelles de ces formules sont les suivantes :

|  | Témoin | NUTRALYS® S85F Roquette Frères | NUTRALYS® Pea-BF | Isolat de protéines de pois selon l'invention n°1 | Isolat de protéines de pois selon l'invention n°2 |
|---|---|---|---|---|---|
| Calories (kCal) | 462 | 472 | 472 | 472 | 472 |
| Protéines | 7,9 | 23,2 | 23,2 | 23,2 | 23,2 |

(suite)

|  | Témoin | NUTRALYS® S85F Roquette Frères | NUTRALYS® Pea-BF | Isolat de protéines de pois selon l'invention n°1 | Isolat de protéines de pois selon l'invention n°2 |
|---|---|---|---|---|---|
| Matière grasse | 15,7 | 17,4 | 17,4 | 17,4 | 17,4 |
| Hydrates de carbone | 72,3 | 55,8 | 55,8 | 55,8 | 55,8 |
| ...dont DP1,2 | 22,9 | 22,9 | 22,9 | 22,9 | 22,9 |
| Fibres | 1,3 | 1,1 | 1,1 | 1,1 | 1,1 |
| ...insolubles | 1,3 | 0,9 | 0,9 | 0,9 | 0,9 |
| ...solubles | 0,0 | 0,2 | 0,2 | 0,2 | 0,2 |
| kCal/ proteins (%) | 7 | 20 | 20 | 20 | 20 |

Le procédé de fabrication étant le suivant :

- Dissoudre les bicarbonates de sodium et d'ammonium dans l'eau. Ajouter le sucre et le sirop de glucose et mélanger dans un mélangeur planétaire de type Hobart équipé de la pâle plate, pendant 1 min vitesse 1 pour bien dissoudre les sucres.
- Ajouter la matière grasse et de la lécithine et mélanger pendant 2 min vitesse 2
- Ajouter le reste des poudres en une seule fois et mélanger pendant 2 min vitesse 1 puis 1 min vitesse 2.
- Laisser reposer la pâte pendant 15 min pour parfaire l'hydratation des poudres et l'homogénéité de la préparation
- Déposer la pâte dans la trémie de la rotative à biscuit afin que la pâte soit pressée dans une empreinte entre deux rouleaux afin de former les biscuits.
- Récupérer les biscuits sur un tapis convoyeur puis déposer sur une plaque de cuisson
- Cuire dans un four à chaleur tournante type MIWE Econo pendant 9 min à 170°C (ventilation vitesse 2)

Les analyses réalisées sont les suivantes :
Un des premiers critères importants dans la réalisation de biscuits sur rotative est la « machinabilité » de la pâte.
Une pâte sur-hydratée sera collante et ne se décollera pas des empreintes.
Une pâte trop sèche ne comblera pas bien les empreintes et formera des biscuits avec des anomalies.
Le fait d'ajouter une quantité importante de protéines a un impact sur la texture de la pâte. Le tableau ci-dessous illustre les ajustements d'hydratation nécessaires pour compenser l'incorporation de différentes protéines dans une pâte à biscuits.
En effet, les protéines ayant une affinité plus ou moins importantes pour l'eau vont lier une partie de l'eau de formulation. Celle-ci ne sera donc plus disponible pour « plastifier » la pâte qui sera alors trop sèche pour être formée. L'augmentation de l'hydratation de la pâte sera alors indispensable pour corriger ce défaut.

|  | Témoin | NUTRALYS® S85F Roquette Frères | NUTRALYS® Pea-BF | Isolat de protéines de pois selon l'invention n°1 | Isolat de protéines de pois selon l'invention n°2 |
|---|---|---|---|---|---|
| % d'augmentation d'eau dans la recette pour que la pâte soit faconnable | 0 | 23,2 | 11,7 | 7,8 | 7,8 |

Malheureusement dans un biscuit sec (moins de 3% d'eau dans le produit fini), il n'est pas souhaitable d'ajouter trop d'eau car cela va impacter le temps et les conditions de cuisson.
De plus, le fait d'ajouter plus d'eau aura un effet sur la cinétique de concentration des sucres et de recristallisation de ceux-ci. Or ce dernier point est déterminant pour la texture, notamment la croustillance, du biscuit.

Des protéines solubles mais peu fonctionnelles comme les isolats de protéines de pois de la présente invention permettent ainsi de limiter cette correction à seulement + 8 % d'eau ajoutée contre près de 12% pour une protéine non fonctionnelle et non soluble et plus de 23% pour une protéine soluble et fonctionnelle.

Une rapide analyse sensorielle réalisée sur les biscuits produits a donné les résultats suivants.

| Témoin | NUTRALYS® S85F Roquette Frères | NUTRALYS® Pea-BF | Isolat de protéines de pois selon l'invention n°1 | Isolat de protéines de pois selon l'invention n°2 |
|---|---|---|---|---|
| Référence en termes de texture et goût | Biscuits peu croustillants, très pâteux en bouche. Arrièregoût de pois | Biscuits sableux et pâteux. Moins de croustillance. Arrièregoût de pois. | Biscuits plus croustillants. Texture moins pâteuse, pas vraiment de goût de pois | Biscuits plus croustillants. Texture moins pâteuse que NUTRALYS® BF |

## Exemple 18 : enrichissement en protéines de muffins au chocolat (destinés à la nutrition spécialisée / minceur ou sportif)

Les formulations présentent la composition suivante :

| | NUTRALYS® S85F Roquette Frères | NUTRALYS® XF Roquette Frères | Isolat de protéines de pois selon l'invention n°2 |
|---|---|---|---|
| Farine de blé complet | 120,0 | 120,0 | 120,0 |
| SweetPearl® P200 Roquette Frères | 210,0 | 210,0 | 210,0 |
| Poudre de lait écrémé | 10,0 | 10,0 | 10,0 |
| Poudre de coco (déshuilée) | 15,0 | 15,0 | 15,0 |
| NUTRISOFT® 55 de Brenntag | 5,0 | 5,0 | 5,0 |
| Levure chimique | 5 | 5 | 5 |
| **Protéines testées** | 60,0 | 60,0 | 60,0 |
| NUTRALYS® WF Roquette Frères | 60,0 | 60,0 | 60,0 |

| | | | |
|---|---|---|---|
| WPC 515 de FONTERRA | 52,0 | 52,0 | 52,0 |
| Sel | 2,0 | 2,0 | 2,0 |
| **Huile végétale** | 80,0 | 80,0 | 80,0 |
| NEOSORB ® 70/70 Roquette Frères | 50,0 | 50,0 | 50,0 |
| Chocolat noir | 20,0 | 20,0 | 20,0 |
| Eau | 160,0 | 160,0 | 160,0 |
| Extrait de vanille | 10,0 | 10,0 | 10,0 |
| OEuf complet liquide | 140,0 | 140,0 | 140,0 |
| | **1000,0** | **1000,0** | **1000,0** |

Les quantités étant indiquées en poids (en gramme).

Le procédé de fabrication étant le suivant :

- Chauffer le mélange B afin de fondre le chocolat
- Mélanger les poudres A dans un mélangeur planétaire type Hobart équipé d'une pâle plate pendant 1 min vitesse 1
- Ajouter aux poudres le mélange B fondu et mélanger 2 min vitesse 1
- Ajouter enfin C et mélanger 2 min Vitesse 1. Racler le bol et mélanger à nouveau 2 min vitesse 2
- Répartir la préparation dans des moules à muffin en papier (70g par moule)
- Cuire dans un four ventilé type MIWE Econo à 180°C pendant 15 min (ventilation vitesse 2, aura fermé).

**Les analyses réalisées sont les suivantes** :

*Viscosité des pâtes des muffins :*

La mesure est effectuée en rhéomètre AR2000 de la société TA Instruments, avec le profil suivant :

- ○ Temps : 600 s
- ○ Vitesse : 160 rpm
- ○ Température : 25°C.

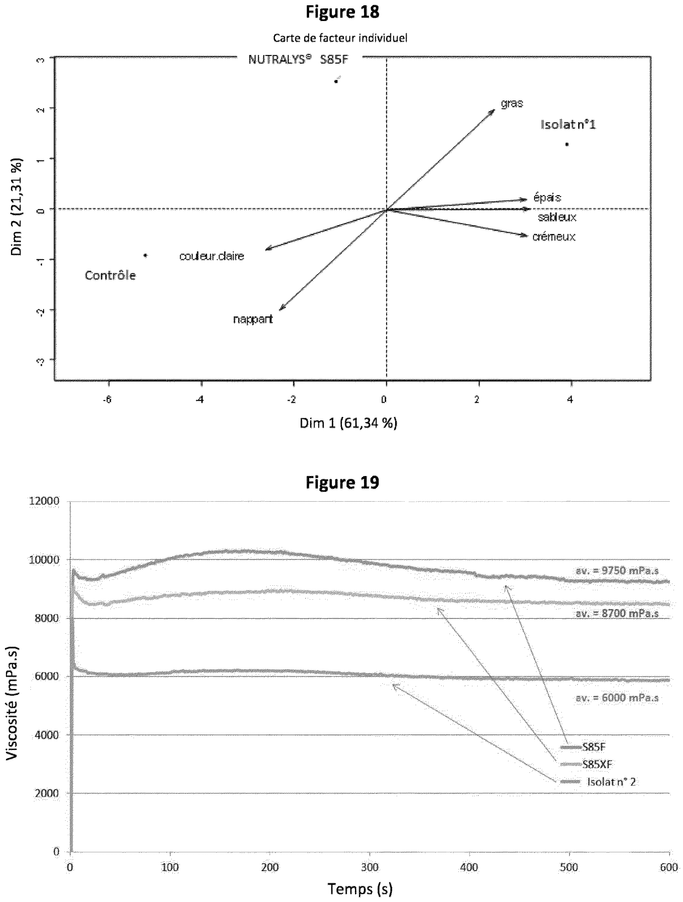

Les résultats sont présentés dans la **Figure 19.**

Dans les muffins, la viscosité de la préparation aura un impact sur la pousse à la cuisson et donc sur le volume final. Les isolats de protéines de pois selon l'invention montrent une viscosité bien plus faible que les autres protéines de pois.

**Exemple 19 : enrichissement en protéines d'un mix instant pour pancakes (destinés à la nutrition spécialisée / minceur ou sportif)**

L'objectif est ici de substituer 50 % des protéines laitières.

Les formulations présentent la composition suivante :

| | NUTRALYS®S85F | NUTRALYS®S85XF | Isolat de protéines de pois selon l'invention n°2 |
|---|---|---|---|
| | % | % | % |
| Farine de blé complet | 14 | 14 | 14 |
| WPC 450 de FONTERRA | 12,5 | 6,2 | 6,2 |
| Protéines | - | 6,3 | 6,3 |
| Farine de blé | 7,3 | 7,3 | 7,3 |
| Poudre de lait écrémé | 2 | 2 | 2 |
| Poudre de blanc d'oeuf | 1,6 | 1,6 | 1,6 |
| Matière grasse en poudre *CEGEPAL* | 1,4 | 1,4 | 1,4 |
| Levure chimique | 0,8 | 0,8 | 0,8 |
| Sel | 0,4 | 0,4 | 0,4 |
| Eau | 60 | 60 | 60 |
| | 100 | 100 | 100 |

Les quantités étant données en pourcentage en poids.

Les valeurs nutritionnelles de ces formules sont les suivantes :

| | CONTROLE | | 50% WPC remplacé par isolats de protéines de pois | |
|---|---|---|---|---|
| Pour 100g | Poudre | Pancakes | Poudre | Pancakes |
| Calories (kCal) | 375 | 211 | 373 | 211 |

(suite)

| | CONTROLE | | 50% WPC remplacé par isolats de protéines de pois | |
|---|---|---|---|---|
| Protéines (%) | 36,6 | 20,7 | 36,6 | 20,7 |
| Matière grasse (%) | 7,1 | 4,0 | 7,2 | 4,0 |
| Hydrates de carbone (%) | 41,1 | 23,2 | 40,6 | 23,0 |
| ...dont sucres (%) | 2,5 | 1,4 | 2,6 | 1,4 |
| **Fibre diététique (%)** | 3,7 | 2,1 | 3,9 | 2,2 |
| kCal/proteins (%) | 39 | 39 | 39 | 39 |

Le procédé de fabrication étant le suivant :

• Mélanger toutes les poudres.

• Ajouter l'eau et mélanger au fouet pour obtenir une préparation homogène

• Laisser reposer 2 minutes

• Cuire dans une poêle à pancake ou multi crêpes pendant environ 2 minutes en retournant les pancakes à mi-cuisson

**Les analyses réalisées sont les suivantes** :

*Impact sur la viscosité des préparations*

La mesure est effectuée en rhéomètre RVA, avec le profil suivant :

| Temps (s) | Vitesse (tours/min) | Température (°C) |
|---|---|---|
| 60 | 20 | 25 |
| 120 | 50 | 25 |
| 180 | 100 | 25 |

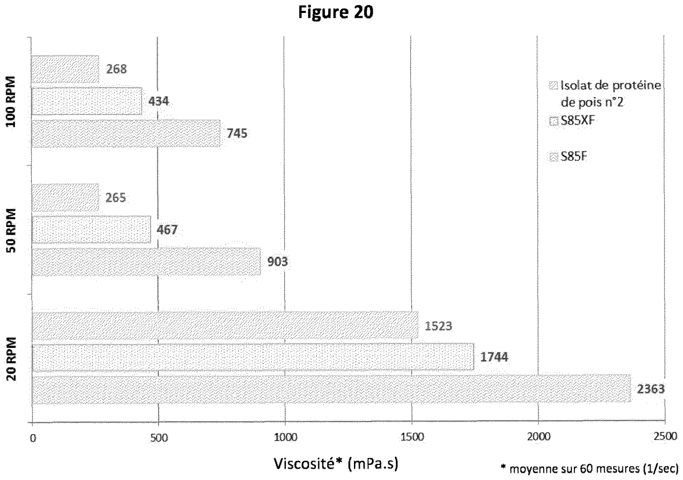

Les résultats sont présentés à la **Figure 20.**
Les mesures de viscosités RVA des préparations enrichies en protéines montrent que celles avec l'isolat de protéines de pois selon l'invention plus sont moins visqueuses qu'avec les autres protéines de pois.
Cette influence sur la viscosité a un impact sur la pousse des pancakes à la cuisson.
Une rapide analyse sensorielle réalisée sur les pancakes produits a donné les résultats suivants.

| Témoin : NUTRALYS®S85F | NUTRALYS®S85XF | Isolat de protéines de pois selon l'invention n°2 |
|---|---|---|
| Pâte liquide | Pâte plus visqueuse | Pâte liquide proche du témoin |
| produit semble plus sec en bouche. Aspect plus "de crêpes" que "pancakes" (plus minces). | De style plus "pancakes" (plus épais) Mais goût pois | Epaisseur proche du témoin Goût plus doux et texture plus souple |
| Mie blanche | Mie plus beige | |

**Exemple 20** : **pain sans gluten enrichi en protéines.**

Le pain traditionnel a une teneur en protéine d'environ 10%.

Dans les produits sans gluten en revanche le teneur en protéines est très faible. La complémentation en protéines de ces produits est alors recherchée pour rééquilibrer les valeurs nutritionnelles grâce à des protéines sans gluten comme la protéine de pois.

Les formulations présentent la composition suivante :

|  | MIX B SCHAR | | Protéines de pois de diverses origines | |
|---|---|---|---|---|
|  | 9 | % | 9 | % |
| MIX B SCHAR | 500,0 | 49,1% | 430,0 | 42,2% |
| Protéine de pois | 0,0 | 0,0% | 70,0 | 6,9% |
| Levure sèche | 8,0 | 0,8% | 8,0 | 0,8% |
| Eau | 500,0 | 49,1% | 500,0 | 49,1% |
| Huile de canola | 6,0 | 0,6% | 6,0 | 0,6% |
| Sel | 5,0 | 0,5% | 5,0 | 0,5% |
|  | 1019,0 | 100% | 1019,0 | 100% |

Les valeurs nutritionnelles de ces formules sont les suivantes :

| *pour 100G* | MIX B SCHAR | Pain enrichi à la protéine de pois de diverses origines | Pain de farine de blé traditionnel |
|---|---|---|---|
| calories (KCAL) | 236 | 241 | 267 |
| Protéines | 2,9 | 9,7 | 9,6 │ |
| Matière crasse | 1,5 | 2,2 | 1,0 |
| Hydrates de carbone | 52,7 | 45,5 | 54,8 │ |
| *...dont DP1,2* | 2,9 | *2,6* | *1,0* |
| Fibres | 2,4 | 2,1 | 1,6 │ |
| *... Fibre insoluble* | 2,4 | *2,1* | *1,6* |
| *...Fibre soluble* | *0,0* | *0,1* | *0,0 │* |
| kCal/proteins (%) | 4,8 | 16,1 | 14,4 |

**Les analyses réalisées sont les suivantes** :

Viscosité des pâtes obtenues à partir de 4 origines de protéines de pois différentes (dont les isolats selon l'invention) :

|  | MIX B SCHÄR | NUTRALYS® F85F | NUTRALYS® S85F | NUTRALYS® PEA-BF | Isolat de protéines de pois selon l'invention n°1 | Isolat de protéines de pois selon l'invention n°2 |
|---|---|---|---|---|---|---|
| Viscosité de la pâte | 0 | ... | ++ | ++ | 0 | 0 |

(suite)

| | MIX B SCHÄR | NUTRALYS® F85F | NUTRALYS® S85F | NUTRALYS® PEA-BF | Isolat de protéines de pois selon l'invention n°1 | Isolat de protéines de pois selon l'invention n°2 |
|---|---|---|---|---|---|---|
| Couleur | Blanc/ nacre | Brun/orange ++ | Brun/orange + | Brun/orange + | Brun/orange | Brun/orange |
| Hauteur maximum (mm) | 67 | 52 | 51 | 58 | 65 | 65 |
| Poids (g) | 345 | 353 | 362 | 357 | 354 | 352 |
| % de perte en eau durant la cuisson | 11% | 9% | 6% | 7% | 8% | 9% |
| Humidité du produit final (%H2O) | 54,2 | 55,0 | 54,8 | 55,0 | 54,5 | 54,7 |

Le mix SCHÀR correspond ici à la référence témoin pour un pain sans gluten. Les résultats montrent que l'enrichissement en protéines de ce mix a un impact sur la viscosité de la préparation et sur le volume final (hauteur maximale) excepté pour les isolats de protéines de pois obtenus selon l'invention qui n'affectent ni la viscosité de la préparation ni le volume final.

**Exemple 21 : pain enrichi en protéines**

Les formulations présentent la composition suivante :

| | NUTRALYS® BF | Isolat de protéines de pois selon l'invention n°2 |
|---|---|---|
| Farine de blé | 900 | 900 |
| Protéines de pois | 100 | 100 |
| Sel | 18 | 18 |
| Levure sèche | 7 | 7 |
| Acide ascorbique | 0,2 | 0,2 |
| NUTRILIFE® AM17, *enzym* | 0,2 | 0,2 |
| eau (20°C) | 725 | 725 |
| | 1750,4 | 1750,4 |

+Les quantités sont indiquées en poids (en gramme).

Les valeurs nutritionnelles de ces formules sont les suivantes :

| | Pain blanc traditionnel | Pain riche en protéines de pois |
|---|---|---|
| Calories (kCal/kJ) | 266 | 252 |
| **Protéines** | **9,6** | **14,3** |
| Matière grasse | 1,0 | 1,3 |
| Hydrates de carbone | 54,8 | 45,9 |
| *...dont DP 1,2* | *0,0* | *0,0* |

(suite)

| | Pain blanc traditionnel | Pain riche en protéines de pois |
|---|---|---|
| Fibres totales | 1,6 | 1,4 |
| ... *fibre insoluble* | *1,6* | *1,3* |
| ... *fibre soluble* | *0,0* | *0,1* |
| kCal/proteins (%) | 14 | 23 |

**Les analyses réalisées sont les suivantes :**

| | NUTRALYS PEA-BF | Isolat de protéine de pois n°2 |
|---|---|---|
| Volume (cm$^3$ ) | 1505 | 1745 |
| Poids du pain après cuisson (g) | 441,3 | 435,0 |
| Perte en eau pendant la cuisson | 11,7% | 13,3% |
| Poids de 3 disques of chapelure / diamètre 50mm (g) | 20,4 | 13,0 |
| Densité du pain (g/cm$^3$) | 0,293 | 0,249 |
| Densité de la mie (g/cm$^3$) | 0,346 | 0,221 |

Volume et densité sont en faveur des isolats de protéines de pois selon l'invention, ce qui permet une meilleure pousse donc un pain plus aéré et moelleux, moins dense.

Une rapide analyse sensorielle réalisée sur les pains produits a donné les résultats suivants.

| NUTRALYS® BF | Isolat de protéines de pois selon l'invention n°2 |
|---|---|
| Goût pois, mie sèche | Absence de goût pois ; Goût "pain grillé » moins intense ; Mie extra légère ; Volume important |

**Exemple 22** : **crisps hyper protéines (teneur en protéines >60%)**

Les crisps hyperprotéinés sont de petites céréales obtenues par cuisson extrusion présentant une teneur en protéines supérieure à 60%.

Ces céréales sont utilisées en inclusion dans des préparations céréalières telles que les barres de céréales ou les clusters.

Ces crisps hyperprotéinées sont parfois la seule solution pour enrichir en protéines ces produits céréaliers car l'incorporation de protéines sous forme poudre impacte trop la texture du produit fini.

La difficulté technique des crisps hyperprotéinés est d'atteindre des teneurs en protéines supérieures à 60%, voire 70%, tout en préservant la croustillance.

La croustillance des produits extrudés est directement liée à l'expansion. Dans les céréales obtenues par cuisson extrusion, l'expansion a lieu en sortie de filière sous la pression de la vapeur d'eau.

Les formulations des crisps hyperprotéinés à 75% de protéines présentent la composition suivante :

| | NUTRALYS® BF | NUTRALYS® S85F | Isolat de protéines de pois selon l'invention n°1 |
|---|---|---|---|
| Protéine de pois | 88% | 88% | 88% |
| PREGEFLO® C100 : amidon de maïs cireux pré-gélatinisé | 8% | 8% | 8% |
| Amidon de maïs | 4% | 4% | 4% |

Les quantités étant données en pourcentage en poids.

Le mode opératoire est le suivant :

Les crisps ou céréales extrudées ont été obtenues sur un extrudeur à bi-vis corotatives de marque CLEXTRAL Evolum 25 équipée d'un profil de vis cisaillant.

Pour comparer les essais les paramètres sont fixés dans un premier temps afin de n'avoir que la variable « type de protéine ».

La conduite d'extrusion est la suivante.

| | Paramètres fixés |
|---|---|
| Vitesse vis (RPM) | 300 |
| Vitesse coupe (RPM) | 800 |
| Débit poudre (kg/h) | 6 |
| Débit eau (kg/h) | 1,4 |
| Filière | 3 trous (3 mm diamètre) dont 2 bouchés |
| Températures des fourreaux 1 à 6 (°C) | 50-80-100-130-150-130 |
| Lame couteaux | double |

Les analyses effectuées sont les suivantes.

La méthode d'évaluation de la qualité des crisps est basée sur la somme des scores obtenus sur différents critères d'aspect et de texture selon le référentiel détaillé ci-dessous.

Une première note sur l'aspect général des crisps est obtenue en faisant la somme de :

- une note de 1 à 4 sur la forme des crisps avec la note de 1 pour une forme très irrégulière et 4 pour une forme bien ronde.
- Une note de 1 à 2 sur la couleur avec 1 pour une couleur non satisfaisante (trop sombre/ irrégulière) et 2 pour une couleur acceptable.

Une seconde note sur l'évaluation de la texture et du niveau d'expansion en faisant la somme de :

- Une note de 1 à 5 sur la dureté avec la note de 1 pour les produits « très durs » / 3 pour les produits « croquants » et 5 pour les produits « croustillants »
- Une mesure du niveau d'expansion radiale établie par le rapport du diamètre moyen des crisps par le diamètre de la filière.

Le tableau suivant résumé les résultats obtenus. Les scores les plus élevés représentent les meilleurs résultats.

| | Isolat de protéines de pois selon l'invention n°1 | NUTRALYS® BF | NUTRALYS S85F |
|---|---|---|---|
| Texture (/6) | 4,7 | 3,66 | 2,1 |
| Aspect | 4,5 | 1 | 1,5 |

Les résultats ci-dessus montrent que les meilleurs produits ont été obtenus avec l'isolat de protéines de pois obtenu selon l'invention.

Une protéine peu fonctionnelle et peu soluble comme la protéine de pois NUTRALYS®BF donne des produits moyens en termes de texture mais inacceptables en termes d'aspect. Une protéine soluble et fonctionnelle comme le NUTRALYS® S85F donne des résultats médiocres en aspect et texture.

**Exemple 23** : **Barres nutritionnelles hyperprotéinées pour sportifs**

Le défi technique dans les barres nutritionnelles hyperprotéinées est la maitrise de la texture durant la durée de conservation du produit.

En effet les barres nutritionnelles hyperprotéinées ont tendance à durcir dans le temps.

Dans la littérature, on trouve différentes hypothèses pour expliquer ce phénomène, notamment la migration d'eau entre les ingrédients et l'agrégation des protéines.

Le choix de la ou des protéines est donc crucial pour la qualité du produit fini.

Préparation des barres nutritionnelles selon différentes recettes :

| | Ingrédients | Recettes : 50% Concentrat de protéines de petit lait + 50% protéines de pois | | | | |
|---|---|---|---|---|---|---|
| | Rapport Isolat de protéines de pois selon l'invention n°2 / NUTRALYS® S85XF | 0% / 50 % | 12.5 % / 37.5% | 25 %/ 25 % | 37.5 % / 12.5 % | 50 % /0 % |
| Sirop | Sirop de glucose / fructose 7081 | 27% | 27% | 27% | 27% | 27% |
| | ***NUTRIOSE®*** | 11% | 11% | 11% | 11% | 11% |
| | ***Sirop de sorbitol*** | 3,9% | 3,9% | 3,9% | 3,9% | 3,9% |
| | Huile de tournesol | 2% | 2% | 2% | 2% | 2% |
| Poudres | Concentrat de protéines de petit lait 80 | 20% | 20% | 20% | 20% | 20% |
| | NUTRALYS® S85 XF | 20% | 15% | 10% | 5% | 0% |
| | Isolat de protéines de pois selon l'invention n°2 | 0% | 5% | 10% | 15% | 20% |
| | ***Arômes, acides aminés*** | 1% | 1% | 1% | 1% | 1% |
| | Poudre de coco dégraissé | 1% | 1% | 1% | 1% | 1% |
| Topping | ***Poudre de chocolat noir*** | 15% | 15% | 15% | 15% | 15% |
| | TOTAL | 100% | 100% | 100% | 100% | 100% |

Les quantités étant données en pourcentage en poids.

Analyses :

*Mesure de la dureté des barres nutritionnelles.*

Est réalisé le suivi de la dureté (déterminée sur pénétromètre INSTRON® - force requise pour la pénétration d'un couteau à 40 % de l'épaisseur de la barre à vitesse constante) pendant 1 mois, avec des mesures à $J_{+1}$, $J_{+7}$, $J_{+14}$, $J_{+21}$, $J_{+28}$ sur les différentes recettes présentées ci-avant.

Les résultats sont les suivants :

| Dureté des barres (mesures Instron) en Newtons en fonction des ratios de protéines de pois et du temps de conservation | | | | | | |
|---|---|---|---|---|---|---|
| Rapport Isolat de protéines de pois selon l'invention n°2 / NUTRALYS® S85XF | | | $J_{+1}$ | $J_{+7}$ | $J_{+14}$ | $J_{+21}$ | $J_{+28}$ |
| 0% | / | 50% | 76,6 | 92,2 | 99,8 | 112 | 120,1 |
| 12,5% | / | 37,5% | 54,4 | 64,9 | 72,4 | 90,4 | 95,6 |
| 25% | / | 25% | 44 | 51,3 | 58,5 | 70 | 79,7 |
| 37,5% | / | 12,5% | 31,1 | 31 | 54,8 | 61,8 | 70,3 |
| 50% | / | 0% | 32,6 | 50,9 | 57,5 | 66,5 | 77,1 |

L'augmentation du taux d'incorporation de l'isolat de protéines de pois selon l'invention n°2 est inversement proportionnelle à la diminution de la dureté des barres, et ce, quel que soit le temps de conservation.

Un optimum est obtenu pour un ratio de 37,5% d'isolat de protéines de pois selon l'invention n°2 / 12,5 % NUTRALYS® S85XF / 50% WPC

**Exemple 24. Fromages végétaliens de type Mozzarella contenant des isolats de protéines de pois.**

La recette de fromage végétalien contenant des isolats de protéines de pois selon l'invention n°2 est donnée dans le tableau suivant.

Le témoin est une recette contenant quant à elle des protéines de pois de type NUTRALYS F85F.

| Recette | N°1 | N°2 |
|---|---|---|
| | NUTRALYS F85F | Isolat de protéines de pois selon l'invention n°2 |
| Huile de colza | 7,98 | 7,98 |
| Huile de coco | 14,82 | 14,82 |
| Fécule de pomme de terre acétéylée CLEARAM PG 9020 de Roquette Frères | 22,9 | 22,9 |
| protéines ou isolat de protéines de pois | 5 | 5 |
| Acide citrique | 0,3 | 0,3 |
| Sel | 1,7 | 1,7 |
| Levures inactivées | 1,1 | 1,1 |
| Eau | 41,7 | 41,9 |
| Saveur fromage | 0,25 | 0,25 |
| Amidon de manioc | 4 | 4 |
| OSI Pea masker 9767A | 0,25 | 0,25 |
| Total | 100 | 100 |

Les quantités étant données en pourcentage en poids.

Le procédé de préparation de la recette est le suivant :

∘ ajouter l'eau dans un récipient muni d'une double enveloppe chauffante (type Stephan Bowl - www.stephan-machinery.com/index.php?id=3 ) et chauffer à 50°C,
∘ ajouter tous les ingrédients poudre, à l'exception de l'acide citrique,
∘ mélanger à 750 rpm pendant 2 minutes à 50°C,
∘ ajouter les huiles et mélanger 2 minutes à 750 rpm,
∘ ajouter l'acide citrique et mélanger 1 minute à 750 rpm,
∘ chauffer le mélange à 75°C en mélangeant à la main régulièrement de manière à éviter que cela ne brunisse,
∘ arrêter l'entrée de vapeur dans la double enveloppe
∘ cuire pendant 5 minutes, en mélangeant régulièrement
∘ arrêter la cuisson et stocker à +6°C.

Les analyses de couleur, de texture, de « shredability » et de stabilité au gel/dégel et à la fonte ont été entreprises.

Si la couleur et la texture des deux recettes sont équivalentes, la recette avec l'isolat de protéines de pois n°2 présente un meilleur comportement en « shredability » et une meilleure stabilité à la fonte. La saveur est par ailleurs reconnue meilleure avec la recette n°2.

**Revendications**

**1.** Isolat de protéines de pois, **caractérisé en ce qu'**il

∘ présente entre 0,5 et 2 % d'acides aminés libres, le taux d'acides aminés libres étant déterminé selon la méthode de mesure décrite dans la description,
◦ présente une viscosité à 20 °C dans l'eau à 15% de matière sèche:

◦ de 11 à 18.10$^{-3}$ Pa.s. à un taux de cisaillement de 10 s$^{-1}$,
◦ de 9 à 16.10$^{-3}$ Pa.s. à un taux de cisaillement à 40 s$^{-1}$, et
◦ de 8 à 16.10$^{-3}$ Pa.s. à un taux de cisaillement de 600 s$^{-1}$,

la viscosité étant déterminée selon la méthode de mesure décrite dans la description,
◦ présente une solubilité à 20 °C dans l'eau : ,

◦ de 30 à 40 % dans des zones de pH de 4 à 5
◦ de 40 à 70 % dans des zones de pH de 6 à 8,

la solubilité étant déterminée selon la méthode de mesure décrite dans la description,
◦ présente un degré d'hydrolyse (DH) compris entre 5 et 10 %, le degré d'hydrolyse étant déterminé selon la méthode de mesure décrite dans la description,

présente une teneur en protéines totales exprimée en N.6,25 de plus de 70% en poids de produit sec.

2. Isolat de protéines de pois selon la revendication 1, **caractérisé en ce que** qu'il présente une digestibilité exprimée selon le Coefficient d'Utilisation Digestive (CUD) d'une valeur comprise entre 93,5 et 95 %, la digestibilité étant déterminée selon la méthode de mesure décrite dans la description.

3. Isolat de protéines de pois selon l'une quelconque des revendications 1 à 2, **caractérisé en ce qu'**il est présenté, selon le test SYMPHID décrit dans la description, comme une protéine de « viscosité rapide », traduisant une assimilation duodénale rapide des acides aminés constitutifs dudit isolat.

4. Isolat de protéines de pois selon l'une quelconque des revendications 1 à 3, **caractérisé en ce qu'**il a été pasteurisé à haute température et de courte durée, une température comprise entre 130°C et 150°C pendant une période d'environ 1 seconde à environ 30 secondes, avant d'être séché par atomisation.

**Patentansprüche**

1. Erbsenproteinisolat, **dadurch gekennzeichnet, dass** es

◦ zwischen 0,5 und 2 % freie Aminosäuren aufweist, wobei der Gehalt an freien Aminosäuren gemäß dem in der Beschreibung beschriebenen Messverfahren bestimmt wird,
◦ eine Viskosität bei 20 °C in Wasser von 15 % Trockensubstanz aufweist:

◦ von 11 bis 18.10$^{-3}$ Pa.s. bei einer Scherrate von 10 s$^{-1}$,
◦ von 9 bis 16.10$^{-3}$ Pa.s. bei einer Scherrate von 40 s$^{-1}$ und
◦ von 8 bis 16.10$^{-3}$ Pa.s. bei einer Scherrate von 600 s$^{-1}$,

wobei die Viskosität gemäß dem in der Beschreibung beschriebenen Messverfahren bestimmt wird,
◦ eine Löslichkeit in Wasser bei 20 °C aufweist:,

◦ von 30 bis 40 % in pH-Bereichen von 4 bis 5,
◦ von 40 bis 70 % in pH-Bereichen von 6 bis 8,

wobei die Löslichkeit gemäß dem in der Beschreibung beschriebenen Messverfahren bestimmt wird,
◦ einen Hydrolysegrad (HG) zwischen 5 und 10 % aufweist,

wobei der Hydrolysegrad gemäß dem in der Beschreibung beschriebenen Messverfahren bestimmt wird,
einen Gesamtproteingehalt, ausgedrückt als N.6,25, von mehr als 70 Gew.-% des Trockenprodukts aufweist.

2. Erbsenproteinsolat nach Anspruch 1, **dadurch gekennzeichnet, dass** es eine Verdaulichkeit, ausgedrückt durch den Nahrungsverwertungskoeffizienten (DUC), von 93,5 bis 95 % aufweist, wobei die Verdaulichkeit gemäß dem in der Beschreibung beschriebenen Messverfahren bestimmt wird.

3. Erbsenproteinsolat nach einem der Ansprüche 1 bis 2, **dadurch gekennzeichnet, dass** es gemäß dem in der Beschreibung beschriebenen SYMPHID-Test als Protein mit "schneller Viskosität" dargestellt wird, was eine schnelle duodenale Assimilation der das Isolat bildenden Aminosäuren widerspiegelt.

4. Erbsenproteinsolat nach einem der Ansprüche 1 bis 3, **dadurch gekennzeichnet, dass** es bei einer Temperatur zwischen 130 °C und 150 °C für einen Zeitraum von etwa 1 Sekunde bis etwa 30 Sekunden kurzzeitig hochtemperaturpasteurisiert wurde, bevor es sprühgetrocknet wird.

**Claims**

1. A pea protein isolate, **characterized in that** it:

   ∘ contains between 0.5 and 2% of free amino acids, the content of free amino acids being determined according to the method described in the description,
   ∘ has a viscosity at 20°C in water at 15% solids:

   - from 11 to $18\times 10^{-3}$ Pa.s. at a shear rate of 10 s$^{-1}$,
   - from 9 to $16\times 10^{-3}$ Pa.s. at a shear rate of 40 s$^{-1}$, and
   - from 8 to $16\times 10^{-3}$ Pa.s. at a shear rate of 600 s$^{-1}$,

   the viscosity being determined according to the method described in the description,
   ∘ has a solubility in water at 20°C:

   - from 30 to 40% in pH zones from 4 to 5
   - from 40 to 70% in pH zones from 6 to 8,

   the solubility being determined according to the method described in the description,
   ∘ has a degree of hydrolysis (DH) of between 5% and 10%, the degree of hydrolysis being determined according to the method described in the description,
   ∘ has a total protein content expressed as N.6.25 of more than 70% by weight of dry product.

2. The pea protein isolate as claimed in claim 1, **characterized in that** it has a digestibility expressed according to the Coefficient of Digestive Use (CDU) of between 93.5 and 95%, the digestibility being determined according to the method described in the description.

3. The pea protein isolate as claimed in any one of claims 1 to 2, **characterized in that** it is presented, according to the SYMPHID test described in the description, as a protein of "rapid viscosity", reflecting rapid duodenal assimilation of the constituent amino acids of said isolate.

4. The pea protein isolate as claimed in any one of claims 1 to 3, **characterized in that** it has been pasteurized at high temperature for a short time, of between 130°C and 150°C for a time from about 1 second to about 30 seconds, before being dried by atomization.

## Figure 1

Formulation contrôle : 100 % protéine de lait

Formulation nutritionnelle selon l'invention n°2
*50% isolats protéines de pois + 50 % protéines de lait*

Formulation nutritionnelle selon l'invention n°1
*50% isolats protéines de pois + 50 % protéines de lait*

Formulation nutritionnelle avec protéines de pois
*100 % protéines de pois*

Distribution des tailles de particules

Volume (%)

Taille de particules (µM)

## Figure 2

Carte de facteur individuel

## Figure 3

### Carte de facteur individuel

## Figure 4

## Figure 5

## Figure 6

### Carte de facteur individuel

## Figure 7

### Carte de facteur individuel

## Figure 8

## Figure 9

## Figure 10

## Figure 11

## Figure 12

## Figure 13
### Carte de facteur individuel

## Figure 14

## Figure 15

## Figure 16

## Figure 17

## Figure 18

Carte de facteur individuel

## Figure 19

# Figure 20

Figure 21

## RÉFÉRENCES CITÉES DANS LA DESCRIPTION

**Documents brevets cités dans la description**

- WO 200717572 A

**Littérature non-brevet citée dans la description**

- *Codex Alimentarius,* 2007
- *Codex Alimentarius,* 1995
- Bioavailabilty of your ingrédients. *Appl Environ Microbiol.,* Janvier 2007, vol. 73 (2), 508-15
- **HALL et al.** Casein and whey exert different effects on plasma amino acid profiles, gastrointestinal hormone sécrétion ans appetite. *Br. J. Nutr.,* 2003, vol. 89, 239-248
- Le profil Flash: Un outil rapide et innovant d'évaluation sensorielle descriptive. **JM SIEFFERMANN.** L'innovation: de l'idée au succès - Douzièmes rencontres AGORAL. Tec & Doc, 2000, 335-340
- **J. PAGÈS.** Multiple factor analysis (AFMULT package). *Computational Statistics & Data Analysis,* Août 1994, vol. 18 (1), 121-140